# EUROPEAN PATENT SPECIFICATION

(11) **EP 1 564 293 B1**
(45) Date of publication and mention of the grant of the patent: **27.08.2008**
(21) Application number: 04076254.4
(22) Date of filing: 20.04.1994
(51) Int. Cl.: C12N 15/12, G01N 33/53, A61K 49/00, A61K 31/70, C12Q 1/68, C12N 5/00, C12P 21/02, C07H 15/12

(54) **Neural thread protein gene expression and detection of Alzheimer's disease**
Geneexpression der Neurofilamentproteine und Nachweis der alzheimeschen Erkrankung
Expression des gènes de proteines des neurofilaments et detection de la maladie d'Alzheimer

(30) Priority: 20.04.1993 US 50559
(43) Date of publication of application: 17.08.2005
(62) Divisional of application: 94915396.9
(73) Proprietor: THE GENERAL HOSPITAL CORPORATION, Boston, MA 02114 (US)
(72) Inventor: De La Monte, Suzanne M., East Greenwich Rhode Island 02818 (US); Wands, Jack R., Waban, MA 02168 (US)
(74) Representative: Chapman, Paul William

(56) References cited:
- CACERES A ET AL: "The effect of tau antisense oligonucleotides on neurite formation of cultured cerebellar macroneurons." J NEUROSCI, JUN 1991, 11 (6) P1515-23, June 1991 (1991-06), XP000604108 UNITED STATES
- JOACHIM C L ET AL: "Amyloid [beta]-protein deposition in tissue other than brain in Alzheimer's disease" NATURE 1989 UNITED KINGDOM, vol. 341, no. 6239, 1989, pages 226-230, XP002326757 ISSN: 0028-0836
- JOHNSON S A ET AL: "Relation of neuronal APP-751/APP-695 mRNA ratio and neuritic plaque density in Alzheimer's disease" SCIENCE 1990 UNITED STATES, vol. 248, no. 4957, 1990, pages 854-857, XP001206274 ISSN: 0036-8075
- GUTIERREZ A A ET AL: "Gene therapy for cancer" LANCET 1992 UNITED KINGDOM, vol. 339, no. 8795, 1992, pages 715-721, XP002940794 ISSN: 0140-6736

### SEQUENCE LISTING

<110> The General Hospital Corporation de la Monte, Suzanne Wands, Jack R.
<120> Neural Thread Protein Gene Expression and Detection of Alzheimer's Disease
<130> P19088EP-1/PWC
<140> EP 04076254.4
   <141> 1994-04-20
<150> US 08/050,559
   <151> 1993-04-20
<160> 121
<170> PatentIn version 3.2
<210> 1
   <211> 21
   <212> DNA
   <213> Homo sapiens
<400> 1
   ccgattccaa cagaccatca t 21
<210> 2
   <211> 21
   <212> DNA
   <213> Homo sapiens
<400> 2
   ccaacagacc atcattccac c 21
<210> 3
   <211> 21
   <212> DNA
   <213> Homo sapiens
<400> 3
   ccaaaccgat tccaacagac c 21
<210> 4
   <211> 30
   <212> DNA
   <213> Homo sapiens
<400> 4
   cctgggcaac aagagcgaaa actccatctc 30
<210> 5
   <211> 30
   <212> DNA
   <213> Homo sapiens
<400> 5
   atcgcttgaa cccgggaggc ggaggttgcg 30
<210> 6
   <211> 30
   <212> DNA
   <213> Homo sapiens
<400> 6
   ggggaggctg aggcaggaga atcgcttgaa 30
<210> 7
   <211> 43
   <212> DNA
   <213> Artificial Sequence
<220>
   <223> Triple helix-forming oligonucleotide
<220>
   <221> misc_feature
   <222> (1)..(21)
   <223> This region is in the 3' to 5' orientation
<220>
   <221> misc_feature
   <222> (22)..(22)
   <223> May be a 0-10 base oligonucleotide linkage between oligonucleotides
<400> 7
   tactaccaga caaccttagc cnccgattcc aacagaccat cat 43
<210> 8
   <211> 43
   <212> DNA
   <213> Artificial Sequence
<220>
   <223> Triple helix-forming oligonucleotide
<220>
   <221> misc_feature
   <222> (22)..(22)
   <223> May be a 0-10 base oligonucleotide linkage between oligonucleotides
<220>
   <221> misc_feature
   <222> (23)..(43)
   <223> This region is in the 3' to 5' orientation
<400> 8
   ccgattccaa cagaccatca tntactacca gacaacctta gcc 43
<210> 9
   <211> 43
   <212> DNA
   <213> Artificial Sequence
<220>
   <223> Triple helix-forming oligonucleotide
<220>
   <221> misc_feature
   <222> (1)..(21)
   <223> This region is in the 3' to 5' orientation
<220>
   <221> misc_feature
   <222> (22)..(22)
   <223> May be a 0-10 base oligonucleotide linkage between oligonucleotides
<400> 9
   ccaccttact accagacaac cnccaacaga ccatcattcc acc 43
<210> 10
   <211> 43
   <212> DNA
   <213> Artificial Sequence
<220>
   <223> Triple helix-forming oligonucleotide
<220>
   <221> misc_feature
   <222> (22)..(22)
   <223> May be a 0-10 base oligonucleotide linkage between oligonucleotides
<220>
   <221> misc_feature
   <222> (23)..(43)
   <223> This region is in the 3' to 5' orientation
<400> 10
   ccaacagacc atcattccac cnccacctta ctaccagaca acc 43
<210> 11
   <211> 43
   <212> DNA
   <213> Artificial Sequence
<220>
   <223> Triple helix-forming oligonucleotide
<220>
   <221> misc_feature
   <222> (1)..(21)
   <223> This region is in the 3' to 5' orientation
<220>
   <221> misc_feature
   <222> (22)..(22)
   <223> May be a 0-10 base oligonucleotide linkage between oligonucleotides
<400> 11
   ccagacaacc ttagccaaac cnccaaaccg attccaacag acc 43
<210> 12
   <211> 43
   <212> DNA
   <213> Artificial Sequence
<220>
   <223> Triple helix-forming oligonucleotide
<220>
   <221> misc_feature
   <222> (22)..(22)
   <223> May be a 0-10 base oligonucleotide linkage between oligonucleotides
<220>
   <221> misc_feature
   <222> (23)..(43)
   <223> This region is in the 3' to 5' orientation
<400> 12
   ccaaaccgat tccaacagac cnccagacaa ccttagccaa acc 43
<210> 13
   <211> 1443
   <212> DNA
   <213> Homo sapiens
<400> 13
   cgctgcgccc aggctggctc tggaaagcct gtgcggtcct ggcaggaagc ccggcccgtg 60
   gagcaggttt tcgttctgct tcagcaataa ataagggtga ccacagggac tttgcttttg 120
   gtttcctttc ctgtgaaaag gttggtttta aagtgagata cacttttccg tagaacaagt 180
   gttctatctt taaaaaccca aattgcagca ccgtggatta ctggtctcag aacaactcat 240
   tgcgcatcag atttgactct ctgattttct gtctattggc caaattgccc tttaactgca 300
   cctgaatcct ttgtgtactg atgcctttga gctgggcacc ttgggagagt gttgtgttgc 360
   tgtttacggt tcttccttcc ccttgctaat tacagtctct ggtgcccagc aagccccttt 420
   ggcttccttc cgtgactggt cacgttgtct gcctgggctc agcgtggacc tgccccatgc 480
   tgcagaacct ggcctcacct ggacttttac tagaattgcc agcttctcaa cttagcagat 540
   catcactcat gcgggcacaa gcaaagatca acactttctt ttttggtaag cttgagtttt 600
   acaagttatt ttttggtgat gcgtaagaca ttgcagtggg aaaccattca acttgagttt 660
   attggagttt gctgttgtag caggttttaa ctcaggaaca actcttgtct gatctctcgc 720
   ccctctgccg ggactacatt actgtctctc ggagccggta gcgttgctgt cgagtcccag 780
   gactatctct gcagactgct atgctcagat cgaagtattt cacaagaata cttgtgtttt 840
   taacagccct tcccctggac ggtgcgccat gagggcctca tgttacgcat tgccttttct 900
   ttctgtggat ccagtatctt cctcggcttt ttagggagca ggaaaaatgc gtctgagagc 960
   aactcttttt aaaaacctgc cctgttgtat ataactgtgt ctgtttcacc gtgtgacctc 1020
   caagggggtg ggaacttgat ataaacgttt aaaggggcca cgatttgccc gagggttact 1080
   cctttgctct caccttgtat ggatgaggag atgaagccat ttcttatcct gtagatgtga 1140
   agcactttca gttttcagcg atgttggaat gtagcatcag aagctcgttc cttcacactc 1200
   agtggcgtct gtgcttgtcc acatgcgctg ggcgtctgga ccttgaatgc ctgccctggt 1260
   tgtgtggact ccttaatgcc aatcatttct tcacttctct ggacacccag ggcgcctgtt 1320
   gacaagtgtg gagaaactcc taatttaaat gtcacagaca atgtcctagt gttgactact 1380
   acaatgttga tgctacactg ttgtaattat taaactgatt atttttctta tgtcaaaaaa 1440
   aaa 1443
<210> 14
   <211> 213
   <212> DNA
   <213> Homo sapiens
<400> 14
   gatcccgttt gacaggtgta ccgccccagt caaactcccc acctggcact gtccccggag 60
   cggtcgcgcc cgcgcgacca cggagctctg ggcgccagaa gcgagagccc ctcgctgccc 120
   cccgcctcac cgggtagtga aaaaacgatg agagtagtgg tatttcaccg gcggcccgcg 180
   aggacccccg cccgacccag tgcggaacgg ggg 213
<210> 15
   <211> 358
   <212> DNA
   <213> Homo sapiens
<220>
   <221> misc_feature
   <222> (56)..(56)
   <223> n is a, c, g, or t
<400> 15
   caccgtggat tactggtctc agaacaactc attgcgcatc agatttactc tctgantttc 60
   tgtctattgg ccattgccct ttaactgcac ctgaatcctt tgtgtactga tcctttgagc 120
   tgggcacctt gggagagtgt tgtgttgctg tttacggttc ttccttcccc ttgctaatta 180
   cagtctctgg tgccagcaag cccctttggc ttccttccgt gactggtcac gttgtctgcc 240
   tgggccagcg tggccccatg ctgcagaacc tggcctcagg acttttcact agaattgccc 300
   ttcctcaact tagcagatca ttcactcatg cgggcacaag caaagatcaa cactttct 358
<210> 16
   <211> 378
   <212> DNA
   <213> Homo sapiens
<220>
   <221> misc_feature
   <222> (242)..(242)
   <223> n is a, c, g, or t
<400> 16
   cattgcagct cagcatggct cagaccagct catacttcat gctgatctcc tgcctgatgt 60
   ttctgtctca gagccaaggc caagaggccc agacagagtt gccccaggcc cggatcagct 120
   gcccagaagg caccaatgcc tatcgctcct actgctacta ctttaatgaa gaccgtgaga 180
   cctgggttga tgcagatctc tattgccaga acatgaattc gggcaacctg gtgtctgtgc 240
   tncccaggcc gagggtgcct ttgtggcctc actgattaag gagagtggca ctgatgactt 300
   caatgtctgg attggcctcc atgaccccaa aaagaaccgc cgctggcact ggagcagtgg 360
   gtccctggtc tcctacaa 378
<210> 17
   <211> 142
   <212> DNA
   <213> Homo sapiens
<400> 17
   ttttcctaga acaagggttc tatctttaaa aacccaaatt gcagcaccgc tggtctcaga 60
   acaactcatt gcgcatcaga tttactctct gatttttctg tctatttggc caaattgccc 120
   ttttaactgc acctgaatct tt 142
<210> 18
   <211> 151
   <212> DNA
   <213> Homo sapiens
<400> 18
   ttctcctata gagattgttg atttgcctct tagcaagaga ttcattgcag ctcagcatgg 60
   ctcagaccag ctcatacttc atgctgatct cctgcctgat gtttctgtct cagagccaag 120
   gtaagatctc ttttccacca accaactctt t 151
<210> 19
   <211> 75
   <212> DNA
   <213> Homo sapiens
<400> 19
   caccgtggat tactggtctc agaacaactc attgcgcatc agatttactc tctgattttt 60
   ctgtctattg gccaa 75
<210> 20
   <211> 77
   <212> DNA
   <213> Homo sapiens
<400> 20
   cattgcagct cagcatggct cagaccagct catacttcat gctgatctcc tgcctgatgt 60
   ttctgtctca gagccaa 77
<210> 21
   <211> 130
   <212> DNA
   <213> Homo sapiens
<400> 21
   gaattcctgg gctcaagtga tcctctcatg cagtctccca aagtgctggg atgacaggct 60
   tgagccacca caccaggccc atcatcagtt tatataaaga aaaaaaaacc ttaaaattgt 120
   taggcaaata 130
<210> 22
   <211> 144
   <212> DNA
   <213> Homo sapiens
<400> 22
   ggagttttgt catcaggcca gcctcatccc gaggtctcct ccaccattgg ccgtagccag 60
   caggttcagt gctcaccgaa agtaaaatcc cctccttcag caagaataaa gcaatataca 120
   ccttaggttc cactaagtaa cata 144
<210> 23
   <211> 96
   <212> DNA
   <213> Homo sapiens
<400> 23
   gttctgtgag tctcaatttg ttccttcttg gaagctgtct ggtgaatctg ttggtccctc 60
   tgtctgctat tctgtctgtc tgtatgtctg tccatg 96
<210> 24
   <211> 105
   <212> DNA
   <213> Homo sapiens
<400> 24
   gttgatttgc ctcttaagca agagattcat tgcagctcag catggctcag accagctcat 60
   acttcatgct gatctcctgc ctgatgtttc tgtctcagag ccaag 105
<210> 25
   <211> 215
   <212> DNA
   <213> Homo sapiens
<400> 25
   actaccaaac ctgcattaaa aaatttcggt tggtcgacct cggagcagaa cccaacctcc 60
   gagcagtaca tgctaagact tcaccagtca aagcgaacgt actatactca attgatccaa 120
   taacttgacc aacggaacaa gttaccctat aacagcgcaa tcctattcta gagtccatat 180
   caacagggtt tacgacctcg atgttggatc aggac 215
<210> 26
   <211> 232
   <212> DNA
   <213> Homo sapiens
<400> 26
   accacggagc tctgggcgcc agaagcgaga gcccctcgct gccccccgcc tcaccgggta 60
   gtgaaaaaac gatgagagta gtggtatttc accggcggcc cgcgaggacc cccgcccgac 120
   ccagtgcgga acgggggagt agtcccgggg gctcacttat tctacattag tctcacgtgc 180
   agactagagt caagctcaac agggtcttct ttcccgctga ttccgccaag tc 232
<210> 27
   <211> 112
   <212> DNA
   <213> Homo sapiens
<400> 27
   agtttcactc tgttgcccag gctggagtgc aatggcacaa tcctggctca ctgcaacctc 60
   cgcctcccga gctcaagcaa ttctcctgcc tcagcctcgt gagccgctgg ga 112
<210> 28
   <211> 120
   <212> DNA
   <213> Homo sapiens
<400> 28
   agatctcgct ctgtcaccca ggctgaagtg cagtggccca atctcggctc actgcgagct 60
   ccacctcccg ggttcacttc attctcctgc ctcactgcct cagcctctga gtagctggga 120
<210> 29
   <211> 594
   <212> DNA
   <213> Homo sapiens
<400> 29
   gaggcgtatt ataccatgct ccatctgcct acgacaaaca gacctaaaat cgctcattgc 60
   atactcttca atcagccaca tagccctcgt agtaacagcc attctcatcc aaaccccctg 120
   aagcttcacc ggcgcagtca ttctcataat cgcccacggg cttacatcct cattactatt 180
   ctgcctagca aactcaaact acgaacgcac tcacagtcgc atcataatcc tctctcaagg 240
   acttcaaact ctactcccac taatagcttt ttgatgactt ctagcaagcc tcgctaacct 300
   cgccttaccc cccactatta acctactggg agaactctct gtgctagtaa ccacgttctc 360
   ctgatcaaat atcactctcc tacttacagg actcaacata ctagtcacag ccctatactc 420
   cctctacata tttaccacaa cacaatgggg ctcactcacc caccacatta acaacataaa 480
   accctcattc acacgagaaa acaccctcat gttcatacac ctatccccca ttctcctcct 540
   atccctcaac cccgacatca ttaccgggtt ttcctcttaa aaaaaaaaaa aaaa 594
<210> 30
   <211> 98
   <212> PRT
   <213> Homo sapiens
<400> 30
   Glu Ala Tyr Tyr Thr Met Leu His Leu Pro Thr Thr Asn Arg Pro Lys
   1 5 10 15
   Ile Ala His Cys Ile Leu Phe Asn Gln Pro His Ser Pro Arg Ser Asn
   20 25 30
   Ser His Ser His Pro Asn Pro Leu Lys Leu His Arg Arg Ser His Ser
   35 40 45
   His Asn Arg Pro Arg Ala Tyr Ile Leu Ile Thr Ile Leu Pro Ser Lys
   50 55 60
   Leu Lys Leu Arg Thr His Ser Gln Ser His His Asn Pro Leu Ser Arg
   65 70 75 80
   Thr Ser Asn Ser Thr Pro Thr Asn Ser Phe Leu Met Thr Ser Ser Lys
   85 90 95
   Pro Arg
<210> 31
   <211> 554
   <212> DNA
   <213> Homo sapiens
<220>
   <221> misc_feature
   <222> (176) .. (176)
   <223> n is a, c, g, or t
<400> 31
   ataccatgct ccatctgcct acgacaaaca gacctaaaat cgctcattgc atactcttca      60
   atcagcacat agccctcgta gtaacagcca ttctcatcca aaccccctga agcttcaccg      120
   gcgcagtcat tctcataatc gcccacgggc ttacatcctc attactattc tgccancaaa     180
   ctcaaactac gaacgcactc acagtcgcat cataatctct ctcaaggact tcaaactcta     240
   ctcccaagct ttgtgacttc tagcaacctc gctaacctcg ccttaccccc actattaact     300
   actgggagaa tgtgctagta accacgttct ccttcaaata tcactctcct acttacagga     360
   ctcaacatac tagtccagcc ctatactccc tctacatatt taccacaaca caatgggctc     420
   actcacccac cacattaacc ataaaaccct cattcacacg agaaaacacc ctcatgttca     480
   tacacctatc ccccattctt cctatccctc aaccccgaca tcaaccgggt ttcctcttaa     540
   aaaaaaaaaa aaaa                                                       554
<210> 32
   <211> 590
   <212> DNA
   <213> Homo sapiens
<400> 32
   atgcctatcg ctcctactgc tactacttta atgaagaccg tgagacctgg gttgatgcag      60
   atctctattg ccagaacatg aattcgggca acctggtgtc tgtgctcacc caggccgagg     120
   gtgcctttgt ggcctcactg attaaggaga gtggcactga tgacttcaat gtctggattg     180
   gcctccatga ccccaaaaag aaccgccgct ggcactggag cagtgggtcc ctggtctcct     240
   acaagtcctg gggcattgga gccccaagca gtgttaatcc tggctactgt gtgagcctga     300
   cctcaagcac aggattccag aaatggaagg atgtgccttg tgaagacaag ttctcctttg     360
   tctgcaagtt caaaaactag aggcagctgg aaaatacatg tctagaactg atccagcaat     420
   tacaacggag tcaaaaatta aaccggacca tctctccaac tcaactcaac ctggacactc     480
   tcttctctgc tgagtttgcc ttgttaatct tcaatagttt tacctacccc agtctttgga     540
   accttaaata ataaaaataa acatgtttcc actaaaaaaa aaaaaaaaaa                590
<210> 33
   <211> 466
   <212> DNA
   <213> Homo sapiens
<400> 33
   cttctttttc aggccaagag gcccagacag agttgcccca ggcccggatc agctgcccag     60
   aaggcaccaa cctatcgctc ctactgctac tactttaatg aagaccgcga gacctgggtt    120
   gatgcagtgt gagtgaggag agcgtgtggg aagggagact catgaaggga ggggaagctg    180
   ccactctcca gtgttcagtg gcgcaatgag atgagactga acccctttat actatcatca    240
   gccccaaact ttccaatcta ctttatccca ttattcagca cattcccagc acaaagaacc    300
   tggtgggtga cagcatcatc acggacatta ctctgctgtc ctttttcacc ctcctcttgg    360
   aggactcagt atatccgtca caaccctcca ctgagtctcc attttcttct gcaacagctc    420
   tattgccaga acatgaattc gggcaacctg gtgtctgtgc tcaccc                   466
<210> 34
   <211> 501
   <212> DNA
   <213> Homo sapiens
<220>
   <221> misc_feature
   <222> (123) .. (123)
   <223> n is a, c, g, or t
<400> 34
   cgtattatac catgctccat ctgcctacga caaacagacc taaaatcgct cattgcatac     60
   tcttcaatca gccacatagc cctcgtagta acagccattc tcatccaaac cccctgaagc    120
   ttnccggcgc agtcattctc ataatcgccc acgggcttac atcctcatta ctattctgcc    180
   tagcaaactc aaactacgaa cgcactcaca gtcgcatcat aatcctctct caaggacttc    240
   aaactctact cccactaata gctttttgat gacttctagc aagcctcgct aacctcgcct    300
   taccccccac tattaaccta ctgggagaac tctctgtgct agtaaccacg ttctcctgat    360
   caaatatcac tctcctactt acaggactca acatactagt cacagcccta tactccctct    420
   acatatttac cacaacacaa tggggctcac tcacccacca cattaacaac ataaaaccct    480
   cattcacacg agaaaacacc c                                              501
<210> 35
   <211> 372
   <212> DNA
   <213> Homo sapiens
<400> 35
   gttcttagtc tatctcttgt acaaacgatg tgctttgaag atgttagtgt ataacaattg      60
   atgtttgttt tctgtttgat tttaaacaga gaaaaaataa aagggggtaa tagctccttt     120
   tttcttcttt cttttttttt ttcatttcaa aattgctgcc agtgttttca atgtaggaca     180
   acagagggat atgctgtaga gtgtttttat tgcctagttg acaaagctgc ttttgaatgc     240
   tggtggttct attcctttgc acatcacgac attttataat catagttaaa tcgtatatga     300
   caaaaatgct ctgatctgat gccaaaggtc aattcagtgt atataacctg aacacactca     360
   tccattgcgt tt                                                          372
<210> 36
   <211> 68
   <212> PRT
   <213> Homo sapiens
<400> 36
   Met Phe Val Phe Cys Leu Ile Leu Asn Arg Glu Lys Ile Lys Gly Gly
   1               5                   10                  15
   Asn Ser Ser Phe Phe Leu Leu Ser Phe Phe Phe Ser Phe Gln Asn Cys
               20                  25                  30
   Cys Gln Cys Phe Gln Cys Arg Thr Thr Glu Gly Tyr Ala Val Glu Cys
           35                  40                  45
   Phe Tyr Cys Leu Val Asp Lys Ala Ala Phe Glu Cys Trp Trp Phe Tyr
       50                  55                  60
   Ser Phe Asp Thr
   65
<210> 37
   <211> 377
   <212> DNA
   <213> Homo sapiens
<220>
   <221> misc_feature
   <222> (59) .. (59)
   <223> n is a, c, g, or t
<400> 37
   actgtctccc cctttgatag ggacactaaa gtggtctgta cttgggtaga ggatggcang      60
   ttaagaatta aaatcgtctg ggtgcggtct gcacgcttgt aatcccagca ctttgggagg     120
   ctgaggcggg cggatcacct gaggtcagga gttcgacacc agcctgatga acatggagaa 180
   accccatctc tactaaaaat acaaatatta gctgggcgtt gtcgcgcgcc tgtaatccca 240
   gcggctcacg aggctgaggc aggagaattg cttgagctcg ggatggcgga ggttgcagtg 300
   agccaggatt gtgccattgc actccagcct gggcaacaag agtgaaactc tgtctcaaaa 360
   aaaaaaaaaa aaaaaaa 377
<210> 38
   <211> 1480
   <212> DNA
   <213> Homo sapiens
<400> 38
   gcgtaaacac atttttgttc ttagtctatc tcttgtacaa acgatgtgct ttgaagatgt 60
   tagtgtataa caattgatgt ttgttttctg tttgatttta aacagagaaa aaataaaagg 120
   gggtaatagc tccttttttc ttctttcttt gattttaaac agagaaaaaa taaaaggggg 180
   taatagctcc ttttttcttc tttctttttt ttttttcatt tcaaaattgc tgccagtgtt 240
   ttcaatgatg gacaacagag ggatatgctg tagagtgttt tattgcctag ttgacaaagc 300
   tgctttgaat gctggtggtt ctattccttt gacactacgc acttttataa tacatgttaa 360
   tgctatagga caagatgctc tgattcctga gtgccagagg ttcaattcag tgtatataac 420
   tgaacacact catccatttg tgcttttgtt ttttttatgg tggcttaaag gtaaagagcc 480
   catcctttgc aagtcatcca tgttgttact taggcatttt atcttggctc aaattgttgg 540
   aagaatggtg gcttgtttca tggtttttgt atttgtgtct aatgcacgtt ttaacatgat 600
   agacgcaatg cattgtgtag ctagttttct ggaaaagtca actcttttag gaattgtttt 660
   tcagatcttc aataaatttt ttctttaaat ttcaaagaac aatgtgcttg tgttgatgcc 720
   ttacaaaaac cattgtatat ttgtgtattc cttcttgtat ttagacagtg gtttttcagg 780
   tgcgtgcttt gttttctggt atggccttta tggaatgaga cgctttagct ttggtacgta 840
   gcgctaatcc atagcagctt tggcagtttc gtgtcttgag tcttagctaa aaagttagaa 900
   gtttacatga ctgttttttt tattttccct aaattattac ttactctgag cattaattaa 960
   gggcattttc acctgtgtaa aattatggtc agcttttttc tgtctataat tgtttacttt 1020
   tgtgggttta ctctagaaac atgagccaaa aatgtcaata gacaacacag tattaaaata 1080
   acccaaaagt tgtaaagggc aacgtttctc ccctttgata gggacactaa agtggtctgt 1140
   acttgggtag aggatggcag acgttaagaa ttaaaatgcg tctgggtgcg gtctcacgct 1200
   tgtaatccca gcactttggg aggctgaggc gggcggatca cctgaggtca ggagttcgac 1260
   accagcctga tgaacatgga gaaaccccat ctctactaaa aatacaaata ttagctgggc 1320
   gttgtcgcgc gcctgtaatc ccagcggctc acgaggctga ggcaggagaa ttgcttgagc 1380
   tcgggatggc ggaggttgca gtgagccagg attgtgccat tgcactccag cctgggcaac 1440
   aagagtgaaa ctctgtctca aaaaaaaaaa aaaaaaaaaa 1480
<210> 39
   <211> 381
   <212> DNA
   <213> Homo sapiens
<400> 39
   ttgggtgagg tggaatgatg gtctgttgga atcggtttgg taaatgggtt tatttcatat     60
   ccgctatctt taactttgga ccgcgttatc tatatcatgg cgttcctttc tactttttaa    120
   tattggttcg tattatatcg ttcctgattg gggatatgga agacgtatta cttaattgta    180
   ctttattgaa acgttcctct cggtttcgat tctgggggct ttggtctgct cgatggattc    240
   ttgtcgattt tctcgtgtgg cagtaacata ccgttttatc acccttctaa atatcccatc    300
   tcccgctgtt tggtaggctc ggaacactat cgaccaacag gttctatcta gaatcaagtt    360
   ggaaattaaa cggtgtcttg g                                              381
<210> 40
   <211> 122
   <212> PRT
   <213> Homo sapiens
<400> 40
   Met Met Val Cys Trp Asn Arg Phe Gly Lys Trp Val Tyr Phe Ile Ser
   1               5                   10                  15
   Ala Ile Phe Asn Phe Gly Pro Arg Tyr Leu Tyr His Gly Val Pro Phe
               20                  25                  30
   Tyr Phe Leu Ile Leu Val Arg Ile Ile Ser Phe Leu Ile Gly Asp Met
           35                  40                  45
   Glu Asp Val Leu Leu Asn Cys Thr Leu Leu Lys Arg Ser Ser Arg Phe
       50                  55                  60
   Arg Phe Trp Gly Ala Leu Val Cys Ser Met Asp Ser Cys Arg Phe Ser
   65                  70                  75                  80
   Arg Val Ala Val Thr Tyr Arg Phe Ile Thr Leu Leu Asn Ile Pro Ser
                   85                  90                  95
   Pro Ala Val Trp Met Ala Arg Asn Thr Ile Asp Gln Gln Val Leu Ser
               100                 105                 110
   Arg Ile Lys Leu Glu Ile Lys Arg Cys Leu
           115                 120
<210> 41
   <211> 420
   <212> DNA
   <213> Homo sapiens
<400> 41
   cccacaggtc ctaaactacc aaacctgcat taaaaaattt cggttggtcg acctcggagc    60
   agaacccaac ctccgagcag tacatgctaa gacttcacca gtcaaagcga acgtactata   120
   ctcaattgat ccaataactt gaccaacgga acaagttacc ctagggataa cagcgcaatc   180
   cgatggtgca gccgctatta aaggttcgtt tgttcaaacg attaaagtcc tcgtgtctga   240
   gttcagaccg aagtaatcca ggtcggtttc tatcttcttc aaattcctcc ctgtaccgaa   300
   aggactaatg agaaataagg cctacttcac aaagcggcct tcccccgtaa tgatatcatc   360
   tcaacttagt attataccca cacccaccca agaacaggtt tgttaaaaaa aaaaaaaaaa   420
<210> 42
   <211> 381
   <212> DNA
   <213> Homo sapiens
<400> 42
   aacccactcc accttactac cagacaacct tagccaaacc atttacccaa ataaagtata    60
   ggcgatagaa attgaaacct ggcgcaatag atatagtacc gcaaggaaag atgaaaaatt   120
   ataaccaagc ataatatagc aaggactaac ccctatacct tctgcataat gaattaacat   180
   gaaataactt tgcaaggaga gccaaagcta agacccccga aaccagacga gctacctaag   240
   aacagctaaa agagcacacc gtcattgtat ggcaaaatag tgggaagatt tatagggtag   300
   agggcgacaa accatccgag ccttgtgata gctggttgtc caagatagat cttagttcaa   360
   cctttaattt gccacagaac c                                             381
<210> 43
   <211> 629
   <212> DNA
   <213> Homo sapiens
<400> 43
   tttttttttt tttttaacaa accctgttct tgggtgggtg tgggtataat actaagttga    60
   gatgatatca ttacggggga aggccgcttt gtgaagtagg ccttatttct cattagtcct   120
   ttcggtacag ggaggaattt gaagaagata gaaaccgacc tggattactt cggtctgaac   180
   tcagacacga ggactttaat cgtttgaaca aacgaacctt taatagcggc tgcaccatcg   240
   ggatgtcctg atccaacatc gaggtcgtaa accctattgt tgatatggac tctagaatag 300
   gattgcgctg ttatccctag ggtaacttgt tccgttggtc aagttattgg atcaattgag 360
   tttagtagtc cgcttggagt ggtgaagtct agaatgtcct gttcgggggt tggtttctgc 420
   tcccaggtcg ccccaaccga attttttatt gaaggttggg tagtttagca cctgtgggtt 480
   ggtaaggtac tgttggaatt aataaattaa agctccatag ggtctcctcg tcttgttgtg 540
   taatgccccc ctctccacgg gaaggtcaat tccactggtt aaaagtaaga gaaagctgaa 600
   ccctcgggga gccatccata caggtcccc 629
<210> 44
   <211> 256
   <212> DNA
   <213> Homo sapiens
<400> 44
   gcgggtaaat tggtttgtta ttttttaaaa aaaacttgca tgtttaaaaa aaagttgatt 60
   gcttcaaatt tctgctacta acttcaagct atgggagttt ggcagtagtc acttgaggat 120
   tttttttcca attcttttct ttttgttgtt aaagctgtac ttcagtgaac agaaaaattg 180
   ccaagcaaac taatggacta taaagcgtaa tttgactgtg tgggactaaa ctacagagcc 240
   tacttgacca gtggat 256
<210> 45
   <211> 270
   <212> DNA
   <213> Homo sapiens
<400> 45
   catgtttaaa aaaaagttga ttgcttcaaa ttactgctac taacttcaag ctatgggagt 60
   ttggcagtag tcacttgagg attttttttc caattcgttt tcatttttgt tgttaaagct 120
   cgtacttcag tgagacagaa aaattgccaa gctaaactaa tggtctataa aagcgtaatt 180
   tgcatgtgtg ggcaaaaact acagagcctc aattgccact gaggtatagt acaaagtttt 240
   aatacatttt gtaaatcaaa ttgaaagaaa 270
<210> 46
   <211> 270
   <212> DNA
   <213> Homo sapiens
<400> 46
   catgtttaaa aaaaagttga ttgcttcaaa ttactgctac taacttcaag ctatgggagt     60
   ttggcagtag tcacttgagg attttttttc caattcgttt tcatttttgt tgttaaagct    120
   cgtacttcag tgagacagaa aaattgccaa gctaaactaa tggtctataa aagcgtaatt    180
   tgcatgtgtg ggcaaaaact acagagcctc aattgccact gaggtatagt acaaagtttt    240
   aatacatttt gtaaatcaaa ttgaaagaaa                                     270
<210> 47
   <211> 184
   <212> DNA
   <213> Homo sapiens
<400> 47
   tctgcccagg ctggtctgaa attcctgggc tgaagtgatc ctccagtctt ggcctcccaa     60
   agtgctggga ttacaggcat gagctactga gcctagcctt aatgattaat tttagagtga    120
   tggcttgtac cttcaagaca catatagatt gagacagaaa atttccatcg tccccgagaa    180
   aact                                                                 184
<210> 48
   <211> 75
   <212> PRT
   <213> Homo sapiens
<400> 48
   Ser Ser Ser Leu Gly Leu Pro Lys Cys Trp Asp Tyr Arg His Glu Leu
   1               5                   10                  15
   Leu Ser Leu Ala Leu Met Ile Asn Phe Arg Val Met Ala Cys Thr Phe
               20                  25                  30
   Lys Gln His Ile Glu Leu Arg Gln Lys Ile Ser Ile Val Pro Arg Lys
           35                  40                  45
   Leu Cys Cys Met Gly Pro Val Cys Pro Val Lys Ile Ala Leu Leu Thr
       50                  55                  60
   Ile Asn Gly His Cys Thr Trp Leu Pro Ala Ser
   65                  70                  75
<210> 49
   <211> 1381
   <212> DNA
   <213> Homo sapiens
<400> 49
   tttttttttt gagatggagt tttcgctctt gttgcccagg ctggagtgca atggcgcaat     60
   ctcagctcac cgcaacctcc gcctcccggg ttcaagcgat tctcctgcct cagcctcccc    120
   agtagctggg attacaggca tgtgcaccac gctcggctaa ttttgtattt ttttttagta     180
   gagatggagt ttaactccat gttggtcagg ctggtctcga actcccgacc tcagatgatc    240
   tcccgtctcg gcctgcccaa agtgctgaga ttacaggcat gagccaccat gcccggcctc    300
   tgcctggcta atttttgtgg tagaaacagg gtttcactga tgttgcccaa gctggtctcc    360
   tgagctcaag cagtccacct gcctcagcct cccaaagtgc tgggattaca ggcgtcagcc    420
   gtgcctggcc tttttatttt atttttttta agacacaggt gtaccactct tacccaggat     480
   gaagtgcagt ggtgtgatca cagctcactg cagccttcaa ctcctgagat caagcaatcc    540
   tcctgcctca gcctcccaag tagctgggac caaagacatg caccactaca cctggtaatt    600
   tttattttta tttttaattt tttgagacag agtctcactc tgtcacccag gctggagtgc    660
   agtggcgcaa tcttggctca ctgcaacctc tgcctcccgg gttcaagtta ttctcctgcc    720
   ccagcctcct gagtagctgg gactacaggc gcccaccacg cctagctaat ttttttgtat    780
   ttttagtaga gatggggttt caccatgttc gccaggttga tcttgatctc ttgaccttgt    840
   gatctgcctg cctcggccta cccaaagtgc tgggattaca ggtcgtgact ccacgccggc    900
   ctatttttaa tttttgtttg tttgaaatgg aatctcactc tgttacccag gtcggagtgc    960
   aatggcaaat ctcggctact cgcaacctct gcctcccggg tcaagcgatt ctcctgtctc   1020
   agcctcccaa gcagctggga ttacgggacc tgcaccacac cccgctaatt tttgtatttt   1080
   cattagaggc gggtttacca tatttgtcag gctgggtctc aaactcctga cctcaggtga   1140
   cccacctgcc tcagccttcc aaagtgctgg gattacaggc gtgagccacc tcacccagcc   1200
   ggctaatttg gaataaaaaa tatgtagcaa tgggggtctg ctatgttgcc caggctggtc   1260
   tcaaacttct ggcttcagtc aatccttcca aatgagccac aacacccagc cagtcacatt   1320
   ttttaaacag ttacatcttt attttagtat actagaaagt aatacaataa acatgtcaaa   1380
   c                                                                   1381
<210> 50
   <211> 2520
   <212> DNA
   <213> Homo sapiens
<400> 50
   ccattgttag gttgtctctt acctgttaaa atcaggagct gacaagaaat gcttaccaca 60
   aaaggagaaa tgccagtcta gttaacagtc aaggagagaa atcaggaaga ttatgtgggt 120
   ggaagaagta gatgatgtgg ctgatgagtg agtgagtgag caagcctccg cccagctgaa 180
   gaaggagtca gaactgccct ttgttcccaa ctatttggcg aaccccagcc ttccctttta 240
   tctatacacc cacagcagag gattcagccc agatgcagaa tgggggcccc tccacacccc 300
   ctgcatcacc ccctgcagat ggctcacctc cattgcttcc ccctgggaac ctcccctgtt 360
   agggaccttt ccccgggacc acacctcttt ggcactagtt cagaatggtg atgtgtcggc 420
   ccctctgcca tactagaaca ccagaaagac aaacgggtga tgtttgtcag ctacagtgag     480
   tctagagccg tcctgttttc ttctgtcccg tcccaagcca ccatgtctct tcgagcctca     540
   aaatgggacg tatgcaggac cagcgcccag attccaagcc atttttcttc actggagcat     600
   ttccatttaa tatgcaagag ctggtactca aggtgagaat tcagaaccca tctcttcgag     660
   aaaatgattt cattgaaatt gaactggacc gacagagctc acctaccaag agttgctcag     720
   agtgtgttgc tgtgagctgg gtgttaatcc agatcaagtg gagaagatca gaaagttacc     780
   caatactctg ttaaggaagg acaaggatgt tgctcgactc aagatttcag gagctggaac     840
   tggttctgat gatagtgaaa ataattttct gttcagaaat gctgcatcac actgactgaa     900
   aggccttgct atacaggaga gcttcaaaac tgacttacta atgcagcagg gacttttata     960
   ctgagtatat gacagtgtgc atcacctctg ggccaaggac aagccatgat ctaaatgcct    1020
   cagatgcccg ggccagtctg gtgcactgca tagtatatac gaacatcatt ctgcccaagg    1080
   taggaagccc catgaccccc aagcagtggt gtccactctt ccaagcctct tggtgcacaa    1140
   taaaccttat tgcttgaagc tttgaacgac tgtgagaatg gtctggcgag gacgagaacg    1200
   tggaattata tgagtgtctt ttgtatccga gaatgtagag agttctctga agacgacgac    1260
   tgagagagag cggacgctat ttctagccac tcctgttgac agtgcacctg aagggctggg    1320
   atgcgttttt cttggtgttg catgctcaca actctgctga cattgggaac ttatgagaga    1380
   ggaagactcg ggaaagcaca gatactggac agatggattc tggtgtgggg aaagcacaga    1440
   tactggacag atggttctag tgtgacttgt gactgtgagg tttcctataa catatttata    1500
   aatgttcatc aggttcaaaa gtctataaga atacagttcg agactgaatt gcttcgaaat    1560
   acttcgatgt tgggaaccaa aagagctttc cctccctcac tttttccttt gtaacactca    1620
   tgactgcttc tctgtctcga gtcatctctg cattaactcc ccttcgtggt cactagaggg    1680
   ctctctgatg cttctaagac actgcttttt acatgccaca cccaccgcgt agagacaggg    1740
   tctcactatg tggcccaggc tggtctcaaa cttctggcct taagtgatcg tcctgtcctt    1800
   cgcgctcgga agaaagtcgt ggggattaca ggtgtgagcc acccgcccag cccctccctt    1860
   gtgtttcaac caatcggaag tgaatttaac tagatgtagt aacctttttt ttctttgact    1920
   tctaaaaaag ttacagttta ctaataaagt taagtctggt tctgtcctag aggaaataaa    1980
   ttcactatta attcatgtct taagttactt gggttaaaac actttcagcc acccagatta    2040
   attaaagtgg agcagtggag cccctggctg gggagatggg cctccagagg agcagctgca    2100
   ggcatgttct ggctacacag aggcaagcaa gggactggtg tctctggtga gaggtgggtt    2160
   tgatgtatct ctgtcctatg ctggtctctc ttctccttta taaatcctcc tgtggtcact    2220
   gactatcgta tcgcagtgat cagactgcac atagtacggt taggctgagc ttaatgtctt 2280
   aatcatgtca ttcgagagaa gacacgtttt gattcatgct ttgtgtaatt aatcaatcaa 2340
   ggattctttt tttagctttg ttgacgtgta attcacccct cctcctccac tgcatattta 2400
   aagcatgtgt tcacactgtg tgtatacatt cactgcgatt ttttcgtttg ctgcattgct 2460
   tggactgttc ataacatcac aagtattatt caaataaaat attaactgac cgaaaaaaaa 2520
<210> 51
   <211> 141
   <212> DNA
   <213> Homo sapiens
<400> 51
   gaattcctgg gctcaagtga tcctctcatg tcagtctccc aaagtgctgg gatgacaggc 60
   ttgagccacc acaccaggcc catcatcagt ttttatataa agaaaaaaaa accttaaaat 120
   tgttagcaaa atactatgac a 141
<210> 52
   <211> 151
   <212> DNA
   <213> Homo sapiens
<220>
   <221> misc_feature
   <222> (96)..(96)
   <223> n is a, c, g, or t
<400> 52
   gaactcctga cctcaggtga tccgcccgcc tcagcctccc aaagtgctgg gattacaagc 60
   gtgcagaccg cacccagacg attttaattc ttaacntgcc atcctctacc caagtacaga 120
   ccactttagt gtccctatca aaggggagac a 151
<210> 53
   <211> 43
   <212> DNA
   <213> Homo sapiens
<400> 53
   aattctcctg cctcagcctc gtgagccgct gggattacag gcg 43
<210> 54
   <211> 45
   <212> DNA
   <213> Homo sapiens
<400> 54
   aagccaactc agactcagcc aacaggtaag tgggcattac aggag 45
<210> 55
   <211> 143
   <212> DNA
   <213> Rattus sp.
<400> 55
   ctcagttttc actttgatct ggtagatagt tttcgtttca gttgggggag aaggatctgt 60
   ttgtaagaac ggagtgacgg gataccataa aaatagaggt aataacatac attgggacgt 120
   gtaaatttat ttttatggaa gtg 143
<210> 56
   <211> 157
   <212> DNA
   <213> Homo sapiens
<400> 56
   ctcacagttt ccttatcttg gtggtcgtaa gttttcgtcg aaacagttga tcgttatttg 60
   tgagattgtc gtatagggag actaacaggt agtaactttt gtgaccgtcg ttaaaacttt 120
   actttttttt ttctttcttc ttttttcctt cataatg 157
<210> 57
   <211> 60
   <212> DNA
   <213> Homo sapiens
<400> 57
   accacgcccc gctaattttt gtatttttag tagagacagg gtttcaccgt gttggccagg 60
<210> 58
   <211> 60
   <212> DNA
   <213> Homo sapiens
<400> 58
   acaacgccca gctaatattt gtatttttag tagagatggg gtttctccat gttcatcagg 60
<210> 59
   <211> 60
   <212> DNA
   <213> Homo sapiens
<400> 59
   ctggtctgaa attcctgggc tgaagtgatc ctccagtctt ggcctcccaa agtgctggga 60
<210> 60
   <211> 60
   <212> DNA
   <213> Homo sapiens
<400> 60
   ctggtgtcga actcctgacc tcaggtgatc cgcccgcctc agcctcccaa agtgctggga 60
<210> 61
   <211> 26
   <212> DNA
   <213> Homo sapiens
<400> 61
   agacacatat agattgagac agaaaa 26
<210> 62
   <211> 30
   <212> DNA
   <213> Homo sapiens
<400> 62
   agtacagacc actttagtgt ccctatcaaa 30
<210> 63
   <211> 31
   <212> DNA
   <213> Homo sapiens
<400> 63
   agatctcgct ctgtcaccca ggctgaagtg c 31
<210> 64
   <211> 34
   <212> DNA
   <213> Homo sapiens
<400> 64
   agagtttcac tcttgcttgc ccaggctgga gtgc 34
<210> 65
   <211> 59
   <212> DNA
   <213> Homo sapiens
<400> 65
   agtggcccaa tctcggctca ctgcgagctc cacctcccgg gttcacttca ttctcctgc 59
<210> 66
   <211> 60
   <212> DNA
   <213> Homo sapiens
<400> 66
   aatggcacaa tcctggctca ctgcaacctc cgccctcccg agctcaagaa cttctcctgc 60
<210> 67
   <211> 66
   <212> DNA
   <213> Homo sapiens
<400> 67
   ctcagcctct gagtagctgg gactacaggc gcccaccaca agccgctaat ttttgtattt 60
   ttgtag 66
<210> 68
   <211> 67
   <212> DNA
   <213> Homo sapiens
<400> 68
   ctcagcctcg tgagccgctg ggattacagg cgcgcgccac aagcgactaa tatttgtatt 60
   tttgtag 67
<210> 69
   <211> 76
   <212> DNA
   <213> Homo sapiens
<400> 69
   accacgcccc gctaattttt gtatttttag tagagacagg gtttcaccgt gttggccagg 60
   atgctcgatc tcctga 76
<210> 70
   <211> 78
   <212> DNA
   <213> Homo sapiens
<400> 70
   acaacgccca gctaatattt gtatttttag tagagatggg gtttctccat gttcatcagg 60
   ctggtgtcga actcctga 78
<210> 71
   <211> 159
   <212> DNA
   <213> Homo sapiens
<400> 71
   ccccaagcag tgttaatcct ggctactgtg tgagctgacc tcaagcacag gtgaaggcag 60
   agaatccatc cacctgtttc tgttctccct gcttagctcc agggatggaa ctgggactgg 120
   gatagaggaa aggtgaactc ctcattaagg aaatggatg 159
<210> 72
   <211> 170
   <212> DNA
   <213> Homo sapiens
<400> 72
   cccctgttct tgggtgggtt tgggtatatt ctggttgaga tgatatcatt tacgggggaa 60
   ggcgctttgt gaagtaggcc ttatttctct tgtcctttcg tacagggagg atttgaagta 120
   gtagaacgct gttactccgg tctgaactca gtcacgtggc tttatcgttg 170
<210> 73
   <211> 52
   <212> DNA
   <213> Homo sapiens
<400> 73
   gatccaagct acgtacgcgt gcatgcacgt catagctctt ctatagtgtc ac 52
<210> 74
   <211> 53
   <212> DNA
   <213> Homo sapiens
<400> 74
   gatccgagct cggtaccaag ttgatgcata gcttgagtat tctatagtgt cac 53
<210> 75
   <211> 115
   <212> DNA
   <213> Homo sapiens
<400> 75
   gtatgggccc gatagcttat ttagccttta gagcacactg gcggccgtta ctagtggatc 60
   cgagctcggt accaacttga tgcatagctt gagtattcta tagtgtcacc taaat 115
<210> 76
   <211> 120
   <212> DNA
   <213> Homo sapiens
<400> 76
   atatagacaa tataacaata tattgtatac tttagagcac actggcagcc gttactagtg 60
   gatccgagct cggtaccaag ttgatgcata gcttgagtat tctatagtgt cactaatagt 120
<210> 77
   <211> 117
   <212> DNA
   <213> Homo sapiens
<400> 77
   cttaatagat agctacttaa aataacttac acactgtttt agagtgcttg aaaactatct 60
   gatcagacat agtattgaaa ccaatgaata cattatataa agtaaaggaa aggagaa 117
<210> 78
   <211> 137
   <212> DNA
   <213> Homo sapiens
<400> 78
   cttactacca gacaacctta gccaaaccat ttacccaaat aaagtatagg cgatagaaat 60
   tgaaacctgg cgcaatagat atagtaccgc aaggaaagat gaaaaattat aaccaagcat 120
   aatatagcaa ggactaa 137
<210> 79
   <211> 198
   <212> DNA
   <213> Homo sapiens
<400> 79
   ctataaaggt cgttgtcaac gataaagcac gtgactgagt tcagaccgga gtaacagcgt 60
   tctactactt caaatcctcc ctgcgaaagg caagagaaat aaggcctact taagcgcctt 120
   cccccgtaaa tgatatcatc tcaaccagaa tatacccaaa ccccccaaga acaggggagg 180
   aaaagaaaaa aaaaaaaa 198
<210> 80
   <211> 200
   <212> DNA
   <213> Homo sapiens
<400> 80
   ctatagagat tgttgatttg cctcttaagc aagagattca ttgcagctca gcatggctca 60
   gaccagctca tacttcatgc tgatctcctg cctgatgttt ctgtctcaga gccaaggtaa 120
   gatctctttt ccaactcttt ctagccctga agacttcact ctatccccaa gcatacgggt 180
   ctacttgaaa aaaaaaaaaa 200
<210> 81
   <211> 82
   <212> DNA
   <213> Homo sapiens
<400> 81
   cagaaaacta gctacacaat gcattggtct atcatgttaa aacgtgcatt agacacaaat 60
   acaaaaacca tgaaacaagc ca 82
<210> 82
   <211> 90
   <212> DNA
   <213> Homo sapiens
<400> 82
   cagaaaaatt gccaagctaa actaatggtc tataaaagcg taatttgcat gtgtgggcat 60
   aaactacaga gctcatgcta gagtatgcaa 90
<210> 83
   <211> 141
   <212> DNA
   <213> Homo sapiens
<400> 83
   aattgggtac cgggcccccc ctagaggtcg acggtatcga taagcttgta tcgaattccg 60
   gactttgctt ttggttttcc tttcctgtga aaaggttggt tttaaagtga gatacacttt 120
   tccgtagaac aagtgttcta t 141
<210> 84
   <211> 155
   <212> DNA
   <213> Homo sapiens
<400> 84
   aagttgattg cttcaaattt ctgctactaa cttcaagcta tgggagtttg gcagtagtca 60
   cttgaggatt ttttttccaa ttcgttttca tttttgttgt taaagctcgt acttcagtga 120
   gacagaaaaa ttgccaagct aaactaatgg tctat 155
<210> 85
   <211> 232
   <212> DNA
   <213> Homo sapiens
<400> 85
   gaattcctgg gctcaagtga tcctctcatg tcagtctccc aaagtgctgg gatgacaggc 60
   ttgagccacc acaccagccc atcatcagtt tttatataaa gaaaaaaaaa ccttaaaatt 120
   gttaggcaaa taatgacaaa ttgtaatata tattcttaca tttcagattt ttatttttta 180
   aactgataag aattgattaa taaataaaat ttagtattaa tctgtctttt aa 232
<210> 86
   <211> 245
   <212> DNA
   <213> Homo sapiens
<400> 86
   aaattcctgg gctgaagtga tcctccagtc ttggcctccc aaagtgctgg gattacaggc 60
   atgagctact gagcctagcc ttaatgatta attttagagt gatggcttgt accttcaagc 120
   aacatataga gttgagacag aaaatttcca tcgtcccgag aaaactgtgc tgcatgggcc 180
   ccgtgtgccc tgtgaagatc gccctattaa ctataaatgg gcattgcaca tggttgccag 240
   cttca 245
<210> 87
   <211> 239
   <212> DNA
   <213> Homo sapiens
<400> 87
   aacgtttcga acctatcgtg aagcccgatt ttagagttaa tacgggtgct tcaagggaac 60
   ggggctatga gaagttttct acggggagca tggaaatttt ctgtctcaat atgtgcttga 120
   aggtacaacc gtatctaaaa ttaatcatta aggctaggct cagtagctct gcctgtaatc 180
   ccagcacttt cgggaggcca agactggagg atcacttcag cccaggaatt tcagacgcc 239
<210> 88
   <211> 260
   <212> DNA
   <213> Homo sapiens
<400> 88
   tactgtgtga gcctgacctc aagcacagga ttccagaaat ggaaggatgt gccttgtgaa 60
   gacaagttct cctttgtctg caagttcaaa aactagaggc agctggaaaa tacatgtcta 120
   gaactgatcc agcaattaca acggagtcaa aaattaaacc ggaccatctc tccaactcaa 180
   ctcaacctgg acactctctt ctctgctgag tttgccttgt taatcttcaa tagttttacc 240
   taccccagtc tttggaacct 260
<210> 89
   <211> 149
   <212> DNA
   <213> Homo sapiens
<400> 89
   cttgcttgcc caggctggag tgcaatggca caatcctggc tcactgcaac ctccccctcc 60
   cgagctcaag aacttctcct gcctcagcct cgtgagccgc tgggattaca ggcgcgcgcc 120
   acaagcgact aatatttgta tttttgtag 149
<210> 90
   <211> 167
   <212> DNA
   <213> Homo sapiens
<400> 90
   ctcgctctgt cacccaggct gaagtgcagt ggcccaatct cggctcactg cgagctccac 60
   ctcccgggtt cacttcattc tcctgcctca ctgcctcagc ctctgagtag ctgggactac 120
   aggcgcccac caccacgtcc cctgctaatt ttttgtattt ttagtag 167
<210> 91
   <211> 84
   <212> DNA
   <213> Homo sapiens
<400> 91
   ccatgttcat caggctggtg tcgaactcct gacctcgtga tccgcccgcc tcagcctccc 60
   aaagtgctgg gattacaagc gtgc 84
<210> 92
   <211> 85
   <212> DNA
   <213> Homo sapiens
<400> 92
   ccgtgttggc caggatggtc tcgatctcct gacctcgtga tccgcccgcc ttggccaccc 60
   aaagagtttg ggattacagg cgtgc 85
<210> 93
   <211> 251
   <212> DNA
   <213> Homo sapiens
<400> 93
   tgcagcaatg gcaacaacgt ctgcaaacta ttaactggcg aactacttac tctagcttcc 60
   cggcaacaat taatagactg gatggaggcg gataaagttg caggaccact tctgcgctcg 120
   gcccttccgg ctggctggtt tattgctgat aaatctggag ccggtcgagc gtgggtctcg 180
   cgtatcattc gagcactggg gccagatggt aagccctccg tatcgtagtt atctcacagc 240
   agggagtcag g 251
<210> 94
   <211> 242
   <212> DNA
   <213> Homo sapiens
<400> 94
   tgcaggagcg gggaggcacg atggccgctt tggtccggat ctttgtgagg aaccttactt 60
   ctgtggtgtg acataattgg acaaactacc tacagagatt taaagctcta aggaaatata 120
   aaatttttaa gtgtataatg tgttaaacta ctgattctaa ttgtttgtgt attttagatt 180
   ccaaccctat ggaacctgat gaatgggagc cagtggtgga atgcctttaa tgaggaaacc 240
   tg 242
<210> 95
   <211> 208
   <212> DNA
   <213> Homo sapiens
<400> 95
   tgcagcaatc tttcttatat acatgcttaa tagatagcta cttaaaataa cttacacacg 60
   ttttagagtt gcttgaaaac tatctgatca agacatagta attgaaacca atgaatacat 120
   tatataaagt aaaggaaagg agaagagagg aaagggaggg gaagaggaga gggagggaca 180
   agcgagaaag gaaagggaag ggagaaaa 208
<210> 96
   <211> 152
   <212> DNA
   <213> Homo sapiens
<400> 96
   ctcactaaag ggatcaagga ataattttga atttcaagtc ttacatttaa taaatacatt 60
   cataaggcta taactaccat acgttgtgat ttctctgatt aatttaaaaa taaattaaaa 120
   cctggaaaga attttaccat tctaggaagc ca 152
<210> 97
   <211> 338
   <212> DNA
   <213> Homo sapiens
<400> 97
   aatctatctt atatacatgc ttaatagata gctacttaaa ataacttaca cacgttttag 60
   agttgcttga aaactatctg atcaagacat agtaattgaa accaatgaat acattatata 120
   aagtaaagga aaggagaaga gaggaaagga ggggagagga gaggaggaca agcgagaaaa 180
   ggaagggaag ggagaaaaag ggggaaaggg aggtagagag agagagaaaa agtgctggtc 240
   atatagtaag tgtacatttt aactttttaa gaaactaccc tactctattc cagagtgatt 300
   gtacatgtgc attttactgc attatagaga tcattttc 338
<210> 98
   <211> 169
   <212> DNA
   <213> Homo sapiens
<400> 98
   tgcaggagtg gggaggcacg atggccgctt tggtccggat ctttgtgaag gaaccttact 60
   tctgtgtgtg acataattgg acaaactacc tacagagatt taaacgtcta aggtaaatat 120
   aaaattttta gtgtataggt taaactactg attctaatgt tgtgtattt 169
<210> 99
   <211> 209
   <212> DNA
   <213> Homo sapiens
<400> 99
   ccccgggctg cagcaatggc aacaacgtct gcaaactatt aactggcgaa ctcattcatc 60
   tagcttcccg gcaacaatta atgactggat ggaggcggat aaagttgcag gaccacttct 120
   cgcgtggccc ttccggctgg ctggtttatt gctgataatt gagcgtgcga gtggctcgcg 180
   tatcattcgc gacatgggcc agtaggtac 209
<210> 100
   <211> 272
   <212> DNA
   <213> Homo sapiens
<400> 100
   cttgcccttc atggagtcat acagccgatc agcaaaatgc aggggcttgt tctgaatgca 60
   ctgaaccagg ttcaggaaag cattttccag gtctccttta acctctttcc tgatgctttc 120
   caacatgtca taagggctgt aactcttgta cctatcaaat actttctgga ggtggggaca 180
   cgctcgcgtc ggtcatgatg ctgatccact tgggaacatc agttctttcc tcttcactcc 240
   agctgcatag agatccgagg actcttggtc aa 272
<210> 101
   <211> 278
   <212> DNA
   <213> Homo sapiens
<400> 101
   acggcccagc ttccttcaaa atgtctactg ttcacgaaat cctgtgcaag ctcagcttgg 60
   agggtgatca ctctacaccc ccaagtgcat atgggtctgt caaagcctat actaactttg 120
   atgctgagcg ggatgctttg aacattgaaa cagccatcaa gaccaaaggt gtggatgagg 180
   tcaccattgt caacattttg accaaccgca gcaatgacac gagacaggat attgccttcg 240
   cctaccagag aaggaccaaa aaaggaactt gcatcaca 278
<210> 102
   <211> 228
   <212> DNA
   <213> Homo sapiens
<220>
   <221> misc_feature
   <222> (226)..(226)
   <223> n is a, c, g, or t
<400> 102
   agcaatagca aaggaaagga aacaatattt agcaaggttt attcttcctt tgtgtcagca 60
   tttctgagtg tgcacacagg cccagtgatt ccatgtattt ttgagtgacc actgcctctg 120
   tctggccctt ccccatagaa ccgccgctgg tggagcgtgg gtccctggtc tcctacaagt 180
   cctggggcat tggagcccca agcagtgtta atcctggcac tgtgtnag 228
<210> 103
   <211> 246
   <212> DNA
   <213> Homo sapiens
<220>
   <221> misc_feature
   <222> (191)..(191)
   <223> n is a, c, g, or t
<400> 103
   agcaatggca acaacgtctg caaactatta actggcgaac tacttactct tagcttccgg 60
   caacaattaa tagactggat ggaggcggat aaagttgcag gaccacttct gcgctcggcc 120
   cttccggctg gctggtttat tgctgataaa tctggagccg gtcgagcgtg ggtctcgcgt 180
   atcattcgag nctggggcca gatggtaagc cctccgtatc gtagttatct cacagcaggg 240
   agtcag 246
<210> 104
   <211> 86
   <212> DNA
   <213> Homo sapiens
<400> 104
   ctggcactgg agcagtgggt ccctggtctc ctacaagtcc tggggcattg gagccccaag 60
   cagtgttaat cctggcactg tgtgag 86
<210> 105
   <211> 154
   <212> DNA
   <213> Homo sapiens
<220>
   <221> misc_feature
   <222> (40)..(40)
   <223> n is a, c, g, or t
<220>
   <221> misc_feature
   <222> (99)..(99)
   <223> n is a, c, g, or t
<400> 105
   ctggagccgg tcgagcgtgg gtctcgcgta tcattcgagn ctggggccag atggtaagcc 60
   ctccgtatcg tagttatctc acagccgtat cattcgagnc tggggccaga tggtaagccc 120
   tccgtatcgt agttatctca cagcagggag tcag 154
<210> 106
   <211> 221
   <212> DNA
   <213> Homo sapiens
<400> 106
   aggagacttg tggtaaaaat ctgctgctgt actgctcatt tgggaacctt agtatactaa 60
   ataatataat atatcaacaa ctaatggtca gccaatgcta tgctggatat gagggtcctg 120
   ggccacaaag acaaaaaatc aggaaccact ttttaagtga gatactttgg gtctctgtca 180
   aattcataac acttatttct tggtggaata cagttaatga g 221
<210> 107
   <211> 231
   <212> DNA
   <213> Homo sapiens
<400> 107
   aggagcgggg aggcacgatg gccgctttgg tccggatctt tgtgaggaac cttacttctg 60
   tggtgtgaca taattggaca aactacctac agagatttaa agctctaagg aaatataaaa 120
   tttttaagtg tataatgtgt taaactactg attctaattg tttgtgtatt ttagattcca 180
   accctatgga acctgatgaa tgggagccag tggtggaatg cctttaatga g 231
<210> 108
   <211> 102
   <212> DNA
   <213> Homo sapiens
<400> 108
   taaatctgga gccggtcgag cgtgggtctc gcgtatcatt cgagcactgg ggccagatgg 60
   taagccctcc gtatcgtagt tatctcacag cagggagtca gg 102
<210> 109
   <211> 110
   <212> DNA
   <213> Homo sapiens
<400> 109
   tagaaccgcc gctggcactg gagcagtggg tccctggtct cctacaagtc ctggggcatt 60
   ggagccccaa gcagtgttaa tcctggctac tgtgtgagcc tgacctcaag 110
<210> 110
   <211> 85
   <212> DNA
   <213> Homo sapiens
<400> 110
   atagatatct actttattcg atttaaattc tgtttagtat tttattatat tttgttaatc 60
   catttgtccc aattcatata cttat 85
<210> 111
   <211> 95
   <212> DNA
   <213> Homo sapiens
<400> 111
   atagatagct acttaaaata acttacacac tgttttagag tgcttgaaaa ctatctgatc 60
   agacatagta attgaaacca atgaatacat tatat 95
<210> 112
   <211> 90
   <212> DNA
   <213> Rattus sp.
<220>
   <221> misc_feature
   <222> (85)..(86)
   <223> n is a, c, g, or t
<220>
   <221> misc_feature
   <222> (89)..(89)
   <223> n is a, c, g, or t
<400> 112
   gaaggatctg tttgtaagaa cggagcggga taccataaaa atagaggtaa taacatacat 60
   tgggacgtgt aaatttattt ttatnnaant 90
<210> 113
   <211> 94
   <212> DNA
   <213> Homo sapiens
<400> 113
   gaaggatctt accattttaa gaaaggtcca aaattaaata aaaatttaat tagtctcttt 60
   agtgttgcat accatcaata tcggaatact aaat 94
<210> 114
   <211> 200
   <212> DNA
   <213> Homo sapiens
<400> 114
   acctggtgct gtgctcaccc aggccgaggg tgcctttgtg gcctcactga ttaaggagag 60
   tggcatgatg acttcaatgt ctggattggc ctccatgacc ccaaaaagaa ccgccgctgg 120
   ggagcgtggg tccctggtct cctacaagtc ctggggcatt ggagccccaa gcagtgttaa 180
   tcctggctac tgtgtgagcc 200
<210> 115
   <211> 218
   <212> DNA
   <213> Homo sapiens
<400> 115
   aactggcgaa ctacttactc tagcttccgg caacaattaa tagactggat ggaggcggat 60
   aaagttgcag gaccacttct gcgctcggcc cttccggctg gctggtttat tgctgataaa 120
   tctggagccg gtgagcgtgg gtctcgcgta tcattgcagc actggggcca gatggtaagc 180
   cctccgtatc gtggttatct acacgacggg gagtacgc 218
<210> 116
   <211> 146
   <212> DNA
   <213> Homo sapiens
<400> 116
   aggcccatca tcagttttta tataaagaaa aaaaaacctt aaaattgtta ggcaaatact     60
   atgacaaatt gtaatatata ttcttacatt tcagattttt attttttaaa ctgtatagaa    120
   ttgattaata aataaaattt agtatt                                          146
<210> 117
   <211> 155
   <212> DNA
   <213> Homo sapiens
<400> 117
   aggcacgatg gccgctttgg tccggatctt tgtgaaggaa ccttacttct gtgtgtgaca     60
   taattggaca aactacctac agagatttaa acgtctaagg taaatataaa atttttagtg    120
   tataggttaa actactgatt ctaatgttgt gtatt                               155
<210> 118
   <211> 102
   <212> DNA
   <213> Homo sapiens
<400> 118
   taaatctgga gccggtgagc gtgggtctcg cgtatcattg cagcactggg gccagatggt     60
   aagccctccg tatcgtggtt atctacacga cggggagtac gg                       102
<210> 119
   <211> 116
   <212> DNA
   <213> Homo sapiens
<400> 119
   tagaaccgcc gctggcactg gagcagtggg tccctggtct cctacaagtc ctggggcatt     60
   ggagccccaa gcagtgttaa tcctggctac tgtgtgagcc tgacctcaag cacagg        116
<210> 120
   <211> 6
   <212> PRT
   <213> Unknown
<220>
   <223> Unknown Peptide
<400> 120
   His Pro Thr Pro Ala Ala
   1               5
<210> 121
   <211> 7
   <212> PRT
   <213> Unknown
<220>
   <223> Unknown peptide
<400> 121
   Phe Val Ala Ser Leu Ile Lys
   1               5

### SEQUENCE LISTING

<110> The General Hospital Corporation de la Monte, Suzanne Wands, Jack R.
<120> Neural Thread Protein Gene Expression and Detection of Alzheimer's Disease
<130> P19088EP-1/PWC
<140> EP 04076254.4
   <141> 1994-04-20
<150> US 08/050,559
   <151> 1993-04-20
<160> 121
<170> PatentIn version 3.2
<210> 1
   <211> 21
   <212> DNA
   <213> Homo sapiens
<400> 1
   ccgattccaa cagaccatca t 21
<210> 2
   <211> 21
   <212> DNA
   <213> Homo sapiens
<400> 2
   ccaacagacc atcattccac c 21
<210> 3
   <211> 21
   <212> DNA
   <213> Homo sapiens
<400> 3
   ccaaaccgat tccaacagac c 21
<210> 4
   <211> 30
   <212> DNA
   <213> Homo sapiens
<400> 4
   cctgggcaac aagagcgaaa actccatctc 30
<210> 5
   <211> 30
   <212> DNA
   <213> Homo sapiens
<400> 5
   atcgcttgaa cccgggaggc ggaggttgcg 30
<210> 6
   <211> 30
   <212> DNA
   <213> Homo sapiens
<400> 6
   ggggaggctg aggcaggaga atcgcttgaa 30
<210> 7
   <211> 43
   <212> DNA
   <213> Artificial Sequence
<220>
   <223> Triple helix-forming oligonucleotide
<220>
   <221> misc_feature
   <222> (1)..(21)
   <223> This region is in the 3' to 5' orientation
<220>
   <221> misc_feature
   <222> (22)..(22)
   <223> May be a 0-10 base oligonucleotide linkage between oligonucleotides
<400> 7
   tactaccaga caaccttagc cnccgattcc aacagaccat cat 43
<210> 8
   <211> 43
   <212> DNA
   <213> Artificial Sequence
<220>
   <223> Triple helix-forming oligonucleotide
<220>
   <221> misc_feature
   <222> (22)..(22)
   <223> May be a 0-10 base oligonucleotide linkage between oligonucleotides
<220>
   <221> misc_feature
   <222> (23)..(43)
   <223> This region is in the 3' to 5' orientation
<400> 8
   ccgattccaa cagaccatca tntactacca gacaacctta gcc 43
<210> 9
   <211> 43
   <212> DNA
   <213> Artificial Sequence
<220>
   <223> Triple helix-forming oligonucleotide
<220>
   <221> misc_feature
   <222> (1)..(21)
   <223> This region is in the 3' to 5' orientation
<220>
   <221> misc_feature
   <222> (22)..(22)
   <223> May be a 0-10 base oligonucleotide linkage between oligonucleotides
<400> 9
   ccaccttact accagacaac cnccaacaga ccatcattcc acc 43
<210> 10
   <211> 43
   <212> DNA
   <213> Artificial Sequence
<220>
   <223> Triple helix-forming oligonucleotide
<220>
   <221> misc_feature
   <222> (22)..(22)
   <223> May be a 0-10 base oligonucleotide linkage between oligonucleotides
<220>
   <221> misc_feature
   <222> (23)..(43)
   <223> This region is in the 3' to 5' orientation
<400> 10
   ccaacagacc atcattccac cnccacctta ctaccagaca acc 43
<210> 11
   <211> 43
   <212> DNA
   <213> Artificial Sequence
<220>
   <223> Triple helix-forming oligonucleotide
<220>
   <221> misc_feature
   <222> (1)..(21)
   <223> This region is in the 3' to 5' orientation
<220>
   <221> misc_feature
   <222> (22)..(22)
   <223> May be a 0-10 base oligonucleotide linkage between oligonucleotides
<400> 11
   ccagacaacc ttagccaaac cnccaaaccg attccaacag acc 43
<210> 12
   <211> 43
   <212> DNA
   <213> Artificial Sequence
<220>
   <223> Triple helix-forming oligonucleotide
<220>
   <221> misc_feature
   <222> (22)..(22)
   <223> May be a 0-10 base oligonucleotide linkage between oligonucleotides
<220>
   <221> misc_feature
   <222> (23)..(43)
   <223> This region is in the 3' to 5' orientation
<400> 12
   ccaaaccgat tccaacagac cnccagacaa ccttagccaa acc 43
<210> 13
   <211> 1443
   <212> DNA
   <213> Homo sapiens
<400> 13
   cgctgcgccc aggctggctc tggaaagcct gtgcggtcct ggcaggaagc ccggcccgtg     60
   gagcaggttt tcgttctgct tcagcaataa ataagggtga ccacagggac tttgcttttg    120
   gtttcctttc ctgtgaaaag gttggtttta aagtgagata cacttttccg tagaacaagt    180
   gttctatctt taaaaaccca aattgcagca ccgtggatta ctggtctcag aacaactcat    240
   tgcgcatcag atttgactct ctgattttct gtctattggc caaattgccc tttaactgca    300
   cctgaatcct ttgtgtactg atgcctttga gctgggcacc ttgggagagt gttgtgttgc    360
   tgtttacggt tcttccttcc ccttgctaat tacagtctct ggtgcccagc aagccccttt    420
   ggcttccttc cgtgactggt cacgttgtct gcctgggctc agcgtggacc tgccccatgc    480
   tgcagaacct ggcctcacct ggacttttac tagaattgcc agcttctcaa cttagcagat    540
   catcactcat gcgggcacaa gcaaagatca acactttctt ttttggtaag cttgagtttt    600
   acaagttatt ttttggtgat gcgtaagaca ttgcagtggg aaaccattca acttgagttt    660
   attggagttt gctgttgtag caggttttaa ctcaggaaca actcttgtct gatctctcgc    720
   ccctctgccg ggactacatt actgtctctc ggagccggta gcgttgctgt cgagtcccag    780
   gactatctct gcagactgct atgctcagat cgaagtattt cacaagaata cttgtgtttt    840
   taacagccct tcccctggac ggtgcgccat gagggcctca tgttacgcat tgccttttct    900
   ttctgtggat ccagtatctt cctcggcttt ttagggagca ggaaaaatgc gtctgagagc    960
   aactcttttt aaaaacctgc cctgttgtat ataactgtgt ctgtttcacc gtgtgacctc   1020
   caagggggtg ggaacttgat ataaacgttt aaaggggcca cgatttgccc gagggttact  1080
   cctttgctct caccttgtat ggatgaggag atgaagccat ttcttatcct gtagatgtga   1140
   agcactttca gttttcagcg atgttggaat gtagcatcag aagctcgttc cttcacactc   1200
   agtggcgtct gtgcttgtcc acatgcgctg ggcgtctgga ccttgaatgc ctgccctggt   1260
   tgtgtggact ccttaatgcc aatcatttct tcacttctct ggacacccag ggcgcctgtt   1320
   gacaagtgtg gagaaactcc taatttaaat gtcacagaca atgtcctagt gttgactact   1380
   acaatgttga tgctacactg ttgtaattat taaactgatt atttttctta tgtcaaaaaa   1440
   aaa                                                                 1443
<210> 14
   <211> 213
   <212> DNA
   <213> Homo sapiens
<400> 14
   gatcccgttt gacaggtgta ccgccccagt caaactcccc acctggcact gtccccggag 60
   cggtcgcgcc cgcgcgacca cggagctctg ggcgccagaa gcgagagccc ctcgctgccc 120
   cccgcctcac cgggtagtga aaaaacgatg agagtagtgg tatttcaccg gcggcccgcg 180
   aggacccccg cccgacccag tgcggaacgg ggg 213
<210> 15
   <211> 358
   <212> DNA
   <213> Homo sapiens
<220>
   <221> misc_feature
   <222> (56)..(56)
   <223> n is a, c, g, or t
<400> 15
   caccgtggat tactggtctc agaacaactc attgcgcatc agatttactc tctgantttc 60
   tgtctattgg ccattgccct ttaactgcac ctgaatcctt tgtgtactga tcctttgagc 120
   tgggcacctt gggagagtgt tgtgttgctg tttacggttc ttccttcccc ttgctaatta 180
   cagtctctgg tgccagcaag cccctttggc ttccttccgt gactggtcac gttgtctgcc 240
   tgggccagcg tggccccatg ctgcagaacc tggcctcagg acttttcact agaattgccc 300
   ttcctcaact tagcagatca ttcactcatg cgggcacaag caaagatcaa cactttct 358
<210> 16
   <211> 378
   <212> DNA
   <213> Homo sapiens
<220>
   <221> misc_feature
   <222> (242)..(242)
   <223> n is a, c, g, or t
<400> 16
   cattgcagct cagcatggct cagaccagct catacttcat gctgatctcc tgcctgatgt 60
   ttctgtctca gagccaaggc caagaggccc agacagagtt gccccaggcc cggatcagct 120
   gcccagaagg caccaatgcc tatcgctcct actgctacta ctttaatgaa gaccgtgaga 180
   cctgggttga tgcagatctc tattgccaga acatgaattc gggcaacctg gtgtctgtgc 240
   tncccaggcc gagggtgcct ttgtggcctc actgattaag gagagtggca ctgatgactt 300
   caatgtctgg attggcctcc atgaccccaa aaagaaccgc cgctggcact ggagcagtgg 360
   gtccctggtc tcctacaa 378
<210> 17
   <211> 142
   <212> DNA
   <213> Homo sapiens
<400> 17
   ttttcctaga acaagggttc tatctttaaa aacccaaatt gcagcaccgc tggtctcaga 60
   acaactcatt gcgcatcaga tttactctct gatttttctg tctatttggc caaattgccc 120
   ttttaactgc acctgaatct tt 142
<210> 18
   <211> 151
   <212> DNA
   <213> Homo sapiens
<400> 18
   ttctcctata gagattgttg atttgcctct tagcaagaga ttcattgcag ctcagcatgg 60
   ctcagaccag ctcatacttc atgctgatct cctgcctgat gtttctgtct cagagccaag 120
   gtaagatctc ttttccacca accaactctt t 151
<210> 19
   <211> 75
   <212> DNA
   <213> Homo sapiens
<400> 19
   caccgtggat tactggtctc agaacaactc attgcgcatc agatttactc tctgattttt 60
   ctgtctattg gccaa 75
<210> 20
   <211> 77
   <212> DNA
   <213> Homo sapiens
<400> 20
   cattgcagct cagcatggct cagaccagct catacttcat gctgatctcc tgcctgatgt     60
   ttctgtctca gagccaa                                                    77
<210> 21
   <211> 130
   <212> DNA
   <213> Homo sapiens
<400> 21
   gaattcctgg gctcaagtga tcctctcatg cagtctccca aagtgctggg atgacaggct     60
   tgagccacca caccaggccc atcatcagtt tatataaaga aaaaaaaacc ttaaaattgt    120
   taggcaaata                                                           130
<210> 22
   <211> 144
   <212> DNA
   <213> Homo sapiens
<400> 22
   ggagttttgt catcaggcca gcctcatccc gaggtctcct ccaccattgg ccgtagccag     60
   caggttcagt gctcaccgaa agtaaaatcc cctccttcag caagaataaa gcaatataca    120
   ccttaggttc cactaagtaa cata                                           144
<210> 23
   <211> 96
   <212> DNA
   <213> Homo sapiens
<400> 23
   gttctgtgag tctcaatttg ttccttcttg gaagctgtct ggtgaatctg ttggtccctc     60
   tgtctgctat tctgtctgtc tgtatgtctg tccatg                               96
<210> 24
   <211> 105
   <212> DNA
   <213> Homo sapiens
<400> 24
   gttgatttgc ctcttaagca agagattcat tgcagctcag catggctcag accagctcat     60
   acttcatgct gatctcctgc ctgatgtttc tgtctcagag ccaag                    105
<210> 25
   <211> 215
   <212> DNA
   <213> Homo sapiens
<400> 25
   actaccaaac ctgcattaaa aaatttcggt tggtcgacct cggagcagaa cccaacctcc      60
   gagcagtaca tgctaagact tcaccagtca aagcgaacgt actatactca attgatccaa     120
   taacttgacc aacggaacaa gttaccctat aacagcgcaa tcctattcta gagtccatat     180
   caacagggtt tacgacctcg atgttggatc aggac                                215
<210> 26
   <211> 232
   <212> DNA
   <213> Homo sapiens
<400> 26
   accacggagc tctgggcgcc agaagcgaga gcccctcgct gccccccgcc tcaccgggta      60
   gtgaaaaaac gatgagagta gtggtatttc accggcggcc cgcgaggacc cccgcccgac     120
   ccagtgcgga acgggggagt agtcccgggg gctcacttat tctacattag tctcacgtgc     180
   agactagagt caagctcaac agggtcttct ttcccgctga ttccgccaag tc             232
<210> 27
   <211> 112
   <212> DNA
   <213> Homo sapiens
<400> 27
   agtttcactc tgttgcccag gctggagtgc aatggcacaa tcctggctca ctgcaacctc      60
   cgcctcccga gctcaagcaa ttctcctgcc tcagcctcgt gagccgctgg ga             112
<210> 28
   <211> 120
   <212> DNA
   <213> Homo sapiens
<400> 28
   agatctcgct ctgtcaccca ggctgaagtg cagtggccca atctcggctc actgcgagct      60
   ccacctcccg ggttcacttc attctcctgc ctcactgcct cagcctctga gtagctggga     120
<210> 29
   <211> 594
   <212> DNA
   <213> Homo sapiens
<400> 29
   gaggcgtatt ataccatgct ccatctgcct acgacaaaca gacctaaaat cgctcattgc     60
   atactcttca atcagccaca tagccctcgt agtaacagcc attctcatcc aaaccccctg   120
   aagcttcacc ggcgcagtca ttctcataat cgcccacggg cttacatcct cattactatt    180
   ctgcctagca aactcaaact acgaacgcac tcacagtcgc atcataatcc tctctcaagg    240
   acttcaaact ctactcccac taatagcttt ttgatgactt ctagcaagcc tcgctaacct    300
   cgccttaccc cccactatta acctactggg agaactctct gtgctagtaa ccacgttctc    360
   ctgatcaaat atcactctcc tacttacagg actcaacata ctagtcacag ccctatactc    420
   cctctacata tttaccacaa cacaatgggg ctcactcacc caccacatta acaacataaa    480
   accctcattc acacgagaaa acaccctcat gttcatacac ctatccccca ttctcctcct    540
   atccctcaac cccgacatca ttaccgggtt ttcctcttaa aaaaaaaaaa aaaa          594
<210> 30
   <211> 98
   <212> PRT
   <213> Homo sapiens
<400> 30
   Glu Ala Tyr Tyr Thr Met Leu His Leu Pro Thr Thr Asn Arg Pro Lys
   1               5                   10                  15
   Ile Ala His Cys Ile Leu Phe Asn Gln Pro His Ser Pro Arg Ser Asn
               20                  25                  30
   Ser His Ser His Pro Asn Pro Leu Lys Leu His Arg Arg Ser His Ser
           35                  40                  45
   His Asn Arg Pro Arg Ala Tyr Ile Leu Ile Thr Ile Leu Pro Ser Lys
       50                  55                  60
   Leu Lys Leu Arg Thr His Ser Gln Ser His His Asn Pro Leu Ser Arg
   65                  70                  75                  80
   Thr Ser Asn Ser Thr Pro Thr Asn Ser Phe Leu Met Thr Ser Ser Lys
                   85                  90                  95
   Pro Arg
<210> 31
   <211> 554
   <212> DNA
   <213> Homo sapiens
<220>
   <221> misc_feature
   <222> (176)..(176)
   <223> n is a, c, g, or t
<400> 31
   ataccatgct ccatctgcct acgacaaaca gacctaaaat cgctcattgc atactcttca     60
   atcagcacat agccctcgta gtaacagcca ttctcatcca aaccccctga agcttcaccg    120
   gcgcagtcat tctcataatc gcccacgggc ttacatcctc attactattc tgccancaaa    180
   ctcaaactac gaacgcactc acagtcgcat cataatctct ctcaaggact tcaaactcta    240
   ctcccaagct ttgtgacttc tagcaacctc gctaacctcg ccttaccccc actattaact    300
   actgggagaa tgtgctagta accacgttct ccttcaaata tcactctcct acttacagga    360
   ctcaacatac tagtccagcc ctatactccc tctacatatt taccacaaca caatgggctc    420
   actcacccac cacattaacc ataaaaccct cattcacacg agaaaacacc ctcatgttca    480
   tacacctatc ccccattctt cctatccctc aaccccgaca tcaaccgggt ttcctcttaa    540
   aaaaaaaaaa aaaa                                                      554
<210> 32
   <211> 590
   <212> DNA
   <213> Homo sapiens
<400> 32
   atgcctatcg ctcctactgc tactacttta atgaagaccg tgagacctgg gttgatgcag     60
   atctctattg ccagaacatg aattcgggca acctggtgtc tgtgctcacc caggccgagg    120
   gtgcctttgt ggcctcactg attaaggaga gtggcactga tgacttcaat gtctggattg    180
   gcctccatga ccccaaaaag aaccgccgct ggcactggag cagtgggtcc ctggtctcct    240
   acaagtcctg ggcattggga gccccaagca gtgttaatcc tggctactgt gtgagcctga    300
   cctcaagcac aggattccag aaatggaagg atgtgccttg tgaagacaag ttctcctttg    360
   tctgcaagtt caaaaactag aggcagctgg aaaatacatg tctagaactg atccagcaat    420
   tacaacggag tcaaaaatta aaccggacca tctctccaac tcaactcaac ctggacactc    480
   tcttctctgc tgagtttgcc ttgttaatct tcaatagttt tacctacccc agtctttgga    540
   accttaaata ataaaaataa acatgtttcc actaaaaaaa aaaaaaaaaa 590
<210> 33
   <211> 466
   <212> DNA
   <213> Homo sapiens
<400> 33
   cttctttttc aggccaagag gcccagacag agttgcccca ggcccggatc agctgcccag 60
   aaggcaccaa cctatcgctc ctactgctac tactttaatg aagaccgcga gacctgggtt 120
   gatgcagtgt gagtgaggag agcgtgtggg aagggagact catgaaggga ggggaagctg 180
   ccactctcca gtgttcagtg gcgcaatgag atgagactga acccctttat actatcatca 240
   gccccaaact ttccaatcta ctttatccca ttattcagca cattcccagc acaaagaacc 300
   tggtgggtga cagcatcatc acggacatta ctctgctgtc ctttttcacc ctcctcttgg 360
   aggactcagt atatccgtca caaccctcca ctgagtctcc attttcttct gcaacagctc 420
   tattgccaga acatgaattc gggcaacctg gtgtctgtgc tcaccc 466
<210> 34
   <211> 501
   <212> DNA
   <213> Homo sapiens
<220>
   <221> misc_feature
   <222> (123)..(123)
   <223> n is a, c, g, or t
<400> 34
   cgtattatac catgctccat ctgcctacga caaacagacc taaaatcgct cattgcatac 60
   tcttcaatca gccacatagc cctcgtagta acagccattc tcatccaaac cccctgaagc 120
   ttnccggcgc agtcattctc ataatcgccc acgggcttac atcctcatta ctattctgcc 180
   tagcaaactc aaactacgaa cgcactcaca gtcgcatcat aatcctctct caaggacttc 240
   aaactctact cccactaata gctttttgat gacttctagc aagcctcgct aacctcgcct 300
   taccccccac tattaaccta ctgggagaac tctctgtgct agtaaccacg ttctcctgat 360
   caaatatcac tctcctactt acaggactca acatactagt cacagcccta tactccctct 420
   acatatttac cacaacacaa tggggctcac tcacccacca cattaacaac ataaaaccct 480
   cattcacacg agaaaacacc c 501
<210> 35
   <211> 372
   <212> DNA
   <213> Homo sapiens
<400> 35
   gttcttagtc tatctcttgt acaaacgatg tgctttgaag atgttagtgt ataacaattg      60
   atgtttgttt tctgtttgat tttaaacaga gaaaaaataa aagggggtaa tagctccttt     120
   tttcttcttt cttttttttt ttcatttcaa aattgctgcc agtgttttca atgtaggaca     180
   acagagggat atgctgtaga gtgtttttat tgcctagttg acaaagctgc ttttgaatgc     240
   tggtggttct attcctttgc acatcacgac attttataat catagttaaa tcgtatatga     300
   caaaaatgct ctgatctgat gccaaaggtc aattcagtgt atataacctg aacacactca     360
   tccattgcgt tt                                                         372
<210> 36
   <211> 68
   <212> PRT
   <213> Homo sapiens
<400> 36
   Met Phe Val Phe Cys Leu Ile Leu Asn Arg Glu Lys Ile Lys Gly Gly
   1               5                   10                  15
   Asn Ser Ser Phe Phe Leu Leu Ser Phe Phe Phe Ser Phe Gln Asn Cys
               20                  25                  30
   Cys Gln Cys Phe Gln Cys Arg Thr Thr Glu Gly Tyr Ala Val Glu Cys
           35                  40                  45
   Phe Tyr Cys Leu Val Asp Lys Ala Ala Phe Glu Cys Trp Trp Phe Tyr
       50                  55                  60
   Ser Phe Asp Thr
   65
<210> 37
   <211> 377
   <212> DNA
   <213> Homo sapiens
<220>
   <221> misc_feature
   <222> (59)..(59)
   <223> n is a, c, g, or t
<400> 37
   actgtctccc cctttgatag ggacactaaa gtggtctgta cttgggtaga ggatggcang      60
   ttaagaatta aaatcgtctg ggtgcggtct gcacgcttgt aatcccagca ctttgggagg     120
   ctgaggcggg cggatcacct gaggtcagga gttcgacacc agcctgatga acatggagaa     180
   accccatctc tactaaaaat acaaatatta gctgggcgtt gtcgcgcgcc tgtaatccca     240
   gcggctcacg aggctgaggc aggagaattg cttgagctcg ggatggcgga ggttgcagtg     300
   agccaggatt gtgccattgc actccagcct gggcaacaag agtgaaactc tgtctcaaaa     360
   aaaaaaaaaa aaaaaaa                                                     377
<210> 38
   <211> 1480
   <212> DNA
   <213> Homo sapiens
<400> 38
   gcgtaaacac atttttgttc ttagtctatc tcttgtacaa acgatgtgct ttgaagatgt     60
   tagtgtataa caattgatgt ttgttttctg tttgatttta aacagagaaa aaataaaagg    120
   gggtaatagc tccttttttc ttctttcttt gattttaaac agagaaaaaa taaaaggggg    180
   taatagctcc ttttttcttc tttctttttt ttttttcatt tcaaaattgc tgccagtgtt    240
   ttcaatgatg gacaacagag ggatatgctg tagagtgttt tattgcctag ttgacaaagc    300
   tgctttgaat gctggtggtt ctattccttt gacactacgc acttttataa tacatgttaa    360
   tgctatagga caagatgctc tgattcctga gtgccagagg ttcaattcag tgtatataac    420
   tgaacacact catccatttg tgcttttgtt ttttttatgg tggcttaaag gtaaagagcc    480
   catcctttgc aagtcatcca tgttgttact taggcatttt atcttggctc aaattgttgg    540
   aagaatggtg gcttgtttca tggtttttgt atttgtgtct aatgcacgtt ttaacatgat    600
   agacgcaatg cattgtgtag ctagttttct ggaaaagtca actcttttag gaattgtttt    660
   tcagatcttc aataaatttt ttctttaaat ttcaaagaac aatgtgcttg tgttgatgcc    720
   ttacaaaaac cattgtatat ttgtgtattc cttcttgtat ttagacagtg gtttttcagg    780
   tgcgtgcttt gttttctggt atggccttta tggaatgaga cgctttagct ttggtacgta    840
   gcgctaatcc atagcagctt tggcagtttc gtgtcttgag tcttagctaa aaagttagaa    900
   gtttacatga ctgttttttt tattttccct aaattattac ttactctgag cattaattaa    960
   gggcattttc acctgtgtaa aattatggtc agcttttttc tgtctataat tgtttacttt   1020
   tgtgggttta ctctagaaac atgagccaaa aatgtcaata gacaacacag tattaaaata   1080
   acccaaaagt tgtaaagggc aacgtttctc ccctttgata gggacactaa agtggtctgt   1140
   acttgggtag aggatggcag acgttaagaa ttaaaatgcg tctgggtgcg gtctcacgct   1200
   tgtaatccca gcactttggg aggctgaggc gggcggatca cctgaggtca ggagttcgac   1260
   accagcctga tgaacatgga gaaaccccat ctctactaaa aatacaaata ttagctgggc   1320
   gttgtcgcgc gcctgtaatc ccagcggctc acgaggctga ggcaggagaa ttgcttgagc   1380
   tcgggatggc ggaggttgca gtgagccagg attgtgccat tgcactccag cctgggcaac   1440
   aagagtgaaa ctctgtctca aaaaaaaaaa aaaaaaaaaa                         1480
<210> 39
   <211> 381
   <212> DNA
   <213> Homo sapiens
<400> 39
   ttgggtgagg tggaatgatg gtctgttgga atcggtttgg taaatgggtt tatttcatat     60
   ccgctatctt taactttgga ccgcgttatc tatatcatgg cgttcctttc tactttttaa    120
   tattggttcg tattatatcg ttcctgattg gggatatgga agacgtatta cttaattgta    180
   ctttattgaa acgttcctct cggtttcgat tctgggggct ttggtctgct cgatggattc    240
   ttgtcgattt tctcgtgtgg cagtaacata ccgttttatc acccttctaa atatcccatc    300
   tcccgctgtt tggtaggctc ggaacactat cgaccaacag gttctatcta gaatcaagtt    360
   ggaaattaaa cggtgtcttg g                                              381
<210> 40
   <211> 122
   <212> PRT
   <213> Homo sapiens
<400> 40
   Met Met Val Cys Trp Asn Arg Phe Gly Lys Trp Val Tyr Phe Ile Ser
   1               5                   10                  15
   Ala Ile Phe Asn Phe Gly Pro Arg Tyr Leu Tyr His Gly Val Pro Phe
               20                  25                  30
   Tyr Phe Leu Ile Leu Val Arg Ile Ile Ser Phe Leu Ile Gly Asp Met
           35                  40                  45
   Glu Asp Val Leu Leu Asn Cys Thr Leu Leu Lys Arg Ser Ser Arg Phe
       50                  55                  60
   Arg Phe Trp Gly Ala Leu Val Cys Ser Met Asp Ser Cys Arg Phe Ser
   65                  70                  75                  80
   Arg Val Ala Val Thr Tyr Arg Phe Ile Thr Leu Leu Asn Ile Pro Ser
                   85                  90                  95
   Pro Ala Val Trp Met Ala Arg Asn Thr Ile Asp Gln Gln Val Leu Ser
               100                 105                 110
   Arg Ile Lys Leu Glu Ile Lys Arg Cys Leu
           115                 120
<210> 41
   <211> 420
   <212> DNA
   <213> Homo sapiens
<400> 41
   cccacaggtc ctaaactacc aaacctgcat taaaaaattt cggttggtcg acctcggagc 60
   agaacccaac ctccgagcag tacatgctaa gacttcacca gtcaaagcga acgtactata 120
   ctcaattgat ccaataactt gaccaacgga acaagttacc ctagggataa cagcgcaatc 180
   cgatggtgca gccgctatta aaggttcgtt tgttcaaacg attaaagtcc tcgtgtctga 240
   gttcagaccg aagtaatcca ggtcggtttc tatcttcttc aaattcctcc ctgtaccgaa 300
   aggactaatg agaaataagg cctacttcac aaagcggcct tcccccgtaa tgatatcatc 360
   tcaacttagt attataccca cacccaccca agaacaggtt tgttaaaaaa aaaaaaaaaa 420
<210> 42
   <211> 381
   <212> DNA
   <213> Homo sapiens
<400> 42
   aacccactcc accttactac cagacaacct tagccaaacc atttacccaa ataaagtata 60
   ggcgatagaa attgaaacct ggcgcaatag atatagtacc gcaaggaaag atgaaaaatt 120
   ataaccaagc ataatatagc aaggactaac ccctatacct tctgcataat gaattaacat 180
   gaaataactt tgcaaggaga gccaaagcta agacccccga aaccagacga gctacctaag 240
   aacagctaaa agagcacacc gtcattgtat ggcaaaatag tgggaagatt tatagggtag 300
   agggcgacaa accatccgag ccttgtgata gctggttgtc caagatagat cttagttcaa 360
   cctttaattt gccacagaac c 381
<210> 43
   <211> 629
   <212> DNA
   <213> Homo sapiens
<400> 43
   tttttttttt tttttaacaa accctgttct tgggtgggtg tgggtataat actaagttga 60
   gatgatatca ttacggggga aggccgcttt gtgaagtagg ccttatttct cattagtcct 120
   ttcggtacag ggaggaattt gaagaagata gaaaccgacc tggattactt cggtctgaac 180
   tcagacacga ggactttaat cgtttgaaca aacgaacctt taatagcggc tgcaccatcg 240
   ggatgtcctg atccaacatc gaggtcgtaa accctattgt tgatatggac tctagaatag    300
   gattgcgctg ttatccctag ggtaacttgt tccgttggtc aagttattgg atcaattgag    360
   tttagtagtc cgcttggagt ggtgaagtct agaatgtcct gttcgggggt tggtttctgc    420
   tcccaggtcg ccccaaccga attttttatt gaaggttggg tagtttagca cctgtgggtt    480
   ggtaaggtac tgttggaatt aataaattaa agctccatag ggtctcctcg tcttgttgtg    540
   taatgccccc ctctccacgg gaaggtcaat tccactggtt aaaagtaaga gaaagctgaa    600
   ccctcgggga gccatccata caggtcccc                                      629
<210> 44
   <211> 256
   <212> DNA
   <213> Homo sapiens
<400> 44
   gcgggtaaat tggtttgtta ttttttaaaa aaaacttgca tgtttaaaaa aaagttgatt     60
   gcttcaaatt tctgctacta acttcaagct atgggagttt ggcagtagtc acttgaggat    120
   tttttttcca attcttttct ttttgttgtt aaagctgtac ttcagtgaac agaaaaattg    180
   ccaagcaaac taatggacta taaagcgtaa tttgactgtg tgggactaaa ctacagagcc    240
   tacttgacca gtggat                                                    256
<210> 45
   <211> 270
   <212> DNA
   <213> Homo sapiens
<400> 45
   catgtttaaa aaaaagttga ttgcttcaaa ttactgctac taacttcaag ctatgggagt     60
   ttggcagtag tcacttgagg attttttttc caattcgttt tcatttttgt tgttaaagct    120
   cgtacttcag tgagacagaa aaattgccaa gctaaactaa tggtctataa aagcgtaatt    180
   tgcatgtgtg ggcaaaaact acagagcctc aattgccact gaggtatagt acaaagtttt    240
   aatacatttt gtaaatcaaa ttgaaagaaa                                     270
<210> 46
   <211> 270
   <212> DNA
   <213> Homo sapiens
<400> 46
   catgtttaaa aaaaagttga ttgcttcaaa ttactgctac taacttcaag ctatgggagt     60
   ttggcagtag tcacttgagg attttttttc caattcgttt tcatttttgt tgttaaagct    120
   cgtacttcag tgagacagaa aaattgccaa gctaaactaa tggtctataa aagcgtaatt    180
   tgcatgtgtg ggcaaaaact acagagcctc aattgccact gaggtatagt acaaagtttt    240
   aatacatttt gtaaatcaaa ttgaaagaaa                                     270
<210> 47
   <211> 184
   <212> DNA
   <213> Homo sapiens
<400> 47
   tctgcccagg ctggtctgaa attcctgggc tgaagtgatc ctccagtctt ggcctcccaa     60
   agtgctggga ttacaggcat gagctactga gcctagcctt aatgattaat tttagagtga    120
   tggcttgtac cttcaagaca catatagatt gagacagaaa atttccatcg tccccgagaa    180
   aact                                                                 184
<210> 48
   <211> 75
   <212> PRT
   <213> Homo sapiens
<400> 48
   Ser Ser Ser Leu Gly Leu Pro Lys Cys Trp Asp Tyr Arg His Glu Leu
   1               5                   10                  15
   Leu Ser Leu Ala Leu Met Ile Asn Phe Arg Val Met Ala Cys Thr Phe
               20                  25                  30
   Lys Gln His Ile Glu Leu Arg Gln Lys Ile Ser Ile Val Pro Arg Lys
           35                  40                  45
   Leu Cys Cys Met Gly Pro Val Cys Pro Val Lys Ile Ala Leu Leu Thr
       50                  55                  60
   Ile Asn Gly His Cys Thr Trp Leu Pro Ala Ser
   65                  70                  75
<210> 49
   <211> 1381
   <212> DNA
   <213> Homo sapiens
<400> 49
   tttttttttt gagatggagt tttcgctctt gttgcccagg ctggagtgca atggcgcaat     60
   ctcagctcac cgcaacctcc gcctcccggg ttcaagcgat tctcctgcct cagcctcccc    120
   agtagctggg attacaggca tgtgcaccac gctcggctaa ttttgtattt ttttttagta     180
   gagatggagt ttaactccat gttggtcagg ctggtctcga actcccgacc tcagatgatc    240
   tcccgtctcg gcctgcccaa agtgctgaga ttacaggcat gagccaccat gcccggcctc    300
   tgcctggcta atttttgtgg tagaaacagg gtttcactga tgttgcccaa gctggtctcc    360
   tgagctcaag cagtccacct gcctcagcct cccaaagtgc tgggattaca ggcgtcagcc    420
   gtgcctggcc tttttatttt atttttttta agacacaggt gtaccactct tacccaggat    480
   gaagtgcagt ggtgtgatca cagctcactg cagccttcaa ctcctgagat caagcaatcc    540
   tcctgcctca gcctcccaag tagctgggac caaagacatg caccactaca cctggtaatt    600
   tttattttta tttttaattt tttgagacag agtctcactc tgtcacccag gctggagtgc    660
   agtggcgcaa tcttggctca ctgcaacctc tgcctcccgg gttcaagtta ttctcctgcc    720
   ccagcctcct gagtagctgg gactacaggc gcccaccacg cctagctaat ttttttgtat    780
   ttttagtaga gatggggttt caccatgttc gccaggttga tcttgatctc ttgaccttgt    840
   gatctgcctg cctcggccta cccaaagtgc tgggattaca ggtcgtgact ccacgccggc    900
   ctatttttaa tttttgtttg tttgaaatgg aatctcactc tgttacccag gtcggagtgc    960
   aatggcaaat ctcggctact cgcaacctct gcctcccggg tcaagcgatt ctcctgtctc   1020
   agcctcccaa gcagctggga ttacgggacc tgcaccacac cccgctaatt tttgtatttt   1080
   cattagaggc gggtttacca tatttgtcag gctgggtctc aaactcctga cctcaggtga   1140
   cccacctgcc tcagccttcc aaagtgctgg gattacaggc gtgagccacc tcacccagcc   1200
   ggctaatttg gaataaaaaa tatgtagcaa tgggggtctg ctatgttgcc caggctggtc   1260
   tcaaacttct ggcttcagtc aatccttcca aatgagccac aacacccagc cagtcacatt   1320
   ttttaaacag ttacatcttt attttagtat actagaaagt aatacaataa acatgtcaaa   1380
   c                                                                   1381
<210> 50
   <211> 2520
   <212> DNA
   <213> Homo sapiens
<400> 50
   ccattgttag gttgtctctt acctgttaaa atcaggagct gacaagaaat gcttaccaca     60
   aaaggagaaa tgccagtcta gttaacagtc aaggagagaa atcaggaaga ttatgtgggt    120
   ggaagaagta gatgatgtgg ctgatgagtg agtgagtgag caagcctccg cccagctgaa    180
   gaaggagtca gaactgccct ttgttcccaa ctatttggcg aaccccagcc ttccctttta    240
   tctatacacc cacagcagag gattcagccc agatgcagaa tgggggcccc tccacacccc    300
   ctgcatcacc ccctgcagat ggctcacctc cattgcttcc ccctgggaac ctcccctgtt    360
   agggaccttt ccccgggacc acacctcttt ggcactagtt cagaatggtg atgtgtcggc    420
   ccctctgcca tactagaaca ccagaaagac aaacgggtga tgtttgtcag ctacagtgag 480
   tctagagccg tcctgttttc ttctgtcccg tcccaagcca ccatgtctct tcgagcctca 540
   aaatgggacg tatgcaggac cagcgcccag attccaagcc atttttcttc actggagcat 600
   ttccatttaa tatgcaagag ctggtactca aggtgagaat tcagaaccca tctcttcgag 660
   aaaatgattt cattgaaatt gaactggacc gacagagctc acctaccaag agttgctcag 720
   agtgtgttgc tgtgagctgg gtgttaatcc agatcaagtg gagaagatca gaaagttacc 780
   caatactctg ttaaggaagg acaaggatgt tgctcgactc aagatttcag gagctggaac 840
   tggttctgat gatagtgaaa ataattttct gttcagaaat gctgcatcac actgactgaa 900
   aggccttgct atacaggaga gcttcaaaac tgacttacta atgcagcagg gacttttata 960
   ctgagtatat gacagtgtgc atcacctctg ggccaaggac aagccatgat ctaaatgcct 1020
   cagatgcccg ggccagtctg gtgcactgca tagtatatac gaacatcatt ctgcccaagg 1080
   taggaagccc catgaccccc aagcagtggt gtccactctt ccaagcctct tggtgcacaa 1140
   taaaccttat tgcttgaagc tttgaacgac tgtgagaatg gtctggcgag gacgagaacg 1200
   tggaattata tgagtgtctt ttgtatccga gaatgtagag agttctctga agacgacgac 1260
   tgagagagag cggacgctat ttctagccac tcctgttgac agtgcacctg aagggctggg 1320
   atgcgttttt cttggtgttg catgctcaca actctgctga cattgggaac ttatgagaga 1380
   ggaagactcg ggaaagcaca gatactggac agatggattc tggtgtgggg aaagcacaga 1440
   tactggacag atggttctag tgtgacttgt gactgtgagg tttcctataa catatttata 1500
   aatgttcatc aggttcaaaa gtctataaga atacagttcg agactgaatt gcttcgaaat 1560
   acttcgatgt tgggaaccaa aagagctttc cctccctcac tttttccttt gtaacactca 1620
   tgactgcttc tctgtctcga gtcatctctg cattaactcc ccttcgtggt cactagaggg 1680
   ctctctgatg cttctaagac actgcttttt acatgccaca cccaccgcgt agagacaggg 1740
   tctcactatg tggcccaggc tggtctcaaa cttctggcct taagtgatcg tcctgtcctt 1800
   cgcgctcgga agaaagtcgt ggggattaca ggtgtgagcc acccgcccag cccctccctt 1860
   gtgtttcaac caatcggaag tgaatttaac tagatgtagt aacctttttt ttctttgact 1920
   tctaaaaaag ttacagttta ctaataaagt taagtctggt tctgtcctag aggaaataaa 1980
   ttcactatta attcatgtct taagttactt gggttaaaac actttcagcc acccagatta 2040
   attaaagtgg agcagtggag cccctggctg gggagatggg cctccagagg agcagctgca 2100
   ggcatgttct ggctacacag aggcaagcaa gggactggtg tctctggtga gaggtgggtt 2160
   tgatgtatct ctgtcctatg ctggtctctc ttctccttta taaatcctcc tgtggtcact 2220
   gactatcgta tcgcagtgat cagactgcac atagtacggt taggctgagc ttaatgtctt 2280
   aatcatgtca ttcgagagaa gacacgtttt gattcatgct ttgtgtaatt aatcaatcaa 2340
   ggattctttt tttagctttg ttgacgtgta attcacccct cctcctccac tgcatattta 2400
   aagcatgtgt tcacactgtg tgtatacatt cactgcgatt ttttcgtttg ctgcattgct 2460
   tggactgttc ataacatcac aagtattatt caaataaaat attaactgac cgaaaaaaaa 2520
<210> 51
   <211> 141
   <212> DNA
   <213> Homo sapiens
<400> 51
   gaattcctgg gctcaagtga tcctctcatg tcagtctccc aaagtgctgg gatgacaggc 60
   ttgagccacc acaccaggcc catcatcagt ttttatataa agaaaaaaaa accttaaaat 120
   tgttagcaaa atactatgac a 141
<210> 52
   <211> 151
   <212> DNA
   <213> Homo sapiens
<220>
   <221> misc_feature
   <222> (96)..(96)
   <223> n is a, c, g, or t
<400> 52
   gaactcctga cctcaggtga tccgcccgcc tcagcctccc aaagtgctgg gattacaagc 60
   gtgcagaccg cacccagacg attttaattc ttaacntgcc atcctctacc caagtacaga 120
   ccactttagt gtccctatca aaggggagac a 151
<210> 53
   <211> 43
   <212> DNA
   <213> Homo sapiens
<400> 53
   aattctcctg cctcagcctc gtgagccgct gggattacag gcg 43
<210> 54
   <211> 45
   <212> DNA
   <213> Homo sapiens
<400> 54
   aagccaactc agactcagcc aacaggtaag tgggcattac aggag 45
<210> 55
   <211> 143
   <212> DNA
   <213> Rattus sp.
<400> 55
   ctcagttttc actttgatct ggtagatagt tttcgtttca gttgggggag aaggatctgt 60
   ttgtaagaac ggagtgacgg gataccataa aaatagaggt aataacatac attgggacgt 120
   gtaaatttat ttttatggaa gtg 143
<210> 56
   <211> 157
   <212> DNA
   <213> Homo sapiens
<400> 56
   ctcacagttt ccttatcttg gtggtcgtaa gttttcgtcg aaacagttga tcgttatttg 60
   tgagattgtc gtatagggag actaacaggt agtaactttt gtgaccgtcg ttaaaacttt 120
   actttttttt ttctttcttc ttttttcctt cataatg 157
<210> 57
   <211> 60
   <212> DNA
   <213> Homo sapiens
<400> 57
   accacgcccc gctaattttt gtatttttag tagagacagg gtttcaccgt gttggccagg 60
<210> 58
   <211> 60
   <212> DNA
   <213> Homo sapiens
<400> 58
   acaacgccca gctaatattt gtatttttag tagagatggg gtttctccat gttcatcagg 60
<210> 59
   <211> 60
   <212> DNA
   <213> Homo sapiens
<400> 59
   ctggtctgaa attcctgggc tgaagtgatc ctccagtctt ggcctcccaa agtgctggga 60
<210> 60
   <211> 60
   <212> DNA
   <213> Homo sapiens
<400> 60
   ctggtgtcga actcctgacc tcaggtgatc cgcccgcctc agcctcccaa agtgctggga 60
<210> 61
   <211> 26
   <212> DNA
   <213> Homo sapiens
<400> 61
   agacacatat agattgagac agaaaa 26
<210> 62
   <211> 30
   <212> DNA
   <213> Homo sapiens
<400> 62
   agtacagacc actttagtgt ccctatcaaa 30
<210> 63
   <211> 31
   <212> DNA
   <213> Homo sapiens
<400> 63
   agatctcgct ctgtcaccca ggctgaagtg c 31
<210> 64
   <211> 34
   <212> DNA
   <213> Homo sapiens
<400> 64
   agagtttcac tcttgcttgc ccaggctgga gtgc 34
<210> 65
   <211> 59
   <212> DNA
   <213> Homo sapiens
<400> 65
   agtggcccaa tctcggctca ctgcgagctc cacctcccgg gttcacttca ttctcctgc 59
<210> 66
   <211> 60
   <212> DNA
   <213> Homo sapiens
<400> 66
   aatggcacaa tcctggctca ctgcaacctc cgccctcccg agctcaagaa cttctcctgc 60
<210> 67
   <211> 66
   <212> DNA
   <213> Homo sapiens
<400> 67
   ctcagcctct gagtagctgg gactacaggc gcccaccaca agccgctaat ttttgtattt 60
   ttgtag 66
<210> 68
   <211> 67
   <212> DNA
   <213> Homo sapiens
<400> 68
   ctcagcctcg tgagccgctg ggattacagg cgcgcgccac aagcgactaa tatttgtatt 60
   tttgtag 67
<210> 69
   <211> 76
   <212> DNA
   <213> Homo sapiens
<400> 69
   accacgcccc gctaattttt gtatttttag tagagacagg gtttcaccgt gttggccagg 60
   atgctcgatc tcctga 76
<210> 70
   <211> 78
   <212> DNA
   <213> Homo sapiens
<400> 70
   acaacgccca gctaatattt gtatttttag tagagatggg gtttctccat gttcatcagg 60
   ctggtgtcga actcctga 78
<210> 71
   <211> 159
   <212> DNA
   <213> Homo sapiens
<400> 71
   ccccaagcag tgttaatcct ggctactgtg tgagctgacc tcaagcacag gtgaaggcag 60
   agaatccatc cacctgtttc tgttctccct gcttagctcc agggatggaa ctgggactgg 120
   gatagaggaa aggtgaactc ctcattaagg aaatggatg 159
<210> 72
   <211> 170
   <212> DNA
   <213> Homo sapiens
<400> 72
   cccctgttct tgggtgggtt tgggtatatt ctggttgaga tgatatcatt tacgggggaa 60
   ggcgctttgt gaagtaggcc ttatttctct tgtcctttcg tacagggagg atttgaagta 120
   gtagaacgct gttactccgg tctgaactca gtcacgtggc tttatcgttg 170
<210> 73
   <211> 52
   <212> DNA
   <213> Homo sapiens
<400> 73
   gatccaagct acgtacgcgt gcatgcacgt catagctctt ctatagtgtc ac 52
<210> 74
   <211> 53
   <212> DNA
   <213> Homo sapiens
<400> 74
   gatccgagct cggtaccaag ttgatgcata gcttgagtat tctatagtgt cac 53
<210> 75
   <211> 115
   <212> DNA
   <213> Homo sapiens
<400> 75
   gtatgggccc gatagcttat ttagccttta gagcacactg gcggccgtta ctagtggatc 60
   cgagctcggt accaacttga tgcatagctt gagtattcta tagtgtcacc taaat 115
<210> 76
   <211> 120
   <212> DNA
   <213> Homo sapiens
<400> 76
   atatagacaa tataacaata tattgtatac tttagagcac actggcagcc gttactagtg 60
   gatccgagct cggtaccaag ttgatgcata gcttgagtat tctatagtgt cactaatagt 120
<210> 77
   <211> 117
   <212> DNA
   <213> Homo sapiens
<400> 77
   cttaatagat agctacttaa aataacttac acactgtttt agagtgcttg aaaactatct 60
   gatcagacat agtattgaaa ccaatgaata cattatataa agtaaaggaa aggagaa 117
<210> 78
   <211> 137
   <212> DNA
   <213> Homo sapiens
<400> 78
   cttactacca gacaacctta gccaaaccat ttacccaaat aaagtatagg cgatagaaat 60
   tgaaacctgg cgcaatagat atagtaccgc aaggaaagat gaaaaattat aaccaagcat 120
   aatatagcaa ggactaa 137
<210> 79
   <211> 198
   <212> DNA
   <213> Homo sapiens
<400> 79
   ctataaaggt cgttgtcaac gataaagcac gtgactgagt tcagaccgga gtaacagcgt 60
   tctactactt caaatcctcc ctgcgaaagg caagagaaat aaggcctact taagcgcctt 120
   cccccgtaaa tgatatcatc tcaaccagaa tatacccaaa ccccccaaga acaggggagg 180
   aaaagaaaaa aaaaaaaa 198
<210> 80
   <211> 200
   <212> DNA
   <213> Homo sapiens
<400> 80
   ctatagagat tgttgatttg cctcttaagc aagagattca ttgcagctca gcatggctca 60
   gaccagctca tacttcatgc tgatctcctg cctgatgttt ctgtctcaga gccaaggtaa 120
   gatctctttt ccaactcttt ctagccctga agacttcact ctatccccaa gcatacgggt 180
   ctacttgaaa aaaaaaaaaa 200
<210> 81
   <211> 82
   <212> DNA
   <213> Homo sapiens
<400> 81
   cagaaaacta gctacacaat gcattggtct atcatgttaa aacgtgcatt agacacaaat 60
   acaaaaacca tgaaacaagc ca 82
<210> 82
   <211> 90
   <212> DNA
   <213> Homo sapiens
<400> 82
   cagaaaaatt gccaagctaa actaatggtc tataaaagcg taatttgcat gtgtgggcat 60
   aaactacaga gctcatgcta gagtatgcaa 90
<210> 83
   <211> 141
   <212> DNA
   <213> Homo sapiens
<400> 83
   aattgggtac cgggcccccc ctagaggtcg acggtatcga taagcttgta tcgaattccg 60
   gactttgctt ttggttttcc tttcctgtga aaaggttggt tttaaagtga gatacacttt 120
   tccgtagaac aagtgttcta t 141
<210> 84
   <211> 155
   <212> DNA
   <213> Homo sapiens
<400> 84
   aagttgattg cttcaaattt ctgctactaa cttcaagcta tgggagtttg gcagtagtca 60
   cttgaggatt ttttttccaa ttcgttttca tttttgttgt taaagctcgt acttcagtga 120
   gacagaaaaa ttgccaagct aaactaatgg tctat 155
<210> 85
   <211> 232
   <212> DNA
   <213> Homo sapiens
<400> 85
   gaattcctgg gctcaagtga tcctctcatg tcagtctccc aaagtgctgg gatgacaggc 60
   ttgagccacc acaccagccc atcatcagtt tttatataaa gaaaaaaaaa ccttaaaatt 120
   gttaggcaaa taatgacaaa ttgtaatata tattcttaca tttcagattt ttatttttta 180
   aactgataag aattgattaa taaataaaat ttagtattaa tctgtctttt aa 232
<210> 86
   <211> 245
   <212> DNA
   <213> Homo sapiens
<400> 86
   aaattcctgg gctgaagtga tcctccagtc ttggcctccc aaagtgctgg gattacaggc 60
   atgagctact gagcctagcc ttaatgatta attttagagt gatggcttgt accttcaagc 120
   aacatataga gttgagacag aaaatttcca tcgtcccgag aaaactgtgc tgcatgggcc 180
   ccgtgtgccc tgtgaagatc gccctattaa ctataaatgg gcattgcaca tggttgccag 240
   cttca 245
<210> 87
   <211> 239
   <212> DNA
   <213> Homo sapiens
<400> 87
   aacgtttcga acctatcgtg aagcccgatt ttagagttaa tacgggtgct tcaagggaac     60
   ggggctatga gaagttttct acggggagca tggaaatttt ctgtctcaat atgtgcttga    120
   aggtacaacc gtatctaaaa ttaatcatta aggctaggct cagtagctct gcctgtaatc    180
   ccagcacttt cgggaggcca agactggagg atcacttcag cccaggaatt tcagacgcc     239
<210> 88
   <211> 260
   <212> DNA
   <213> Homo sapiens
<400> 88
   tactgtgtga gcctgacctc aagcacagga ttccagaaat ggaaggatgt gccttgtgaa     60
   gacaagttct cctttgtctg caagttcaaa aactagaggc agctggaaaa tacatgtcta    120
   gaactgatcc agcaattaca acggagtcaa aaattaaacc ggaccatctc tccaactcaa    180
   ctcaacctgg acactctctt ctctgctgag tttgccttgt taatcttcaa tagttttacc    240
   taccccagtc tttggaacct                                                260
<210> 89
   <211> 149
   <212> DNA
   <213> Homo sapiens
<400> 89
   cttgcttgcc caggctggag tgcaatggca caatcctggc tcactgcaac ctccccctcc     60
   cgagctcaag aacttctcct gcctcagcct cgtgagccgc tgggattaca ggcgcgcgcc    120
   acaagcgact aatatttgta tttttgtag                                      149
<210> 90
   <211> 167
   <212> DNA
   <213> Homo sapiens
<400> 90
   ctcgctctgt cacccaggct gaagtgcagt ggcccaatct cggctcactg cgagctccac 60
   ctcccgggtt cacttcattc tcctgcctca ctgcctcagc ctctgagtag ctgggactac 120
   aggcgcccac caccacgtcc cctgctaatt ttttgtattt ttagtag 167
<210> 91
   <211> 84
   <212> DNA
   <213> Homo sapiens
<400> 91
   ccatgttcat caggctggtg tcgaactcct gacctcgtga tccgcccgcc tcagcctccc 60
   aaagtgctgg gattacaagc gtgc 84
<210> 92
   <211> 85
   <212> DNA
   <213> Homo sapiens
<400> 92
   ccgtgttggc caggatggtc tcgatctcct gacctcgtga tccgcccgcc ttggccaccc 60
   aaagagtttg ggattacagg cgtgc 85
<210> 93
   <211> 251
   <212> DNA
   <213> Homo sapiens
<400> 93
   tgcagcaatg gcaacaacgt ctgcaaacta ttaactggcg aactacttac tctagcttcc 60
   cggcaacaat taatagactg gatggaggcg gataaagttg caggaccact tctgcgctcg 120
   gcccttccgg ctggctggtt tattgctgat aaatctggag ccggtcgagc gtgggtctcg 180
   cgtatcattc gagcactggg gccagatggt aagccctccg tatcgtagtt atctcacagc 240
   agggagtcag g 251
<210> 94
   <211> 242
   <212> DNA
   <213> Homo sapiens
<400> 94
   tgcaggagcg gggaggcacg atggccgctt tggtccggat ctttgtgagg aaccttactt 60
   ctgtggtgtg acataattgg acaaactacc tacagagatt taaagctcta aggaaatata 120
   aaatttttaa gtgtataatg tgttaaacta ctgattctaa ttgtttgtgt attttagatt 180
   ccaaccctat ggaacctgat gaatgggagc cagtggtgga atgcctttaa tgaggaaacc 240
   tg 242
<210> 95
   <211> 208
   <212> DNA
   <213> Homo sapiens
<400> 95
   tgcagcaatc tttcttatat acatgcttaa tagatagcta cttaaaataa cttacacacg 60
   ttttagagtt gcttgaaaac tatctgatca agacatagta attgaaacca atgaatacat 120
   tatataaagt aaaggaaagg agaagagagg aaagggaggg gaagaggaga gggagggaca 180
   agcgagaaag gaaagggaag ggagaaaa 208
<210> 96
   <211> 152
   <212> DNA
   <213> Homo sapiens
<400> 96
   ctcactaaag ggatcaagga ataattttga atttcaagtc ttacatttaa taaatacatt 60
   cataaggcta taactaccat acgttgtgat ttctctgatt aatttaaaaa taaattaaaa 120
   cctggaaaga attttaccat tctaggaagc ca 152
<210> 97
   <211> 338
   <212> DNA
   <213> Homo sapiens
<400> 97
   aatctatctt atatacatgc ttaatagata gctacttaaa ataacttaca cacgttttag 60
   agttgcttga aaactatctg atcaagacat agtaattgaa accaatgaat acattatata 120
   aagtaaagga aaggagaaga gaggaaagga ggggagagga gaggaggaca agcgagaaaa 180
   ggaagggaag ggagaaaaag ggggaaaggg aggtagagag agagagaaaa agtgctggtc 240
   atatagtaag tgtacatttt aactttttaa gaaactaccc tactctattc cagagtgatt 300
   gtacatgtgc attttactgc attatagaga tcattttc 338
<210> 98
   <211> 169
   <212> DNA
   <213> Homo sapiens
<400> 98
   tgcaggagtg gggaggcacg atggccgctt tggtccggat ctttgtgaag gaaccttact 60
   tctgtgtgtg acataattgg acaaactacc tacagagatt taaacgtcta aggtaaatat 120
   aaaattttta gtgtataggt taaactactg attctaatgt tgtgtattt 169
<210> 99
   <211> 209
   <212> DNA
   <213> Homo sapiens
<400> 99
   ccccgggctg cagcaatggc aacaacgtct gcaaactatt aactggcgaa ctcattcatc 60
   tagcttcccg gcaacaatta atgactggat ggaggcggat aaagttgcag gaccacttct 120
   cgcgtggccc ttccggctgg ctggtttatt gctgataatt gagcgtgcga gtggctcgcg 180
   tatcattcgc gacatgggcc agtaggtac 209
<210> 100
   <211> 272
   <212> DNA
   <213> Homo sapiens
<400> 100
   cttgcccttc atggagtcat acagccgatc agcaaaatgc aggggcttgt tctgaatgca     60
   ctgaaccagg ttcaggaaag cattttccag gtctccttta acctctttcc tgatgctttc    120
   caacatgtca taagggctgt aactcttgta cctatcaaat actttctgga ggtggggaca    180
   cgctcgcgtc ggtcatgatg ctgatccact tgggaacatc agttctttcc tcttcactcc    240
   agctgcatag agatccgagg actcttggtc aa                                  272
<210> 101
   <211> 278
   <212> DNA
   <213> Homo sapiens
<400> 101
   acggcccagc ttccttcaaa atgtctactg ttcacgaaat cctgtgcaag ctcagcttgg     60
   agggtgatca ctctacaccc ccaagtgcat atgggtctgt caaagcctat actaactttg    120
   atgctgagcg ggatgctttg aacattgaaa cagccatcaa gaccaaaggt gtggatgagg    180
   tcaccattgt caacattttg accaaccgca gcaatgacac gagacaggat attgccttcg    240
   cctaccagag aaggaccaaa aaaggaactt gcatcaca                            278
<210> 102
   <211> 228
   <212> DNA
   <213> Homo sapiens
<220>
   <221> misc_feature
   <222> (226)..(226)
   <223> n is a, c, g, or t
<400> 102
   agcaatagca aaggaaagga aacaatattt agcaaggttt attcttcctt tgtgtcagca     60
   tttctgagtg tgcacacagg cccagtgatt ccatgtattt ttgagtgacc actgcctctg    120
   tctggccctt ccccatagaa ccgccgctgg tggagcgtgg gtccctggtc tcctacaagt    180
   cctggggcat tggagcccca agcagtgtta atcctggcac tgtgtnag                 228
<210> 103
   <211> 246
   <212> DNA
   <213> Homo sapiens
<220>
   <221> misc_feature
   <222> (191)..(191)
   <223> n is a, c, g, or t
<400> 103
   agcaatggca acaacgtctg caaactatta actggcgaac tacttactct tagcttccgg 60
   caacaattaa tagactggat ggaggcggat aaagttgcag gaccacttct gcgctcggcc 120
   cttccggctg gctggtttat tgctgataaa tctggagccg gtcgagcgtg ggtctcgcgt 180
   atcattcgag nctggggcca gatggtaagc cctccgtatc gtagttatct cacagcaggg 240
   agtcag 246
<210> 104
   <211> 86
   <212> DNA
   <213> Homo sapiens
<400> 104
   ctggcactgg agcagtgggt ccctggtctc ctacaagtcc tggggcattg gagccccaag 60
   cagtgttaat cctggcactg tgtgag 86
<210> 105
   <211> 154
   <212> DNA
   <213> Homo sapiens
<220>
   <221> misc_feature
   <222> (40)..(40)
   <223> n is a, c, g, or t
<220>
   <221> misc_feature
   <222> (99)..(99)
   <223> n is a, c, g, or t
<400> 105
   ctggagccgg tcgagcgtgg gtctcgcgta tcattcgagn ctggggccag atggtaagcc 60
   ctccgtatcg tagttatctc acagccgtat cattcgagnc tggggccaga tggtaagccc 120
   tccgtatcgt agttatctca cagcagggag tcag 154
<210> 106
   <211> 221
   <212> DNA
   <213> Homo sapiens
<400> 106
   aggagacttg tggtaaaaat ctgctgctgt actgctcatt tgggaacctt agtatactaa 60
   ataatataat atatcaacaa ctaatggtca gccaatgcta tgctggatat gagggtcctg 120
   ggccacaaag acaaaaaatc aggaaccact ttttaagtga gatactttgg gtctctgtca 180
   aattcataac acttatttct tggtggaata cagttaatga g 221
<210> 107
   <211> 231
   <212> DNA
   <213> Homo sapiens
<400> 107
   aggagcgggg aggcacgatg gccgctttgg tccggatctt tgtgaggaac cttacttctg 60
   tggtgtgaca taattggaca aactacctac agagatttaa agctctaagg aaatataaaa 120
   tttttaagtg tataatgtgt taaactactg attctaattg tttgtgtatt ttagattcca 180
   accctatgga acctgatgaa tgggagccag tggtggaatg cctttaatga g 231
<210> 108
   <211> 102
   <212> DNA
   <213> Homo sapiens
<400> 108
   taaatctgga gccggtcgag cgtgggtctc gcgtatcatt cgagcactgg ggccagatgg 60
   taagccctcc gtatcgtagt tatctcacag cagggagtca gg 102
<210> 109
   <211> 110
   <212> DNA
   <213> Homo sapiens
<400> 109
   tagaaccgcc gctggcactg gagcagtggg tccctggtct cctacaagtc ctggggcatt 60
   ggagccccaa gcagtgttaa tcctggctac tgtgtgagcc tgacctcaag 110
<210> 110
   <211> 85
   <212> DNA
   <213> Homo sapiens
<400> 110
   atagatatct actttattcg atttaaattc tgtttagtat tttattatat tttgttaatc 60
   catttgtccc aattcatata cttat 85
<210> 111
   <211> 95
   <212> DNA
   <213> Homo sapiens
<400> 111
   atagatagct acttaaaata acttacacac tgttttagag tgcttgaaaa ctatctgatc 60
   agacatagta attgaaacca atgaatacat tatat 95
<210> 112
   <211> 90
   <212> DNA
   <213> Rattus sp.
<220>
   <221> misc_feature
   <222> (85)..(86)
   <223> n is a, c, g, or t
<220>
   <221> misc_feature
   <222> (89)..(89)
   <223> n is a, c, g, or t
<400> 112
   gaaggatctg tttgtaagaa cggagcggga taccataaaa atagaggtaa taacatacat 60
   tgggacgtgt aaatttattt ttatnnaant 90
<210> 113
   <211> 94
   <212> DNA
   <213> Homo sapiens
<400> 113
   gaaggatctt accattttaa gaaaggtcca aaattaaata aaaatttaat tagtctcttt 60
   agtgttgcat accatcaata tcggaatact aaat 94
<210> 114
   <211> 200
   <212> DNA
   <213> Homo sapiens
<400> 114
   acctggtgct gtgctcaccc aggccgaggg tgcctttgtg gcctcactga ttaaggagag 60
   tggcatgatg acttcaatgt ctggattggc ctccatgacc ccaaaaagaa ccgccgctgg 120
   ggagcgtggg tccctggtct cctacaagtc ctggggcatt ggagccccaa gcagtgttaa 180
   tcctggctac tgtgtgagcc 200
<210> 115
   <211> 218
   <212> DNA
   <213> Homo sapiens
<400> 115
   aactggcgaa ctacttactc tagcttccgg caacaattaa tagactggat ggaggcggat 60
   aaagttgcag gaccacttct gcgctcggcc cttccggctg gctggtttat tgctgataaa 120
   tctggagccg gtgagcgtgg gtctcgcgta tcattgcagc actggggcca gatggtaagc 180
   cctccgtatc gtggttatct acacgacggg gagtacgc 218
<210> 116
   <211> 146
   <212> DNA
   <213> Homo sapiens
<400> 116
   aggcccatca tcagttttta tataaagaaa aaaaaacctt aaaattgtta ggcaaatact 60
   atgacaaatt gtaatatata ttcttacatt tcagattttt attttttaaa ctgtatagaa 120
   ttgattaata aataaaattt agtatt 146
<210> 117
   <211> 155
   <212> DNA
   <213> Homo sapiens
<400> 117
   aggcacgatg gccgctttgg tccggatctt tgtgaaggaa ccttacttct gtgtgtgaca 60
   taattggaca aactacctac agagatttaa acgtctaagg taaatataaa atttttagtg 120
   tataggttaa actactgatt ctaatgttgt gtatt 155
<210> 118
   <211> 102
   <212> DNA
   <213> Homo sapiens
<400> 118
   taaatctgga gccggtgagc gtgggtctcg cgtatcattg cagcactggg gccagatggt     60
   aagccctccg tatcgtggtt atctacacga cggggagtac gg                       102
<210> 119
   <211> 116
   <212> DNA
   <213> Homo sapiens
<400> 119
   tagaaccgcc gctggcactg gagcagtggg tccctggtct cctacaagtc ctggggcatt     60
   ggagccccaa gcagtgttaa tcctggctac tgtgtgagcc tgacctcaag cacagg        116
<210> 120
   <211> 6
   <212> PRT
   <213> Unknown
<220>
   <223> Unknown Peptide
<400> 120
   His Pro Thr Pro Ala Ala
   1               5
<210> 121
   <211> 7
   <212> PRT
   <213> Unknown
<220>
   <223> Unknown peptide
<400> 121
   Phe Val Ala Ser Leu Ile Lys
   1               5

## Description

### Field of the Invention

The present invention is in the field of genetic engineering and molecular biology. This invention is directed to recombinant hosts expressing novel proteins associated with Alzheimer's Disease, neuroectodermal tumors, malignant astrocytomas, and glioblastomas. This invention is specifically directed to the recombinant hosts and vectors which contain the genes coding for the neuronal thread proteins. This invention is also directed to substantially pure neural thread proteins, immunodiagnostic and molecular diagnostic methods to detect the presence of neural thread proteins, and the use of nucleic acid sequences which code for neural thread proteins in gene therapy.

### Background of the Invention

### Alzheimer's Disease

Alzheimer's Disease (AD) is the most frequent cause of dementia in the United States, affecting over two million individuals each year. It is a degenerative brain disorder characterized clinically by loss of memory, confusion, and gradual physical deterioration. It is the fourth most common cause of death. The etiology of the disease is virtually unknown but has been attributed to various viruses, toxins, heavy metals, as well as genetic defects. The disease is at present incurable.

Until quite recently, AD was thought to account for relatively few of the cases generally classified as senile dementia. Other factors can lead to such a condition, including repetitious mild strokes, thyroid disorders, alcoholism, and deficiencies of certain vitamins, many of which are potentially treatable. It can be appreciated, then, that a diagnostic test specific for AD would be very useful for the clinical diagnosis and proper clinical treatment of subjects presenting with symptoms common to all of these conditions.

The brains of individuals with AD exhibit characteristic pathological accumulations of congophilic fibrous material which occurs as neurofibrillary tangles within neuronal cell bodies, and neuritic (or senile) plaques. Neurofibrillary tangles may also be found in the walls of certain cerebral blood vessels. The major organized structural components of neurofibrillary tangles are paired helical filaments. Qualitatively indistinguishable amyloid deposits also occur in normal aged brains but in much smaller numbers with restricted topographical distribution.

There has been considerable recent investigative activity regarding the characterization of proteins found in neuritic plaques and neurofibrillary tangles of AD and other neurologic diseases. One of the amyloid proteins initially described by Glenner *et al.* has been cloned and sequenced (Glenner et al.. Biochem. Biophys. Res. Commun. 120:1131-1135 (1984): U.S. Patent No. 4,666.829). The A4 amyloid protein found in neuritic plaques and blood vessels has been determined to be a component of a 695 amino acid precursor; a protein postulated to function as a glycosylated cell surface receptor (Masters et al., Proc. Natl. Acad. Sci. USA 82:4245-4249 (1985), Kang et al., Nature 325:733-736 (1987)). In addition, the amyloid protein has been postulated to function as a cell adhesion molecule and as a calcium ion channel protein (Hooper, J. NIH Res. 4: 48-54 (1992); Rensberger, Wayward Protein Molecule May Be Elusive Killer of Brain Cells, The Washington Post, January 25, 1993, §1, at A3 (1993)). The gene coding for A4 is located on chromosome 21 (Kang *et al. , ibid. ;* Goldgaber et al. , Science 235:877-880 (1987); Tanzi et al., Science 235:880-885 (1987); St. George-Hyslop et al., Science 235:885-889 (1987)) but apparently is not linked to the familial form of the disease (Van Broekhoven et al., Nature 329:153-155 (1987)). There appears to be little, if any, protein sequence homology between amyloid A4 and B protein, their higher molecular weight precursor, and pancreatic thread protein (PTP) (Gross et al., J. Clin. Invest. 76:2115-2126 (1985)).

A number of other proteins thought to be associated with the disease have been described, including Ubiquitin, ALZ-50, microtubular-associated proteins τ and MAP2, and neurofilament protein (*see,* for example, Manetto et al.. Proc. Natl. Acad. Sci. USA 85:4502-4505 (1988): Wolozin et al., Science 232:648-651 (1986), Selkoe, Neurobiol. Aging 7:425-432 (1986): Perry et al., in: Alterations of the Neuronal Cytoskeleton in Alzheimer's Disease, Plenum, New York, pp 137-149 (1987)). More recently, a serine protease inhibitor called α₁-anti-chymotrypsin has been found in AD amyloid deposits (Abraham et al., Cell 52:487-501 (1988)).

There is currently no useful diagnostic test for AD being practiced clinically. A definitive diagnosis is possible only postmortem, or during life through a brain biopsy, to reveal the presence of the characteristic plaques, tangles, paired helical filaments, and other cerebrovascular deposits which characterize the disorder. Such an invasive surgical procedure is inherently dangerous and is therefore rarely utilized. As a result, the clinical misdiagnosis of AD is estimated to be approximately 20%-30%.

### Thread Proteins

The prototype thread protein molecule is pancreatic thread protein (PTP) which bears the unusual physical property of forming insoluble fibrils at neutral pH, but is highly soluble at acid or alkaline pH (Gross *et al., supra*). PTP is highly abundant, synthesized by pancreatic acinar cells, and secreted into pancreatic juice in concentrations exceeding 1 mg/ml (*Id.*)*.* An increased thread protein immunoreactivity has been demonstrated in brains with AD lesions, using monoclonal antibodies to PTP (Ozturk et al., Proc. Natl. Acad. Sci. USA 86:419-423 (1989)). In addition, a highly sensitive forward sandwich immunoradiometric assay was used to demonstrate that at least three distinct antigenic epitopes were shared between PTP and the related protein in the brain (*Id.*) Despite similarities, the pancreatic and neuronal forms of the thread protein are almost certainly distinct since the mRNA molecules and proteins differ in size, and many of the antigenic epitopes which are present in the pancreatic thread protein are not detectable in brain tissue (de la Monte et. al., J. Clin. Invest. 86:1004-1013 (1990); de la Monte et. al., J. Neurol. Sci. 113:152-164 (1992); de la Monte et al., Ann. Neurol. 32:733-742 (1992)).

The central nervous system form of the thread protein, designated hereafter as "neural thread protein" (NTP), has been identified in AD and Down's Syndrome brain tissue (Wands et al., International Application Publication No. WO 90/06993). NTP has been found in all AD brains studied where characteristic neuropathologic changes of the disease exist (*Id.*). The saline- extractable soluble immunoreactivity shares has a molecular weight of approximately 17 to 20 kD (*Id.*).

Quantitative measurements of NTP immunoreactivity in various regions of AD brains revealed levels varying from 12 to 295 ng/gm tissue (Mean = 116 ng/gm tissue) compared to 1-11 ng/gm tissue (Mean = 5 ng/gm tissue) in comparable ares of control brains (*Id.*).

Immunocytochemistry performed with monoclonal antibodies directed against the pancreatic form of PTP demonstrated that NTP is localized within cells, within fine processes within the neuropil, or is extracellular in both AD and Down's Syndrome brains (*Id*.). Two types of cell contain NTP: neurons and astrocytes (*Id*.). The affected neurons are the large pyramidal type which typically contain the neurofibrillary tangles well known in AD brain (*Id*.).

That NTP accumulation within neurons is intrinsically important or integrally related to the evolution of AD lesions is corroborated by the presence of identical patterns of immunolabeling for NTP in Down's Syndrome brains, but not in control brains (*Id*.). It is important to note that the same structural abnormalities of AD occur in brains of all middle-age individuals with Down's syndrome, whether or not they are demented. There is also a higher incidence of AD in family members of Down's Syndrome patients. Moreover, the regional differences in the densities of NTP-containing neurons parallels the density distributions of neurofibrillary tangles in both AD and Down's Syndrome. This provides further evidence that NTP is germane to the pathophysiology of AD. Whether NTP accumulates within neuronal perikarya, as a result of aberrant cellular metabolism or transport is not yet known.

### Summary of the Invention

A need exists for a definitive diagnostic test which can be performed on individuals suspected of having, or being at risk for AD. The present invention satisfies such needs and provides further advantages.

The manner in which these and other objects are realized by the present invention will be apparent from the summary and detailed description set forth below.

Unless defined otherwise, various items used herein have the same meaning as is well understood in the art to which the invention belongs.

This invention is directed to recombinant hosts expressing a novel protein associated with Alzheimer's Disease, neuroectodermal tumors, malignant astrocytomas, and glioblastomas. This invention is specifically directed to the recombinant hosts and vectors which contain the gene coding for the neuronal thread protein (NTP) having a molecular weight of about 42 kDa. In the first aspect, the present invention relates to a neural thread protein (NTP) substantially free of any natural impurities and coded for by the AD3-4DH1 DNA molecule present in the *E. coli* cells that are on deposit at the American Type Culture Collection, Manassass, Va., under accession no. 69260. This invention is also directed to the substantially pure neural threat proteins, immunodiagnostic and molecular diagnostic methods to detect the presence of neural thread proteins, and the use of nucleic acid sequences which code for neural thread proteins in gene therapy.

In particular, the invention includes an *in vitro* method for detecting the presence of an NTP encoded by AD3-4DH1 DNA molecule, comprising:
(a) contacting a biological sample from a human subject that is suspected of containing NTP encoded by AD3-4DH1 DNA molecule with at least one molecule capable of binding to said NTP; and
(b) detecting the molecule bound to said NTP.

The invention additionally includes the method as above, wherein the binding molecule is selected from the group consisting of:
(a) an antibody substantially free of natural impurities;
(b) a monoclonal antibody; and
(c) a fragment of (a) or (b).

The invention additionally includes the method as above, wherein the detecting molecule is detectably labeled and where a combination of such binding molecules is used.

The invention additionally includes an *in vitro* method for detecting the presence of a genetic sequence coding for an NTP encoded by AD3-4DH1∼DNA molecule in a biological sample using a polynucleotide probe derived from a recombinant human NTP of this invention.

The invention additionally includes a method for determining the presence of a condition in a human subject, said condition including, but not limited to, the group consisting of Alzheimer's Disease, the presence of neuroectodermal tumors, the presence of malignant astrocytomas, and the presence of gliomas.

The invention additionally includes a method of diagnosing the presence of AD in a human subject suspected of having AD which comprises:
(a) incubating a biological sample from said subject suspected of containing an NTP encoded by AD3-4DH1 DNA molecule with a molecule capable of identifying an NTP encoded by AD3-4DH1 DNA molecule; and
(b) detecting the molecule which is bound in the sample, wherein the detection indicates that the subject has AD.

The invention additionally includes a method of diagnosing the presence of neuroectodermal tumors in a human subject suspected of having neuroectodermal tumors which comprises:
(a) incubating a biological sample from said subject suspected of containing an NTP encoded by AD3-4DH1 DNA molecule with a molecule capable of identifying an NTP encoded by AD3-4DH1 DNA molecule; and
(b) detecting the molecule which is bound in the sample, wherein the detection indicates that the subject has neuroectodermal tumors.

The invention additionally includes a method of diagnosing the presence of a malignant astrocytoma in a human subject suspected of having a malignant astrocytoma which comprises:
(a) incubating a biological sample from said subject, which is suspected of containing an NTP encoded by AD3-4DH1 DNA molecule, in the presence of a binding molecule capable of identifying an NTP encoded by AD3-4DH1 DNA molecule; and
(b) detecting molecule which is bound in the sample, wherein the detection indicates that the subject has a malignant astrocytoma.

The invention additionally includes a method of diagnosing the presence of a glioblastoma in a human subject suspected of having a glioblastoma which comprises:
(a) incubating a biological sample from said subject, which is suspected of containing an NTP encoded by AD3-4DH1, in the presence of a binding molecule capable of identifying an NTP encoded by AD3-4DH1; and
(b) detecting molecule which is bound in the sample, wherein the detection indicates that the subject has a glioblastoma.

The invention additionally includes the methods as above, wherein a biological sample is removed a human subject prior to contacting the sample with the molecule.

The invention additionally includes the methods as above, wherein detecting any of the molecules bound to the protein is performed by in situ imaging.

The invention additionally includes the methods as above, wherein detecting of any of the molecule bound to the protein is performed by in vivo imaging.

The invention additionally includes the methods as above, wherein the biological sample is reacted with the binding molecule in a manner and under such conditions sufficient to determine the presence and the distribution of the protein.

The present invention also particularly relates to the diagnostic methods recited above, wherein the immunoassay comprises two different antibodies bound to a solid phase support combined with a third different detectably labeled antibody in solution.

The invention is also directed to a method of producing an NTP encoded by AD3-4DH1, said method comprising:
(a) culturing a recombinant host comprising a human gene coding for said NTP encoded by AD3-4DH1; and
(b) isolating said NTP from said host.

Additionally, the invention is directed to a substantially pure NTP encoded by AD3-4DH1 obtained by the such a process.

The invention is also directed to ribozymes comprising a target sequence which is complementary to a sequence of an NTP encoded by AD3-4DH1 and nonhomologous to PTP nucleic acid sequences, as well as pharmaceutical compositions comprising such ribozymes and a pharmaceutically acceptable carrier.

The invention is also directed to a method of achieving pharmaceutical delivery of NTP encoded by AD3-4DH1 to the brain through acceptable carriers or expression vectors.

The invention is also directed to oligodeoxynucleotides that form triple stranded regions with the gene of the NTP encoded by AD3-4DH1 (nucleic acid sequences) and which are nonhomologous to PTP nucleic acid sequences, as well as pharmaceutical compositions comprising such oligodeoxynucleotides and a pharmaceutically acceptable carried.

The invention is also directed to methods for the differential diagnosis of sporadic and familial Alzeimer's disease.

### Brief Description of the Drawings

Figures 1A-1J show neural thread protein immunoreactivity in CNS-derived tumors.
Figure 2 depicts a graph showing neuraL thread protein levels in PNET1, PNET2, A172, C6, and Huh7 hepatocellular carcinoma cells measured by a forward sandwich monoclonal antibody-based immunoradiometric assay (M-IRMA).
Figure 3 shows molecular size of neural thread proteins in SH-Sy5y, A172, and C6 cells demonstrated-by immunoprecipitation and Western blot analysis using the Th9 monoclonal antibody.
Figure 4 shows molecular sizes of neural thread proteins in PNET1 cells (a) and C6 glioblastoma cells (b) demonstrated by pulse-chase metabolic labeling with ³⁵S-methionine, and immunoprecipitation with Th9 monoclonal antibody (Figure 4A). The molecular weights are 8, 14, 17, 21, 26 and 42 kDa (arrows).
Figures 5A-5E depict a series of five graphs showing the 21 kDa and 17 kDa neural thread proteins in SH-Sy5y, PNET1, A172, and C6 cells and the absence thereof in Huh7 cells by SDS-PAGE/M-IRMA.
Figure 6 depicts a gel showing that the 21 kDa neural thread protein in C6 glioblastoma cells is phosphorylated.
Figure 7 depicts a bar graph showing altered neural thread protein expression in PNET1 cells with growth phase.
Figures 8A-8F show altered phenotype of PNET1 cells with cessation of cell growth and overnight serum starvation.
Figure 9 shows the 1-9a partial cDNA. sequence, and Figure 9A shows a partial sequence of the second 5' anchor PCR product corresponding to the 5'region of the 1-9a cDNA (WP5' Sequence).
Figure 10 shows alignment of partial sequences between 1-9a and human PTP and the Reg gene (the nucleic acid sequence corresponding to the genomic clone of human NTP).
Figure 10A shows alignment between 1-9a and Exon 2 of the human Reg gene, and between the first 5' anchor PCR product of 1-9a (WP03-417) and Exon 2 and Reg.
Figure 10B shows alignment between the 1-9a and its second 5' anchor PCR product (WP5') and AD 3-4 and AD 2-2 cDNAs.
Figure 11A shows the partial nucleic acid and deduced amino acid sequences of the HB4 cDNA. Figures 11B and 11C show a protein hydrophilicity window plot. Hydrophilicity Window Size = 7; scale = Kyte-Doolittle.
Figure 11D shows alignment between HB4 and human FTP.
Figure 11E shows alignment between HB4 and human Reg gene.
Figures 12A-12C show the expression of mRNA molecules corresponding to the 1-9a CNS neural thread protein cDNA sequence in neuroectodermal tumor cell lines and in rat pancreas.
Figures 13A and 13B, show mRNA transcripts corresponding to the 1-9a CNS neural thread protein cDNA sequence in human brain. This figure also demonstrates higher levels of 1-9a CNS neural thread protein-related mRNAs in AD brains compared with aged-matched controls (Figure 13A). Figure 13B demonstrates four different transcripts with greater abundance of the lower molecular size mRNAs in AD compared with aged controls.
Figures 14A-14C show 1-9a Southern blot analysis of RT/PCR-derived cDNAs in neuroectodermal cell lines. A- and B-PCR amplification of 1-9a mRNA sequences in neuroectodermal cell lines, and using mRNA from newborn rat (NB) brain, AD brain, and aged control brain. Figure 14A is a longer exposure of Figure 14-B. Figure 14C shows hybridization of the same blot using the O18 rat PTP probe.

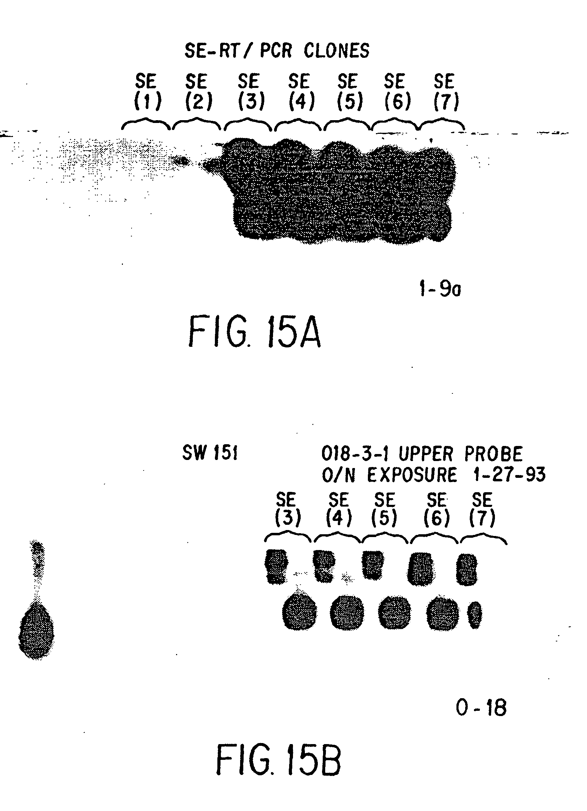

Figures 15A and 15B (SE-RT/PCR) show hybridization of the 1-9a and O18 probes with several clones isolated from SH-Sy5y cells by reverse transcribing mRNA and amplifying with primers corresponding to the know sequence of the 1-9a partial cDNA.
Figures 16A, 16D and 16E show the partial nucleic acid sequences of the AD 2-2 cDNAs isolated from the AD brain library. Figures 16B and 16C show a hydrophilicity window plot of AD2-2 T7. Hydrophilicity Window Size = 7; scale = Kyte-Doolittle.
Figures 16F, 16I, 16J and 16K show the partial nucleic acid sequences of the AD 3-4 cDNAs isolated from the AD brain library. Figures 16G and 16H show a hydrophilicity window plot of AD3-4. Hydrophilicity Window Size = 7; scale = Kyte-Doolittle.
Figures 16L, 16M and 16N show the martial nucleic acid sequences of the AD 4-4 cDNAs isolated from the AD brain library.
Figure 16O shows the partial nucleic acid sequences of the AD 16c (also called AD 10-7) cDNAs isolated from the AD brain library. Figures 16P and 16Q show a hydrophilicity window plot of AD16c-T7. Hydrophilicity Window Size =7; scale = Kyte-Doolittle.
Figure 16R shows the complete nucleotide sequence of the AD10-7 cDNA clone that was isolated from an AD library.
Figure 16S shows the complete nucleotide sequence of the AD16c cDNA clone that was isolated from the AD brain library.
Figure 17 shows alignment of martial sequences between AD 2-2 and human Reg gene.
Figure 17A shows alignment of partial sequences between AD 2-2 and Exon 1 of Reg and rat FTP.
Figure 17B shows alignment of partial sequences between AD 2-2 and 1-9a.
Figure 17C shows alignment of partial sequences between AD 2-2 and AD 16c.
Figure 18 shows alignment of partial sequences between AD 3-4 (also called AD 5-3) and the Reg gene.
Figure 18A shows alignment of partial sequences between AD 3-4 and the 5 anchor PCR products of the 1-9a mRNA, termed WPO3-5 and 18-4.
Figures 18B shows alignment of partial sequences between AD 3-4 and the G2a-a *Eco*RI/*Pst*I genomic clone.
Figure 19 shows alignment of partial sequences between AD 4-4 and AD 2-2 and 1-9a (also called SE-4 corresponding to the PCR clone which is identical to 1-9a).
Figure. 20 shows alignment of partial sequences between AD 16c and Reg gene.
Figure 20A shows alignment of partial sequences between AD 16c and human PTP.
Figure 20B. shows alignment of partial sequences between AD 16c and AD 2-2.

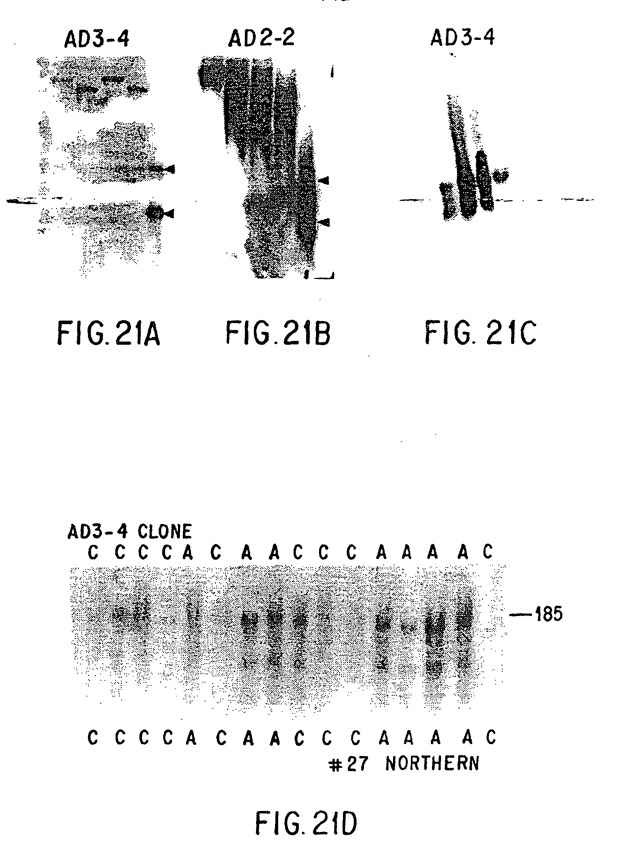

Figures 21A-21D show a genomic Southern blot analysis using the AD 3-4 as a probe; Figure 21B shows a simitar pattern of hybridization on a genomic Southern using AD 2-2 as a probe. Figures 21A-21D show a Northern blot analysis of neuroectodermal tumor cell lines using And 3-4 as a probe. The four cell lines that exhibit AD 3-4 transcripts are neuronal in phenotype; C6 glioma cell mRNA did not hybridize with the AD 3-4 probe. Figure 21D shows a Northern: analysis of human AD and aged control brain temporal lobe tissue using the AD 3-4 probe, and demonstrates over-expression of the Corresponding gene in AD (lanes labeled A) compared with aged control brains (lanes labeled C).
Figures 22, 22A, 22B, 22C, 22D, 22E, 22F, 22G and 22H show partial sequences of four genomic clones (isolated using both the 1-9a cDNA and rat PTP O-18 cDNA as probes.
Figures 23 and 23A show the alignment of the G2a-2 *Pst*I partial sequence with the Reg gene.
Figure 23B shows alignment of the G2a-2 *Pst*I-*Eco*RI sequence and the Reg gene and the tat PTP.
Figures 23C and 23D show the alignment of the G5d-1 *Pst*I sequence and the Reg gene.
Figures 24A-24D show neutral thread protein expression by the 1-9a cDNA (Figure 24A) and the G2a-2 *Pst*I genomic clone (Figure 24B). Figures 24C and 24D show negative expression by the G5d-1 *Eco*RI/*Pst*I genomic clone, and pBluescript which lacks a cloned insert, respectively.
Figures 25A and 25B depict a Northern blot analysis of AD16c mRNA in AD and aged control brains. The data shows elevated levels of AD16c mRNA expression in 6 of 9AD compared to 1 of 6 age-matched controls.
Figure 26 depicts a Western blot analysis of AD10-7 fusion proteins using monoclonal antibodies against the expressed tag protein (T7-tag mouse monoclonal antibodies.
Figures 27A and 27B depict brightfield and darkfield microscopic analysis of the *in situ* hybridization of sense and antisense cRNA probes to human brain tissue sections of early AD.

### Definitions

In the description that follows, a number of terms used in recombinant DNA technology are utilized extensively. In order to provide a clear and consistent understanding of the specification and claims, including the scope to be given such terms, the following definitions are provided.

***Cloning vector**.* A plasmid or phage DNA or other DNA sequence which is able to replicate autonomously in a host cell, and which is characterized by one or a small number of restriction endonuclease recognition sites at which such DNA sequences may be cut in a determinable fashion without loss of an essential biological function of the vector, and into which a DNA fragment may be spliced in order to bring about its replication and cloning. The cloning vector may further contain a marker suitable for use in the identification of cells transformed with the cloning vector. Markers, for example, provide tetracycline resistance or ampicillin resistance.

***Expression vector**.* A vector similar to a cloning vector but which is capable of enhancing the expression of a gene which has been cloned into it, after transformation into a host. The cloried gene is usually placed under the control of (i.e., operably linked to) certain control sequences such as promoter sequences. Promoter sequences may be either constitutive or inducible.

***Substantially pure.*** As used herein means that the desired purified protein is essentially free from contaminating cellular components, said components being associated with the desired protein in nature, as evidenced by a single band following polyacrylamide-sodium dodecyl sulfate gel electrophoresis. Contaminating cellular components may include, but are not limited to, proteinaceous, carbohydrate, or lipid impurities.

The term "substantially pure" is further meant to describe a molecule which is homogeneous by one or more purity or homogeneity characteristics used by those of skill in the art. For example, a substantially pure NTP will show constant and reproducible characteristics within standard experimental deviations for parameters such as the following: molecular weight, chromatographic migration, amino acid composition, amino acid sequence, blocked or unblocked N-terminus, HPLC elution profile, biological activity, and other such parameters. The term, however, is not meant to exclude artificial or synthetic mixtures of the factor with other compounds. In addition, the term is not meant to exclude NTP fusion proteins isolated from a recombinant host.

***Recombinant Host**.* According to the invention, a recombinant host may be any prokaryotic or eukaryotic cell which contains the desired cloned genes on an expression vector or cloning vector. This term is also meant to include those prokaryotic or eukaryotic cells that have been genetically engineered to contain the desired gene(s) in the chromosome or genome of that organism.

***Recombinant vector**.* Any cloning vector or expression vector which contains the desired cloned gene(s).

***Host**.* Any prokaryotic or eukaryotic cell that is the recipient of a replicable expression vector or cloning vector. A "host," as the term is used herein, also includes prokaryotic or eukaryotic cells that can be genetically engineered by well known techniques to contain desired gene(s) on its chromosome or genome. For examples of such hosts, *see* Sambrook et al., Molecular Cloning: A Laboratory Manual, Second Edition, Cold Spring Harbor Laboratory, Cold Spring Harbor, New York (1989).

***Promoter**.* A DNA sequence generally described as the 5' region of a gene, located proximal to the start codon. The transcription of an adjacent gene(s) is initiated at the promoter region. If a promoter is an inducible promoter, then the rate of transcription increases in response to an inducing agent. In contrast, the rate of transcription is not regulated by an inducing agent if the promoter is a constitutive promoter.

***Gene**.* A DNA sequence that contains information needed for expressing a polypeptide or protein.

***Structural gene**.* A DNA sequence that is transcribed into messenger RNA (mRNA) that is then translated into a sequence of amino acids characteristic of a specific polypeptide.

***Antisense RNA gene*/*****Antisense RNA**.* In eukaryotes, mRNA is transcribed by RNA polymerase II. However, it is also known that one may construct a gene containing a RNA polymerase II template wherein a RNA sequence is transcribed which has a sequence complementary to that of a specific mRNA but is not normally translated. Such a gene construct is herein termed an "antisense RNA gene" and such a RNA transcript is termed an "antisense RNA." Antisense RNAs are not normally translatable due to the presence of translation stop codons in the antisense RNA sequence.

***Antisense oligonucleotide**.* A DNA or RNA molecule containing a nucleotide sequence which is complementary to that of a specific mRNA. An antisense oligonucleotide binds to the complementary sequence in a specific mRNA and inhibits translation of the mRNA.

***Antisense Therapy.*** A method of treatment wherein antisense oligonucleotides are administered to a patient in order to inhibit the expression of the corresponding protein.

***Complementary DNA (cDNA)**.* A "complementary DNA," or "cDNA" gene includes recombinant genes synthesized by reverse transcription of mRNA and from which intervening sequences (introns) have been removed.

***Expression**.* Expression is the process by which a polypeptide is produced from a structural gene. The process involves transcription of the gene into mRNA and the translation of such mRNA into polypeptide(s).

***Homologous*/*****Nonhomologous*** Two nucleic acid molecules are considered to be "homologous" if their nucleotide sequences share a similarity of greater than 50%, as determined by HASH-coding algorithms (Wilber, W.J. and Lipman, D.J., Proc. Natl. Acad. Sci. 80:726-730 (1983)). Two nucleic acid molecules are considered to be "nonhomologous" if their nucleotide sequences share a similarity of less than 50%.

***Ribozyme**.* A ribozyme is an RNA molecule that contains a catalytic center. The term includes RNA enzymes, self-splicing RNAs, and self-cleaving RNAs.

***Ribozyme Therapy**.* A method of treatment wherein ribozyme is administered to a patient in order to inhibit the translation of the target mRNA.

***Fragment**.* A "fragment" of a molecule such as NTP is meant to refer to any polypeptide subset of that molecule.

***Functional Derivative**.* The term "functional derivatives" is intended to include the "variants," "analogues," or "chemical derivatives" of the molecule. A "variant" of a molecule such as NTP is meant to refer to a naturally occurring molecule substantially similar to either the entire molecule, or a fragment thereof. An "analogue" of a molecule such as NTP is meant to refer to a non-natural molecule substantially similar to either the entire molecule or a fragment thereof.

A molecule is said to be "substantially similar" to another molecule if the sequence of amino acids in both molecules is substantially the same, and if both molecules possess a similar biological activity. Thus, provided that two molecules possess a similar activity, they are considered variants as that term is used herein even if one of the molecules contains additional amino acid residues not found in the other, or if the sequence of amino acid residues is not identical.

As used herein, a molecule is said to be a "chemical derivative" of another molecule when it contains additional chemical moieties not normally a part of the molecule. Such moieties may improve the molecule's solubility, absorption, biological half-life, etc. The moieties may alternatively decrease the toxicity of the molecule, eliminate or attenuate any undesirable side effect of the molecule, etc. Examples of moieties capable of mediating such effects are disclosed in Remington's Pharmaceutical Sciences (1980) and will be apparent to those of ordinary skill in the art.

***NTP**.* The term "NTP" refers to a neural thread protein encoded by the AD3-4DH1 DNA molecule as described herein.

***Immuno-Polymerase Chain Reaction**.* A method for the detection of antigens using specific antibody-DNA conjugates. According to this method, a linker molecule with bispecific binding affinity for DNA and antibodies is used to attach a DNA molecule specifically to an antigen-antibody complex. As a result, a specific antigen-antibody-DNA conjugate is formed. The attached DNA can be amplified by the polymerase chain reaction (PCR) using appropriate oligonucleotide primers. The presence of specific PCR products demonstrates that DNA molecules are attached specifically to antigen-antibody complexes, thus indicating the presence of antigen. (Sano et al., Science 258:120-122 (1992)).

For example, Sano *et al., supra,* constructed a streptavidin-protein A chimera that possesses specific binding affinity for biotin and immunoglobulin G. This chimera (i.e., the "linker molecule") was used to attach a biotinylated DNA specifically to antigen-monoclonal antibody complexes that had been immobilized on microtiter plate wells. A segment of the attached DNA was subsequently amplified by PCR.

### Detailed Description of the Invention

This invention is directed to a neural thread protein (NTP) encoded by AD3-4DH1 DNA molecule, genetic sequences coding for the NTP mRNA or antisense mRNA, expression vectors containing the genetic sequences, recombinant hosts transformed therewith, and RNA for the NTP encoded by AD3-4DH1 DNA molecule and antisense RNA produced by such transformed recombinant host expression. This invention further relates to NTP ribozymes, and recombinant DNA molecules which code for ribozymes for the NTP encoded by AD3-4DH1 DNA molecule and antisense oligonucleotides for the NTP encoded by AD3-4DH1 DNA molecule. This invention further relates to antibodies directed against an NTP, as well as the use of NTP encoded by AD3-4DH1 DNA molecule antibodies and nucleic acid sequences of the NTP encoded by AD3-4DH1 DNA molecule for detection of the presence of an NTP encoded by AD3-4DH1 DNA molecule in biological samples. The invention further relates to the use of coding sequences for the NTP encoded by AD3-4DH1 DNA molecule in gene therapy.

### 1. Isolation of DNA Sequences Coding for Neuronal Thread Proteins

DNA sequences coding for an NTP are derived from a variety of sources. These sources include genomic DNA, cDNA, synthetic DNA, and combinations thereof.

Human NTP genomic DNA can be extracted and purified from any human cell or tissue, by means well known in the art (for example, *see* Sambrook et al., Molecular Cloning: A Laboratory Manual, Second Edition. Cold Spring Harbor Laboratory Press, 1989). The NTP genomic DNA of the invention may or may not include naturally occurring introns. Moreover, such genomic DNA may be obtained in association with the 5' promoter region of the NTP gene sequences and/or with the 3' translational termination region. Further, such genomic DNA may be obtained in association with DNA sequences which encode the 5' nontranslated region of the NTP mRNA and/or with the genetic sequences which encode the 3' nontranslated region. To the extent that a host cell can recognize the transcriptional and/or translational regulatory signals associated with the expression of the mRNA and protein, then the 5' and/or 3' nontranscribed regions of the native gene, and/or, the 5' and/or 3' nontranslated regions of the mRNA, may be retained and employed for transcriptional and translational regulation.

Alternatively, an NTP mRNA can be isolated from any cell which expresses an NTP, and used to produce cDNA by means well known in the art (for example, *see* Sambrook *et al., supra*). Preferably, the mRNA preparation used will be enriched in mRNA coding for an NTP, either naturally, by isolation from cells which produce large amounts of an NTP, or *in vitro,* by techniques commonly used to enrich mRNA preparations for specific sequences, such as sucrose gradient centrifugation, or both. An NTP mRNA may be obtained from mammalian neuronal tissue, or from cell lines derived therefrom. Preferably, human cDNA libraries are constructed from 17-18 week old fetal brain, 2 year old temporal lobe neocortex, end-stage AD cerebral cortex, or from cell lines derived from human neuronal tissue. Such cell lines may include, but are not limited to, central nervous system primitive neuroectodermal tumor cells (such as PNET1 or PNET2, as described herein), neuroblastoma cells (such as SH-Sy5y. as described herein), or human glioma cells (such as A172; ATCC CRL 1620). Alternatively, a rat cDNA library can be prepared from mRNA isolated from rat glioma cells, for example, C6 rat glioma cells (ATCC CCL107).

For cloning into a vector, suitable DNA preparations (either genomic or cDNA) are randomly sheared or enzymatically cleaved, respectively, and ligated into appropriate vectors to form a recombinant gene (either genomic or cDNA) library. A DNA sequence encoding an NTP may be inserted into a vector in accordance with conventional techniques, including blunt-ending or staggered-ending termini for ligation, restriction enzyme digestion to provide appropriate termini, filling in of cohesive ends as appropriate, alkaline phosphatase treatment to avoid undesirable joining, and ligation with appropriate ligases. Techniques for such manipulation are disclosed by Sambrook *et al., supra,* and are well known in the art.

Libraries containing NTP clones may be screened and the NTP clones identified by any means which specifically selects for NTP DNA such as, for example: 1) by hybridization with an appropriate nucleic acid probe(s) containing a sequence specific for the DNA of this protein; or, 2) by hybridization-selected translational analysis in which native mRNA hybridizes to the clone in question, is translated *in vitro*, and the translation products are further characterized; or, 3) if the cloned DNA sequences are themselves capable of expressing mRNA, by immunoprecipitation of a translated NTP product produced by the host containing the clone.

Oligonucleotide probes specific for an NTP which can be used to identify clones to this protein can be designed from knowledge of the amino acid sequence of the corresponding NTP, or homologous regions of the PTP. Alternatively, oligonucleotide probes can be designed from knowledge of the nucleotide sequence of PTP (de la Monte et al., J. Clin. Invest. 86:1004-1013 (1990)).

The suitable oligonucleotide, or set of oligonucleotides, which is capable of encoding a fragment of the NTP gene (or which is complementary to such an oligonucleotide, or set of oligonucleotides) may be synthesized by means well known in the art (for example, *see* Sambrook *et al., supra*). Techniques of nucleic acid hybridization and clone identification are disclosed by Sambrook *et al., supra.* Those members of the above-described gene library which are found to be capable of such hybridization are then analyzed to determine the extent and nature of the NTP encoding sequences which they contain.

To facilitate the detection of the desired NTP coding sequence, the above-described DNA probe is labeled with a detectable group. Such detectable group can be any material having a detectable physical or chemical property. Such materials have been well-developed in the field of nucleic acid hybridization and in general most any label useful in such methods can be applied to the present invention. Particularly useful are radioactive labels including ³²P, ³H, ¹⁴C, ¹²⁵I, or the like. Any radioactive label may be employed which provides for an adequate signal and has sufficient half-life. The DNA probe may be labeled, for example, by nick-translation, by T4 DNA polymerase replacement synthesis, or by random priming, among other methods well known in the art (*see* Sambrook *et al. supra*).

Alternatively, DNA probes can be labeled with non-radioactive markers such as biotin, an enzyme, or fluorescent group.

In an alternative method of cloning NTP DNA sequences, NTP cDNAs are obtained by direct cloning of cDNAs from cell lines and brain tissue, using the 3'- and 5'-RACE methods, as described herein. Preferably, a human neuroectodermal tumor cell line or AD brain tissue is used as a source of mRNA.

### II. Expressing the Gene Coding for NTP

The above-discussed methods are, therefore, capable of identifying DNA sequences which are code for an NTP or fragments thereof. In order to further characterize such DNA sequences, and in order to produce the recombinant protein, it is desirable to express the proteins which the DNA sequences encode.

To express an NTP, transcriptional and translational signals recognizable by an appropriate host are necessary. The cloned NTP DNA sequences, obtained through the methods described above, and preferably in double-stranded form, may be "operably linked" to sequences controlling transcriptional expression in an expression vector, and introduced into a host cell, either prokaryotic or eukaryotic, to produce recombinant NTP. Depending upon which strand of the NTP coding sequence is operably linked to the sequences controlling transcriptional expression, it is also possible to express an NTP antisense RNA.

Expression of the NTP in different hosts may result in different post-translational modifications which may alter the properties of the NTP. Preferably, the present invention encompasses the expression of an NTP in eukaryotic cells, and especially mammalian, insect, and yeast cells. Especially preferred eukaryotic hosts are mammalian cells. Mammalian cells provide post-translational modifications to recombinant NTP which include folding and/or phosphorylation. Most preferably, mammalian host cells include human CNS primitive neuroectodermal tumor cells, human neuroblastoma cells, human glioma cells, or rat glioma cells. Especially preferred primitive neuroectodermal tumor cells include PNET1 and PNET2, especially preferred human glioblastoma cells include Hg16 and Hg17, especially preferred human glioma cells include A 172, and especially preferred rat glioma cells include C6 (*see* Example 1).

Alternatively, an NTP may be expressed by prokaryotic host cells. Preferably, a recombinant NTP is expressed by such cells as a fusion protein, as described herein. An especially preferred prokaryotic host is *E. coli.* Preferred strains of *E. coli* include Y1088, Y1089, CSH18, ER1451. and ER1647 (*see,* for example, Molecular Biology LabFax, Brown. T.A.. Ed. , Academic Press, New York (1991)). An alternative preferred host is *Bacillus subtilus,* including such strains as BR151, YB886, MI119, MI120, and B170 (*see,* for example, Hardy, "Bacillus Cloning Methods," in DNA Cloning: A Practical Approach, IRL Press, Washington, D.C. (1985)).

A nucleic acid molecule, such as DNA, is said to be "capable of expressing" a polypeptide if it contains expression control sequences which in turn contain transcriptional regulatory information and such sequences are "operably linked" to the nucleotide sequence which encodes the protein.

Two sequences of a nucleic acid molecule are said to be operably linked when they are linked to each other in a manner which either permits both sequences to be transcribed onto the same RNA transcript, or permits an RNA transcript, begun in one sequence to be extended into the second sequence. Thus, two sequences, such as a promoter sequence and any other "second" sequence of DNA or RNA are operably linked if transcription commencing in the promoter sequence will produce an RNA transcript of the operably linked second sequence. In order to be operably linked it is not necessary that two sequences be immediately adjacent to one another.

The promoter sequences of the present invention may be either prokaryotic, eukaryotic or viral. Suitable promoters are repressible, constitutive, or inducible. Examples of suitable prokaryotic promoters include promoters capable of recognizing the T4 polymerases (Malik et al., J. Biol. Chem. 263:1174-1181 (1984); Rosenberg et al., Gene 59:191-200 (1987); Shinedling et al., J. Molec. Biol. 195:471-480 (1987); Hu et al., Gene 42:21-30 (1986)), T3, Sp6, and T7 (Chamberlin et al., Nature 228:227-231 (1970); Bailey et al., Proc. Natl. Acad. Sci. (U.S.A.) 80:2814-2818 (1983); Davanloo et al., Proc. Natl. Acad. Sci. (U.S.A.) 81:2035-2039 (1984)); the P_{R} and P_{L} promoters of bacteriophage lambda (The Bacteriophage Lambda, Hershey, A.D., Ed., Cold Spring Harbor Press, Cold Spring Harbor, NY (1973); Lambda II, Hendrix, R.W., Ed., Cold Spring Harbor Press, Cold Spring Harbor, NY (1980)); the trp, recA, heat shock, and lacZ promoters of *E. coli;* the α-amylase (Ulmanen et al., J. Bacteriol. 162:176-182 (1985)) and the delta-28-specific promoters of *B. subtilis* (Gilman et al., Gene 32:11-20 (1984)); the promoters of the bacteriophages of *Bacillus* (Gryczan, In: The Molecular Biology of the Bacilli, Academic Press, Inc., NY (1982)); *Streptomyces* promoters (Ward et al., Mol. Gen. Genet. 203:468-478 (1986)); the *int* promoter of bacteriophage lambda; the *bla* promoter of the β-lactamase gene of pBR322, and the CAT promoter of the chloramphenicol acetyl transferase gene of pBR325, etc. Prokaryotic promoters are reviewed by Glick, J. Ind. Microbiol. 1:277-282 (1987); Cenatiempo, Biochimie 68:505-516 (1986); Watson et al., In: Molecular Biology of the Gene, Fourth Edition, Benjamin Cummins, Menlo Park, CA (1987); Gottesman, Ann. Rev. Genet. 18:415-442 (1984); and Sambrook *et al., supra.*

Preferred eukaryotic promoters include the promoter of the mouse metallothionein I gene (Hamer et al., J. Mol. Appl. Gen. 1:273-288 (1982)); the TK promoter of Herpes virus (McKnight, Cell 31:355-365 (1982)); the SV40 early promoter (Benoist, et al., Nature (London) 290:304-310 (1981)); and the yeast *gal4* gene promoter (Johnston, et al., Proc. Natl. Acad. Sci. (USA) 79:6971-6975 (1982); Silver, et al., Proc. Natl. Acad. Sci. (USA) 81:5951-5955 (1984)).

Strong promoters are the most preferred promoters of the present invention. Examples of such preferred promoters are those which recognize the T3, SP6 and T7 polymerase promoters, the P_{L} promoter of bacteriophage lambda; the *recA* promoter and the promoter of the mouse metallothionein I gene. The most preferred promoter for expression in prokaryotic cells is one which is capable of recognizing the T7 polymerase promoter. The sequences of such polymerase recognition sequences are disclosed by Watson, et al. (In: Molecular Biology of the Gene, Fourth Edition, Benjamin Cummins, Menlo Park, CA, (1987)). The most preferred promoter for expression in mammalian cells is SV40 (Gorman, "High Efficiency Gene Transfer into Mammalian cells," in DNA Cloning: A Practical Approach, Volume II, IRL Press, Washington, D.C., pp. 143-190 (1985)).

### 111. Methods of Detecting NTP

This invention is directed towards methods of detecting neurological disease in a human subject, utilizing the nucleic acid probes hybridizable to NTP genes or transcripts of an NTP encoded by AD3-4DH1 DNA molecules, or antibodies specific for an NTP encoded by AD3-4DH1 DNA molecules. By "neurological disease" is meant Alzheimer's Disease (AD), or other neurodegenerative disorders with the Alzheimer's type pathogenic changes (for example, Parkinson's disease with AD-type neurodegeneration), as well as neuroectodermal tumors, malignant astrocytomas, and glioblastomas. By "human subject" is meant any human being or any developmental form thereof, such as a human embryo or fetus, prior to birth. The diagnostic methods of the present invention do not require invasive removal of neural tissue.

The present invention additionally pertains to assays, both nucleic acid hybridization assays and immunoassays, for detecting the presence of NTP encoded by AD3-4DH1 DNA molecules in cells or in the biological fluids of a human subject using light or electron microscopic histology, imaging, radioactive or enzyme based assays, and the like.

### a. Nucleic Acid Hybridization Assays

In testing a tissue sample for an NTP using a nucleic acid hybridization assay, RNA can be isolated from tissue by sectioning on a cryostat and lysing the sections with a detergent such as SDS and a chelating agent such as EDTA, optionally with overnight digestion with proteinase K (50 µg/ml). Such tissue is obtained by autopsy and biopsy. A preferred quantity of tissue is in the range of 1-10 milligrams. Protein is removed by phenol and chloroform extractions, and nucleic acids are precipitated with ethanol. RNA is isolated by chromatography on an oligo dT column and then eluted therefrom. Further fractionation can also be carried out, according to methods well known to those of ordinary skill in the art.

A number of techniques for molecular hybridization are used for the detection of DNA or RNA sequences in tissues; each has certain advantages and disadvantages. When large amounts of tissue are available, analysis of hybridization kinetics provides the opportunity to accurately quantitate the amount of DNA or RNA present, as well as to distinguish sequences that are closely related but not identical to the probe, and determine the percent homology.

Reactions are run under conditions of hybridization (Tm-25°C) in which the rate of reassociation of the probe is optimal (Wetmur et al., J. Mol. Biol. 31:349-370 (1968)). The kinetics of the reaction are second- order when the sequences in the tissue are identical to those of the probe: however, the reaction exhibits complex kinetics when probe sequences have partial homology to those in the tissue (Sharp et al., J. Mol. Biol. 86:709-726 (1974)).

The ratio of probe to cell RNA is determined by the sensitivity desired. To detect one transcript per cell would require about 100 pg of probe per µg of total cellular DNA or RNA. The nucleic acids are mixed, denatured, brought to the appropriate salt concentration and temperature, and allowed to hybridize for various periods of time. The rate of reassociation can be determined by quantitating the amount of probe hybridized either by hydroxy apatite chromatography (Britten et al., Science 161:529-540 (1968)) or S1 nuclease digestion (Sutton, Biochim. Biophys. Acta 240:522-531 (1971)).

A more flexible method of hybridization is the northern blot technique. This technique offers variability in the stringency of the hybridization reaction, as well as determination of the state of the retroviral sequences in the specimen under analysis. Northern analysis can be performed as described herein.

A major consideration associated with hybridization analysis of DNA or RNA sequences is the degree of relatedness the probe has with the sequences present in the specimen under study. This is important with the blotting technique, since a moderate degree of sequence homology under nonstringent conditions of hybridization can yield a strong signal even though the probe and sequences in the sample represent non-homologous genes.

The particular hybridization technique is not essential to the invention, any technique commonly used in the art being within the scope of the present invention. Typical probe technology is described in United States Patent 4,358,535 to Falkow et al*.* For example, hybridization can be carried out in a solution containing 6 x SSC (10x SSC: 1.5 M sodium chloride, 0.15 M sodium citrate, pH 7.0), 5 x Denhardt's (1 x Denhardt's: 0.2% bovine serum albumin, 0.2% polyvinylpyrrolidone, 0.02% Ficoll 400), 10 mM EDTA, 0.5 % SDS and about 10⁷ cpm of nick-translated DNA for 16 hours at 65°C.

The labeled probes, as described above, provide a general diagnostic method for detection of an NTP in tissue. The method is reasonably rapid, has a simple protocol, has reagents which can be standardized and provided as commercial kits, and allows for rapid screening of large numbers of samples.

In one method for carrying out the procedure, a clinical isolate containing RNA transcripts is fixed to a support. The affixed nucleic acid is contacted with a labeled polynucleotide having a base sequence complementary or homologous to the coding strand of the NTP gene.

The hybridization assays of the present invention are particularly well suited for preparation and commercialization in kit form, the kit comprising a carrier means compartmentalized to receive one or more container means (vial, test tube, etc.) in close confinement, each of said container means comprising one of the separate elements to be used in hybridization assay.

For example, there may be a container means containing NTP cDNA molecules suitable for labeling by "nick translation" (*see,* for example, *Sambrook et al., supra,* for standard methodology), or labeled NTP cDNA or RNA molecules. Further container means may contain standard solutions for nick translation of NTP cDNA comprising DNA polymerase I/DNase I and unlabeled deoxyribonucleotides (i.e., dCTP, dTTP, dGTP, and dATP).

The presence of NTP RNA is determined by the variation in the appearance and/or quantity of probe-related RNA in tested tissue.

The DNA probes of this invention can also be used for differential diagnosis of hereditary or familial AD and non-hereditary or sporadic AD. The familial form of AD often occurs at an earlier age and is associated with Down's syndrome in the family. Thus, a genetic test for familial AD allows for genetic counseling of families. While much effort has been directed toward characterizing a genetic marker for familial AD (Gusella, FASEB J 3:2036-2041 (1989); Hooper, J NIH Res. 4:48-54 (1992)), genetic linkage analysis only identifies a genetic marker sequence without providing the knowledge of the function of the genomic sequence. In contrast, the cDNA probes described herein and obtained from individuals with sporatic AD encode a known protein of known function which is over-expressed in brain tissue of patients with AD.

Most cases of the AD disorder appear to be the sporadic form, although there are well-documented familial cases (Gusella, *supra;* Harrison's Principles of Internal Medicine, Braunwald et al., Eds., Eleventh Edition, Mc-Graw-Hill Book Company, New York, pp. 2012-2013 (1987)). A patient with familial AD. unlike a patient with sporadic AD, inherited the predisposing mutation through the germ cells. Some of the familial cases have been shown to follow an autosomal dominant pattern of inheritance (*Id*.). Thus, the DNA of a patient with familial AD will contain the inherited genetic alteration which is absent from the DNA of a patient with sporadic AD.

A method of differentiating between sporadic and familial AD in a human subject involves obtaining a biological sample from the human subject who is suspected of having Alzheimer's Disease. Then, DNA is purified from the biological sample. Finally, the DNA is contacted with a NTP DNA probe under conditions of hybridization. Familial AD is indicated by the detection of a hybrid of the probe and the DNA, whereas sporadic AD is indicated by the absence of detection of hybridization.

For example, the biological sample can be a blood sample which is subjected to differential centrifugation to enrich for white blood cells within three days of collection (Park, "PCR in the Diagnosis of Retinoblastoma," in PCR Protocols, Innis et al., Eds., Academic Press, Inc., New York, pp. 407-415 (1990)). The DNA sample can be prepared using the sodium N-lauroylsarcosine-Proteinase K, phenol, and RNase method (Sambrook *et al., supra*)*.* DNA analysis can be performed by digesting the DNA sample, preferably 5 micrograms, with a restriction endonuclease (such as *Hind*III)*.* Digested DNA is then fractionated using agarose gel electrophoresis, preferably, a 1 % horizontal agarose gel, for 18 hours in a buffer preferably containing 89 mM Tris-Hcl (pH 8), 89 mM sodium borate and 2 mM EDTA (Gusella et al., Nature 306:234-238 (1983)). Southern analysis can be performed using conventional techniques (Sambrook *et al., supra*)*,* and the labelled AD cDNA probes can be hybridized under conditions described above. The preferred DNA probes for this differential diagnosis method include 1-9a, AD3-4, AD4-4 and G2-2 PstI.

### b. Immunoassays

Antibodies directed against an NTP can be used, as taught by the present invention, to detect and diagnose AD. Various histological staining methods, including immunohistochemical staining methods, may also be used effectively according to the teaching of the invention. Silver stain is but one method of visualizing NTP. Other staining methods useful in the present invention will be obvious to the artisan, the determination of which would not involve undue experimentation (*see generally,* for example, A Textbook of Histology, Eds. Bloom and Fawcett, W. B. Saunders Co., Philadelphia (1964)).

One screening method for determining whether a given compound is an NTP functional derivative comprises, for example, immunoassays employing radioimmunoassay (RIA) or enzyme-linked immunosorbant assay (ELISA) methodologies, based on the production of specific antibodies (monoclonal or polyclonal) to an NTP. For these assays, biological samples are obtained by venepuncture (blood), spinal tap (cerebral spinal fluid (CSF)), urine and other body secretions such as sweat and tears. For example, in one form of RIA, the substance under test is mixed with diluted antiserum in the presence of radiolabeled antigen. In this method, the concentration of the test substance will be inversely proportional to the amount of labeled antigen bound to the specific antibody and directly related to the amount of free labeled antigen.Other suitable screening methods will be readily apparent to those of skill in the art.

The present invention also relates to methods of detecting an NTP molecule encoded by AD3-4DH1 DNA molecule or functional derivatives in a sample or subject. For example, antibodies specific for an NTP, or a functional derivative, may be detectably labeled with any appropriate marker, for example, a radioisotope, an enzyme, a fluorescent label, a paramagnetic label, or a free radical.

Alternatively, antibodies specific for an NTP, or a functional derivative, may be detectably labeled with DNA by the technique of immunopolymerase chain reaction (Sano et al., Science 258: 120-122 (1992)). The polymerase chain reaction (PCR) procedure amplifies specific nucleic acid sequences through a series of manipulations including denaturation, annealing of oligonucleotide primers, and extension of the primers with DNA polymerase (*see*, for example, Mullis et al., U.S. Patent No. 4,683,202; Mullis et al., U.S. Patent No. 4,683,195; Loh et al., Science 243:217 (1988)). The steps can be repeated many times, resulting in a large amplification of the number of copies of the original specific sequence. As little as a single copy of a DNA sequence can be amplified to produce hundreds of nanograms of product (Li et al., Nature 335:414 (1988)). Other known nucleic acid amplification procedures include transcription-based amplification systems (Kwoh et al., Proc. Natl. Acad. Sci. USA 86:1173 (1989); Gingeras et al., WO 88/10315), and the "ligase chain reaction" in which two (or more) oligonucleotides are ligated in the presence of a nucleic acid target having the sequence of the resulting "di-oligonucleotide" thereby amplifying the di oligonucleotide (Wu et al., Genomics 4:560 (1989); Backman et al., EP 320,308; Wallace, EP 336,731; Orgel, WO 89/09835). For example, the immuno-PCR assay can be carried out by immobilizing various amounts of the test material on the surface of microtiter wells (*see* Sanzo *et al., supra,* page 122, footnote 7). The wells are subsequently incubated with an NTP monoclonal antibody, washed, and then incubated with biotinylated NTP DNA molecules which have been conjugated to streptavidin-protein chimera (*Id*.). This chimera binds biotin (via the streptavidin moiety) and the Fc portion of an immunoglobulin G molecule (via the protein A moiety) (*Id.,* at 120; Sanzo et al., Bio/Technology 9:1378 (1991)). The wells are then washed to remove unbound conjugates. Any NTP present in the test material will be bound by the NTP monoclonal antibody, which in turn, is bound by the protein A moiety of the biotinylated NTP DNA - streptavidin-protein A conjugate. Then, the NTP DNA sequences are amplified using PCR. Briefly, the microtiter wells are incubated with deoxyribonucleoside triphosphates, NTP oligonucleotide primers, and Taq DNA polymerase (*see* Sanzo *et al., supra,* page 122, footnote 11). An automated thermal cycler (such as the PTC-100-96 Thermal Cycler, MJ Research, Inc.) can be used to perform PCR under standard conditions (*Id.*). The PCR products are then analyzed by agarose gel electrophoresis after staining with ethidium bromide.

Methods of making and detecting such detectably labeled antibodies or their functional derivatives are well known to those of ordinary skill in the art, and are described in more detail below. Standard reference works setting forth the general principles of immunology include the work of Klein (Immunology: The Science of Self-Nonself Discrimination, John Wiley & Sons, New York (1982)); Kennett et al. (Monoclonal Antibodies and Hybridomas: A New Dimension in Biological Analyses, Plenum Press, New York (1980)); Campbell ("Monoclonal Antibody Technology," In: Laboratory Techniques in Biochemistry and Molecular Biology, Volume 13 (Burdon, R., et al., eds.). Elsevier. Amsterdam (1984)); and Eisen (In: Microbiology, 3rd Ed. (Davis, et al., Harper & Row, Philadelphia (1980)).

The term "antibody" refers both to monoclonal antibodies which are a substantially homogeneous population and to polyclonal antibodies which are heterogeneous populations. Polyclonal antibodies are derived from the sera of animals immunized with an antigen. Monoclonal antibodies (mAbs) to specific antigens may be obtained by methods known to those skilled in the art. *See,* for example, Kohler and Milstein, Nature 256:495-497 (1975) and U.S. Patent No. 4,376,110. Such antibodies may be of any immunoglobulin class including IgG, IgM, IgE, IgA, IgD and any subclass thereof.

The monoclonal antibodies, particularly mAbs Th7, Th9, and Th10 used in the present invention, may be prepared as previously described (Gross et al., J. Clin. Invest. 76:2115-2126 (1985); Ozturk et al., Proc. Natl. Acad. Sci. USA 86:419-423 (1989); de la Monte et. al., J. Clin. Invest. 86:1004-1013 (1990); de la Monte et. al., J. Neurol. Sci. 113:152-164 (1992); de la Monte et al., Ann. Neurol. 32:733-742 (1992)). The Th monoclonal antibodies were generated against the purified pancreatic form of thread protein (*Id*.). NTP-specifc polyclonal and monoclonal antibodies can also be generated against a substantially pure NTP isolated from recombinant hosts (for example, *see* Carroll et al., "Production and Purification of Polyclonal Antibodies to the Foreign Segment of β-Galactosidase Fusion Proteins," in DNA Cloning: A Practical Approach, Volume III, IRL Press, Washington, D.C., pp. 89-111 (1987); Mole et al., "Production of Monoclonal Antibodies Against Fusion Proteins Produced in Escherichia coli," in DNA Cloning: A Practical Approach, Volume III, IRL Press, Washington, D.C., pp. 113-1139: (1987)). Alternatively, NTP-specific polyclonal and monoclonal antibodies can be generated against a substantially pure NTP isolated from biological material such as brain tissue and cell lines, by using well known techniques.

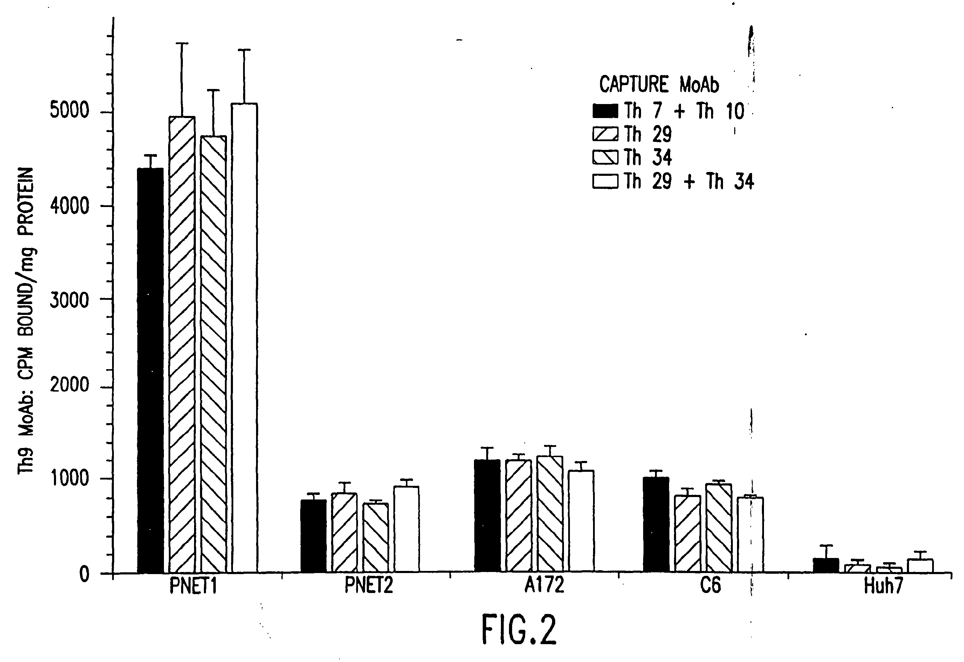

For example, monoclonal antibodies specific for the NTP molecules molecules encoded by AD3-4DH1 DNA molecule may be prepared from recombinant-derived proteins, which are expressed, isolated and purified from the cDNA (i.e., 1-9a), genomic clones (G2-2 *Pst*I) and AD-NTP 3-4 cDNA clones. These NTP molecules are derived from the above cDNA's and genomic clones, inserted and produced in suitable expression vectors (*see* Figures 2A and 2B). Since there are regions of 60-70% homology in the 5' ends of the 1-9a NTP cDNA and PTP, one can obtain monoclonal antibodies that bind specifically to the NTP recombinant proteins and not to the pancreatic form by performing routine differential screening (*see,* for example, de la Monte et al., J. Clin. Invest. 86: 1004-1013 (1990)). Although there will be monoclonal antibodies that bind to both NTP and PTP, it will be possible to generate NTP-specific monoclonal antibodies because there is a substantial sequence divergence between NTP molecules of various forms (e.g., 8, 14, 17, 21, 26 and 42 kDa) and because an epitope may be defined by as few as 6-8 amino acids.

The term "antibody" is also meant to include both intact molecules as well as fragments thereof, such as, for example, Fab and F(ab')₂, which are capable of binding antigen. Fab and F(ab')₂ fragments lack the Fc fragment of intact antibody, clear more rapidly from the circulation, and may have less non-specific tissue binding than an intact antibody (Wahl et al., J. Nucl. Med. 24:316-325 (1983)).

It will be appreciated that Fab and F(ab'), and other fragments of the antibodies useful in the present invention may be used for the detection and quantitation of an NTP according to the methods disclosed herein in order to detect and diagnose AD in the same manner as an intact antibody. Such fragments are typically produced by proteolytic cleavage, using enzymes such as papain (to produce Fab fragments) or pepsin (to produce F(ab'), fragments).

An antibody is said to be "capable of binding" a molecule if it is capable of specifically reacting with the molecule to thereby bind the molecule to the antibody. The term "epitope" is meant to refer to that portion of any molecule capable of being bound by an antibody which can also be recognized by that antibody. Epitopic determinants usually consist of chemically active surface groupings of molecules such as amino acids or sugar side chains and have specific three dimensional structural characteristics as well as specific charge characteristics.

An "antigen" is a molecule capable of being bound by an antibody which is additionally capable of inducing an animal to produce antibody capable of binding to an epitope of that antigen. An antigen may have one, or more than one epitope. The specific reaction referred to above is meant to indicate that the antigen will react, in a highly selective manner, with its corresponding antibody and not with the multitude of other antibodies which may be evoked by other antigens.

The antibodies, or fragments of antibodies, useful in the present invention may be used to quantitatively or qualitatively detect the presence of cells which contain the NTP antigens. Thus, the antibodies (or fragments thereof) useful in the present invention may be employed histologically to detect or visualize the presence of an NTP.

Such an assay for an NTP typically comprises incubating a biological sample from said subject suspected of having such a condition in the presence of a detectably labeled binding molecule (e.g., antibody) capable of identifying an NTP, and detecting said binding molecule which is bound in a sample.

Thus, in this aspect of the invention, a biological sample may be treated with nitrocellulose, or other solid support which is capable of immobilizing cells, cell particles or soluble proteins. The support may then be washed with suitable buffers followed by treatment with the detectably labeled NTP-specific antibody. The solid phase support may then be washed with the buffer a second time to remove unbound antibody. The amount of bound label on said solid support may then be detected by conventional means.

By "solid phase support" is intended any support capable of binding antigen or antibodies. Well-known supports, or carriers, include glass, polystyrene, polypropylene, polyethylene, dextran, nylon, amylases, natural and modified celluloses, polyacrylamides, agaroses, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will note many other suitable carriers for binding monoclonal antibody or antigen, or will be able to ascertain the same by use of routine experimentation.

One embodiment for carrying out the diagnostic assay of the present invention on a biological sample containing an NTP, comprises:
(a) contacting a detectably labeled specific for an NTP encoded by AD3-4DH1 DNA molecule antibody with a solid support to effect immobilization of said NTP-specific antibody or a fragment thereof;
(b) contacting a sample suspected of containing an NTP encoded by AD3-4DH1 DNA molecule with said solid support;
(c) incubating said detectably labeled specific for an NTP encoded by AD3-4DH1 DNA. molecule antibody with said support for a time sufficient to allow the immobilized NTP-specific antibody to bind to the NTP;
(d) separating the solid phase support from the incubation mixture obtained in step (c); and
(e) detecting the bound label and thereby detecting and quantifying NTP.

Alternatively, labeled NTP-specific antibody/NTP complexes in a sample may be separated from a reaction mixture by contacting the complex with an immobilized antibody or protein which is specific for an immunoglobulin, e.g., *Staphylococcus* protein A, *Staphylococcus* protein G, anti-IgM or anti-IgG antibodies. Such anti-immunoglobulin antibodies may be polyclonal, but are preferably monoclonal. The solid support may then be washed with a suitable buffer to give an immobilized NTP/labeled NTPspecific antibody complex. The label may then be detected to give a measure of an NTP.

This aspect of the invention relates to a method for detecting an NTP encoded by AD3-4DH1 DNA molecule or a fragment thereof in a sample comprising:
(a) contacting a sample suspected of containing an NTP with an NTP-specific antibody or fragment thereof which binds to NTP encoded by AD3-4DH1; and
(b) detecting whether a complex is formed.

The invention also relates to a method of detecting an NTP encoded by AD3-4DH1 in a sample, further comprising:
(c) contacting the mixture obtained in step (a) with an Fc binding molecule, such as an antibody, *Staphylococcus* protein A, or *Staphylococcus* protein G, which is immobilized on a solid phase support and is specific for the NTP-specific antibody to give a NTP/NTP-specific antibody encoded by AD3-4DH1 DNA molecule immobilized antibody complex;
(d) washing the solid phase support obtained in step (c) to remove unbound NTP/NTP-specific antibody complex;
(e) and detecting the label bound to said solid support.

Of course, the specific concentrations of detectably labeled antibody and NTP, the temperature and time of incubation, as well as other assay conditions may be varied, depending on various factors including the concentration of an NTP in the sample, the nature of the sample, and the like. The binding activity of a given lot of anti-NTP antibody may be determined according to well known methods. Those skilled in the art will be able to determine operative and optimal assay conditions for each determination by employing routine experimentation.

Other such steps as washing, stirring, shaking, filtering and the like may be added to the assays as is customary or necessary for the particular situation.

One of the ways in which the NTP-specific antibody can be detectably labeled is by linking the same to an enzyme. This enzyme, in turn, when later exposed to its substrate, will react with the substrate in such a manner as to produce a chemical moiety which can be detected, for example, by spectrophotometric, fluorometric or by visual means. Enzymes which can be used to detectably label the NTP-specific antibody include, but are not limited to, malate dehydrogenase staphylococcal nuclease, delta-V-steroid isomerase, yeast alcohol dehydrogenase, α-glycerophosphate dehydrogenase, triose phosphate isomerase, horseradish peroxidase, alkaline phosphatase, asparaginase, glucose oxidase, β-galactosidase, ribonuclease, urease, catalase, glucose-VI-phosphate dehydrogenase, glucoamylase and acetylcholinesterase.

Detection may be accomplished using any of a variety of immunoassays. For example, by radioactively labeling the NTP-specific antibodies or antibody fragments, it is possible to detect NTP through the use of radioimmune assays. A good description of a radioimmune assay may be found in Laboratory Techniques and Biochemistry in Molecular Biology, by Work, et al., North Holland Publishing Company, NY (1978), with particular reference to the chapter entitled "An Introduction to Radioimmune Assay and Related Techniques" by Chard.

The radioactive isotope can be detected by such means as the use of a gamma counter or a scintillation counter or by autoradiography. Isotopes which are particularly useful for the purpose of the present invention are: ³H, ¹²⁵I, ¹³¹I ³⁵S, ¹⁴C, and preferably ¹²⁵I.

It is also possible to label the NTP-specific antibody with a fluorescent compound. When the fluorescently labeled antibody is exposed to light of the proper wave length, its presence can then be detected due to fluorescence. Among the most commonly used fluorescent labelling compounds are fluorescein isothiocyanate, rhodamine, phycoerytherin, phycocyanin, allophycocyanin, o-phthaldehyde and fluorescamine.

The NTP-specific antibody can also be detectably labeled using fluorescence emitting metals such as ¹⁵²Eu, or others of the lanthanide series. These metals can be attached to the NTP-specific antibody using such metal chelating groups as diethylenetriaminepentaacetic acid (DTPA) or ethylenediaminetetraacetic acid (EDTA).

The NTP-specific antibody also can be detectably labeled by coupling is to a chemiluminescent compound. The presence of the chemiluminescent-tagged NTP-specific antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

The NTP-specific antibody may also be labeled with biotin and then reacted with avidin. A biotin-labeled DNA fragment will be linked to the NTP-biotinylated monoclonal antibody by an avidin bridge. NTP molecules are then detected by polymerase chain reaction (PCR) amplification of the DNA fragment with specific primers (Sano et al., Science 258:120-122 (1992)).

Likewise, a bioluminescent compound may be used to label the NTP-specific antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

Detection of the NTP-specific antibody may be accomplished by a scintillation counter, for example, if the detectable label is a radioactive gamma emitter, or by a fluorometer, for example, if the label is a fluorescent material. In the case of an enzyme label, the detection can be accomplished by colorimetric methods which employ a substrate for the enzyme. Detection may also be accomplished by visual comparison of the extent of enzymatic reaction of a substrate in comparison with similarly prepared standards.

The detection of foci of such detectably labeled antibodies is indicative of a disease or dysfunctional state as previously described. For the purposes of the present invention, the NTP which is detected by this assay may be present in a biological sample. Any sample containing an NTP can be used. However, one of the benefits of the present diagnostic invention is that invasive tissue removal may be avoided. Therefore, preferably, the sample is a biological solution such as, for example, cerebrospinal fluid, amniotic fluid, blood, serum, urine and the like. However, the invention is not limited to assays using only these samples, it being possible for one of ordinary skill in the art to determine suitable conditions which allow the use of other samples.

For example, the three-site monoclonal antibody-based immunoradiometric assays (M-IRMA) may be used to measure NTP levels in a biological fluid, such as CSF. It is possible to obtain, by spinal tap, on a routine basis, CSF from individuals suspected of having AD. Thus, the diagnosis of AD can be established by a simple, non-invasive immunoassay which reveals NTP levels greatly increased over normal levels.

In one embodiment, as described above, this examination for AD is accomplished by removing samples of biological fluid and incubating such samples in the presence of detectably labeled antibodies (or antibody fragments). In a preferred embodiment, this technique is accomplished in a non-invasive manner through the use of magnetic imaging, fluorography, etc.

There are many different *in vivo* labels and methods of labeling known to those of ordinary skill in the art. Examples of the types of labels which can be used in the present invention include radioactive isotopes and paramagnetic isotopes. Those of ordinary skill in the art will know of other suitable labels for binding to the antibodies used in the invention, or will be able to ascertain such, using routine experimentation. Furthermore, the binding of these labels to the antibodies can be done using standard techniques common to those of ordinary skill in the art.

The antibodies (or fragments thereof) useful in the present invention are also particularly suited for use in in vitro immunoassays to detect the presence of an NTP in body tissue, fluids (such as CSF), or cellular extracts. In such immunoassays, the antibodies (or antibody fragments) may be utilized in liquid phase or, preferably, bound to a solid-phase carrier, as described above.

Those of ordinary skill in the art will know of other suitable labels which may be employed in accordance with the present invention. The binding of these labels to antibodies or fragments thereof can be accomplished using standard techniques commonly known to those of ordinary skill in the art. Typical techniques are described by Kennedy, et al. (Clin. Chim. Acta 70:1-31 (1976)) and Schurs, et al. (Clin. Chim. Acta 81:1-40 (1977)). Coupling techniques mentioned in the latter are the glutaraldehyde method, the periodate method, the dimaleimide method, the m-maleimidobenzyl-Nhydroxy-succinimide ester method.

*In situ* detection may be accomplished by removing a histological specimen from a patient, and providing thecombinationof labeled antibodies of the present invention to such a specimen. The antibody (or fragment) is preferably provided by applying or by overlaying the labeled antibody (or fragment) to a biological sample. Through the use of such a procedure, it is possible to determine not only the presence of an NTP, but also the distribution of an NTP on the examined tissue. Using the present invention, those of ordinary skill will readily perceive that any of a wide variety of histological methods (such as staining procedures) can be modified in order to achieve such *in situ* detection.

The binding molecules of the present invention may be adapted for utilization in an immunometric assay, also known as a "two-site" or "sandwich" assay. In a typical immunometric assay, a quantity of unlabeled antibody (or fragment of antibody) is bound to a solid support that is insoluble in the fluid being tested (i.e., CSF) and a quantity of detectably labeled soluble antibody is added to permit detection and/or quantitation of the ternary complex formed between solid-phase antibody, antigen, and labeled antibody.

Typical, and preferred, immunometric assays include "forward" assays in which the antibody bound to the solid phase is first contacted with the sample being tested to extract the antigen from the sample by formation of a binary solid phase antibody-antigen complex. After a suitable incubation period, the solid support is washed to remove the residue of the fluid sample, including unreacted antigen, if any, and then contacted with the solution containing an unknown quantity of labeled antibody (which functions as a "reporter molecule"). After a second incubation period to permit the labeled antibody to complex with the antigen bound to the solid support through the unlabeled antibody, the solid support is washed a second time to remove the unreacted labeled antibody. This type of forward sandwich assay may be a simple "yes/no" assay to determine whether antigen is present or may be made quantitative by comparing the measure of labeled antibody with that obtained for a standard sample containing known quantities of antigen. Such "two-site" or "sandwich" assays are described by Wide at pages 199-206 of Radioimmune Assay Method, edited by Kirkham and Hunter, E. & S. Livingstone, Edinburgh, 1970.

ln another type of "sandwich" assay, which may also be useful with the antigens of the present invention, the so-called "simultaneous" and "reverse" assays are used. A simultaneous assay involves a single incubation step as the antibody bound to the solid support and labeled antibody are both added to the sample being tested at the same time. After the incubation is completed, the solid support is washed to remove the residue of fluid sample and uncomplexed labeled antibody. The presence of labeled antibody associated with the solid support is then determined as it would be in a conventional "forward" sandwich assay.

In the "reverse" assay, stepwise addition first of a solution of labeled antibody to the fluid sample followed by the addition of unlabeled antibody bound to a solid support after a suitable incubation period is utilized. After a second incubation, the solid phase is washed in conventional fashion to free it of the residue of the sample being tested and the solution of unreacted labeled antibody. The determination of labeled antibody associated with a solid support is then determined as in the "simultaneous" and "forward" assays.

The above-described *in vitro* or *in vivo* detection methods may be used in the detection and diagnosis of AD without the necessity of removing tissue. Such detection methods may be used to assist in the determination of the stage of neurological deterioration in AD by evaluating and comparing the concentration of an NTP in the biological sample.

As used herein, an effective amount of a diagnostic reagent (such as an antibody or antibody fragment) is one capable of achieving the desired diagnostic discrimination and will vary depending on such factors as age, condition, sex, the extent of disease of the subject, counterindications, if any, and other variables to be adjusted by the physician. The amount of such materials which are typically used in a diagnostic test are generally between 0.1 to 5 mg, and preferably between 0.1 to 0.5 mg.

The assay of the present invention is also ideally suited for the preparation of a kit. Such a kit may comprise a carrier means being compartmentalized to receive in close confinement therewith one or more container means such as vials, tubes and the like, each of said container means comprising the separate elements of the immunoassay.

For example, there may be a container means containing a first antibody immobilized on a solid phase support, and a further container means containing a second detectably labeled antibody in solution. Further container means may contain standard solutions comprising serial dilutions of the NTP to be detected. The standard solutions of an NTP may be used to prepare a standard curve with the concentration of NTP plotted on the abscissa and the detection signal on the ordinate. The results obtained from a sample containing an NTP may be interpolated from such a plot to give the concentration of the NTP.

### IV. Isolation of NTP

The NTP proteins encoded by AD3-4DH1 DNA molecule or fragments of this invention may be obtained by expression from recombinant DNA as described above. Alternatively, the NTP encoded by AD3-4DH1 DNA molecule may be purified from biological material.

For purposes of the present invention, one method of purification which is illustrative, without being limiting, consists of the following steps.

A first step in the purification of an NTP encoded by AD3-4DH1 DNA molecule includes extraction of the NTP fraction from a biological sample, such as brain tissue or CSF, in buffers, with or without solubilizing agents such as urea, formic acid, detergent, or thiocyanate.

A second step includes subjecting the solubilized material to ionexchange chromatography on Mono-Q or Mono-S columns (Pharmacia LKB Biotechnology, Inc; Piscataway, NJ). Similarly, the solubilized material may be separated by any other process wherein molecules can be separated according to charge density, charge distribution and molecular size, for example. Elution of the NTP from the ionexchange resin are monitored by an immunoassay, such as M-IRMA, on each fraction. Immunoreactive peaks would are then dialyzed, lyophilized, and subjected to molecular sieve, or gel chromatography.

Molecular sieve or gel chromatography is a type of partition chromatography in which separation is based on molecular size. Dextran, polyacrylamide, and agarose gels are commonly used for this type of separation. One useful gel for the present invention is Sepharose 12 (Pharmacia LKB Biotechnology, Inc.). However, other methods, known to those of skill in the art may be used to effectively separate molecules based on size.

A fourth step in a purification protocol for an NTP includes analyzing the immunoreactive peaks by sodium dodecyl sulfate-polyacrylamide gel electrophoresis (SDS-PAGE), a further gel chromatographic purification step, and staining, such as, for example, silver staining.

A fifth step in a purification method includes subjecting the NTP obtained after SDS-PAGE to affinity chromatography, or any other procedure based upon affinity between a substance to be isolated and a molecule to which it can specifically bind. For further purification of an NTP, affinity chromatography on Sepharose conjugated to anti-NTP mAbs (such as Th9, or specific mABs generated against substantially pure NTP) can be used. Alternative methods, such as reverse-phase HPLC, or any other method characterized by rapid separation with good peak resolution are useful.

Another method to purify the NTP encoded by AD3-4DH1 DNA molecule is to use concentrated CSF obtained from patients with AD. For this procedure, 30-40 milliliters are concentrated bylyophilization or Amicon filtration or the like, and subjected to two dimensional gel electrophoresis. Proteins are separated in one direction by charge in a pH gradient and then, subjected to molecular sieve chromatography in the other direction by polyacrylamide gel electrophoresis. NTP-immunoreactive proteins are identified as spots by the Th monoclonal antibodies (for example, Th 9) using Western blot anaylsis. The gel is cut and NTP proteins are eluted from the gel. NTP purified in this manner can be sequenced or used to make new monoclonal antibodies.

It will be appreciated that other purification steps may be substituted for the preferred method described above. Those of skill in the art will be able to devise alternate purification schemes without undue experimentation.

### V. Gene Therapy Using Ribozymes

Ribozymes provide method to inhibit mRNA function. Ribozyines may be RNA enzymes, self-splicing RNAs, and self-cleaving RNA (Cech et al. , Journal of Biological Chemistry 267: 17479-17482 (1992)). It is possible to construct *de novo* ribozymes which have an endonuclease activity directed in *trans* to a certain target sequence. Since these ribozymes can act on various sequences, ribozymes can be designed for virtually any RNA substrate. Thus, ribozymes are very flexible tools for inhibiting the expression of specific genes and provide an alternative to antisense constructs.

A ribozyme against chloramphenicol acetyltransferase RNA has been successfully constructed (Haseloff et al., Nature 334:585-591 (1988); Uhlenbeck et al., Nature 328:596-600 (1987)). The ribozyme contains three structural domains: 1) a highly conserved region of nucleotides which flank the cleavage site in the 5' direction; 2) the highly conserved sequences contained in naturally occurring cleavage domains of ribozymes, forming a base-paired stem; and 3) the regions which flank the cleavage site on both sides and ensure the exact arrangement of the ribozyme in relation to the cleavage site and the cohesion of the substrate and enzyme. RNA enzymes constructed according to this model have already proved suitable *in vitro* for the specific cleaving of RNA sequences (Haseloff *et al., supra*).

Alternatively, hairpin ribozymes may be used in which the active site is derived from the minus strand of the satellite RNA of tobacco ring spot virus (Hampel et al., Biochemistry 28:4929-4933 (1989)). Recently, a hairpin ribozyme was designed which cleaves human immunodeficiency virus type I RNA (Ojwang et al., Proc. Natl. Acad. Sci. USA 89:10802-10806 (1992)). Other self cleaving RNA activities are associated with hepatitis delta virus (Kuo et al., J. Virol. 62:4429-4444 (1988)).

Preferred targets for NTP ribozymes are the nucleotide sequences which are not homologous with PTP sequences. Preferably, the NTP ribozyme molecule of the present invention is designed based upon the chloramphenicol acetyltransferase ribozyme or hairpin ribozymes, described above. Alternatively, NTP ribozyme molecules are designed as described by Eckstein et al. (International Publication No. WO 92/07065) who disclose catalytically active ribozyme constructions which have increased stability against chemical and enzymatic degradation, and thus are useful as therapeutic agents.

In an alternative approach, an external guide sequence (EGS) can be constructed for directing the endogenous ribozyme, RNase P, to intracellular NTP mRNA, which is subsequently cleaved by the cellular ribozyme (Altman et al., U.S. Patent No. 5,168,053). Preferably, the NTP EGS comprises a ten to fifteen nucleotide sequence complementary to an NTP mRNA and a 3'-NCCA nucleotide sequence, wherein N is preferably a purine (*Id*.). After NTP EGS molecules are delivered to cells, as described below, the molecules bind to the targeted NTP mRNA species by forming base pairs between the NTP mRNA and the complementary NTP EGS sequences, thus promoting cleavage of NTP mRNA by RNase P at the nucleotide at the 5'-side of the base-paired region (*Id.*)*.*

Alternatively, NTP antisense RNA molecules, NTP ribozymes, and NTP EGS can be coded by DNA constructs which are administered in the form of virions, which are preferably incapable of replicating *in vivo* (*see,* for example, Taylor, WO 92/06693). For example, such DNA constructs may be administered using herpes-based viruses (Gage et al., U.S. Patent No. 5,082,670). Alternatively, NTP antisense RNA sequences, NTP ribozymes, and NTP EGS can be coded by RNA constructs which are administered in the form of virions, such as retroviruses. The preparation of retroviral vectors is well known in the art (see, for example, Brown et al., "Retroviral Vectors," in DNA Cloning: A Practical Approach, Volume 3, IRL Press, Washington, D.C. (1987)).

Specificity for gene expression in the central nervous system can be conferred by using appropriate cell-specific regulatory sequences, such as cellspecific enhancers and promoters. For example, such sequences include the sequences that regulate the oligodendroglial-specific expression of JC virus, glial-specific expression of the proteolipid protein, and the glial fibrillary acidic protein genes (Gage *et al., supra*). Since protein phosphorylation is critical for neuronal regulation (Kennedy, "Second Messengers and Neuronal Function," in An Introduction to MolecularNeurobiology, Hall, Ed., Sinauer Associates, Inc. (1992)), protein kinase promoter sequences can be used to achieve sufficient levels of NTP gene expression.

Thus, gene therapy can be used to alleviate AD by inhibiting the inappropriate expression of the NTP encoded by AD3-4HD1 DNA molecule. Moreover, gene therapy can be used to alleviate AD by providing the appropriate expression level of a particular form of an NTP encoded by AD3-4HD1 DNA molecule. In this case, particular NTP nucleic acid sequences may be coded by DNA or RNA constructs which are administered in the form of viruses, as described above. Alternatively, "donor cells" may be modified *in vitro* using viral or retroviral vectors containing NTP sequences, or using other well known techniques of introducing foreign DNA into cells *(see,* for example, *Sambrook et al., supra*). Such donor cells include fibroblast cells, neuronal cells, glial cells, and connective tissue cells (Gage *et al., supra*). Following genetic manipulation, the donor cells are grafted into the central nervous system and thus, the genetically-modified cells provide the therapeutic form of NTP (*Id.*).

Moreover, such virions may be introduced into the blood stream for delivery to the brain. This is accomplished through the osmotic disruption of the blood brain barrier prior to administration of the virions (*see,* for example, Neuwelt, United States Patent No. 4,866,042). The blood brain barrier may be disrupted by administration of a pharmaceutically effective, nontoxic hypertonic solution, such as mannitol, arabinose, or glycerol (*Id*.).

The following clones in *E. coli* were deposited according to the Budapest Treaty with the American Type Culture Collection (12301 Parklawn Drive, Rockville, Maryland, 20852): G2-2 PstI-DH5 (ATCC No. 69257); G5d-PstI-DH5 (ATCC No. 69258); 1-9a-LX-1 blue (ATCC No. 69259); AD3-4-DH1 (ATCC No. 69260); HB4-XL-blue (ATCC No. 69261); AD10-7-DH1 (ATCC No. 69262); AD2-2-DH1- (ATCC No. 69263); G5d-1PstI-EcoRI-DH5 (ATCC No. 69264); and G2-2PstI-EcoRI-DH5 (ATCC No. 69265).

Having now generally described the invention, the same will be more readily understood through reference to the following Examples which are provided by way of illustration, and are not intended to be limiting of the present invention, unless specified.

### Example 1

### Expression of NTP Immunoreactivity in Cell Lines

Seven cell lines of central nervous system origin were identified that express thread protein immunoreactivity using the Th9 monoclonal antibody which was generated to the pancreatic form of the protein (Gross et al., J. Clin. Invest. 76:2115-2126 (1985)), but cross-reacts with thread proteins present in brain tissue and cerebrospinal fluid (Ozturk et al., Proc. Natl. Acad. Sci. USA 86:419-423 (1989); de la Monte et. al., J. Clin. Invest. 86:1004-1013 (1990); de la Monte et. al., J. Neurol. Sci. 113:152-164 (1992); de la Monte et al., Ann. Neurol. 32:733-742 (1992)). Among them were the following: two primitive neuroectodermal tumor (PNET) cell lines designated PNET1 and PNET2; three glioblastoma cell lines Hg1 16, Hg1 17, and C6; the A172 glial cell line; and the SH-Sy5y neuroblastoma cell line. The glioblastoma cell lines and the A172 cells were obtained from the American Type Culture Collection (ATCC). SH-Sy5y cells were obtained from Dr. Biedler at Sloan-Kettering Memorial Hospital. The PNET cell lines have been described previously (The et al., Nature genetics 3:62-66 (1993)), and were obtained from Dr. Rene' Bernards at the MGH Cancer Center. A11 tell lines were maintained in Earl's Modified Eagle Medium supplemented with 10% fetal calf serum, and without antibiotics.

To examine the cells for thread protein and other immunoreactivities, the cultures were harvested in phosphate buffered saline (PBS) (137 mM NaCl, 2.7 mM KCl, 4.3 mM Na₂HPO₄, 1.4 mM KH₂PO₄, pH 7.3) containing 2 mM EDTA, and cytospin preparations were made using 10⁵ cells per slide. The cytospin preparations were fixed immediately in 100% methanol (-20°C), air-dried, and then stored at -80°C until used. Prior to immunostaining, the slides were equilibrated to room temperature and hydrated in PBS. Nonspecific antibody binding was blocked with 3% nonimmune horse serum. Replicate cytospin preparations from the same cultures were incubated overnight at 4°C with 5 or 10 µg/ml of primary antibody. Immunoreactivity was revealed by the avidin-biotin horseradish peroxidase method using the Vectastain Elite kit (Vector Laboratories, Burlingame, CA) according to the manufacturer's protocol, and with 3-3' diaminobenzidine (0.5 mg/ml plus 0.03% hydrogen peroxide) as the chromogen. The cells then were counterstained with hematoxylin, dehydrated in graded alcohol solutions, cleared in xylenes, and preserved under coverglass with Permount (Fisher Scientific).

Cytospin preparations of each cell line were immunostained with the thread protein monoclonal antibodies Th9, Th7, Th10, Th29, Th34, TH46, Th67, and Th90. In addition, replicate slides were immunostained with positive (neurofilament, glial fibrillary acidic protein (GFAP), and vimentin) and negative (desmin, Hepatitis B surface antigen-5C3) control monoclonal antibodies. Except for 5C3 which was generated in the inventor's laboratory (Fujita et al., Gastroenterology 91:1357-1363 (1986)), the control antibodies were purchased (Boehringer-Mannheim). All serological reagents were diluted in PBS containing I % bovine serum albumin (BSA), and all incubations except the one with primary antibody were carried out at room temperature in humidified chambers. The slides were washed in 3 changes of PBS between each step.

Both PNET1 and PNET2 cells expressed high and middle molecular weight neurofilament proteins and little or no glial fibrillary acidic protein or vimentin. The PNET1, PNET2, and SH-Sy5y cells expressed GAP-43, an abundant calmodulin-binding phosphoprotein that is highly expressed in immature neurons and in neurons undergoing regenerative cell growth (Benowitz et al., J. Neurosci. 3:2153-2163 (1983); DeGraan et al., Neurosci. Lett. 61:235-241 (1985); Kalil et al., J. Neurosci. 6:2563-2570 (1986)). The A172 and C6 cells expressed GFAP and vimentin. However, A172 also exhibited neurofilament immunoreactivity, raising doubt about its purely glial cell nature. None of the cell lines manifested immunoreactivity with monoclonal antibodies to desmin or to Hepatitis B surface antigen. As a negative control cell line, the Huh7 hepatocellular carcinoma cell line was similarly immunostained, and found not to exhibit any immunoreactivity with the above antibodies. However, the Huh cells were immunoreactive with monoclonal antibodies to the insulin receptor substrate protein, IRS-1 (data not shown) which was used as a positive control for this cell line (Sasaki et al., J. Biol. Chem. 268:1-4 (1993)).

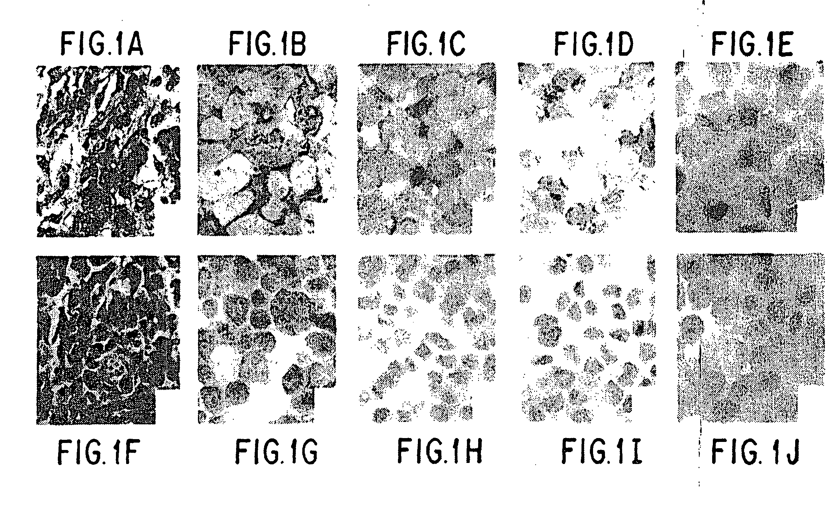

Using the Th9 monoclonal antibody, thread protein immunoreactivity was detected in primary PNET (A), primary glioblastoma (F), PNET1 (B), and C6 cells (G), but not in hepatocellular carcinoma cell lines (Figures 1A-1J). In addition, Th9 immunoreactivity was detected in histological sections from 8 of the 9 primary human CNS PNETs, and from all 5 of the primary human glioblastomas studied (Figures 1A-1J). Although all 5 cell lines exhibited intense immunoreactivity with the Th9 monoclonal antibody, they differed with respect to immunoreactivity for other Th monoclonal antibodies. The immunostaining reaction generated with the Th10 (C,H), Th7 (D,1), or Th46 monoclonal antibodies was either low-level (C,D) or absent (H,I,E,J) in PNET1 (C-E) and C6 (H-J). PNET2 cells exhibited only low levels of immunoreactivity with Th7 and Th29, and they manifested no immunostaining with the other Th monoclonal antibodies. A172, C6, and SH-Sy5y cells displayed little or no immunoreactivity with Th monoclonal antibodies other than Th9. Huh7 cells exhibited no immunoreactivity with any of the thread protein monoclonal antibodies employed, whereas human pancreatic tissue was immunoreactive with all of the Th antibodies, which had been generated against the purified pancreatic form of thread protein (Gross et al., J. Clin. Invest. 76:2115-2126 (1985)).

### Example 2

### Analysis of Thread Proteins by Monoclonal Antibody-Based Immunoradiometric Assay (M-IRMA)

Cultured cells were washed in PBS and recovered in PBS containing 2 mM EDTA. The cells were pelleted by centrifugation at 1000 x g for 15 min, and then resuspended in lysis buffer containing 50 mM Tris-HCl (pH 7.5), 1% Triton X-100, 2 mM EGTA. 10 mM EDTA, 100 mM NaF, I mM Na₄P₂O₇, 2 mM Na₃VO₄, 100 µg/ml phenylmethylsulfonyl fluoride, 1µg/ml aprotinin, 1 µg/ml pepstatin A, and I µg/ml leupeptin. The supernatant fractions obtained by centrifugation of the lysates at 14,000 x g for 10 min were used for the Western blot analysis, immunoprecipitation studies, and M-IRMA. Protein concentration was determined by the Lowry colorimetric assay. The samples were stored at -40°C.

M-IRMA is a highly sensitive two- or three-site forward sandwich assay which permits quantitation of picomolar NTP in cell lysates, tissue culture medium, tissue homogenates, and body fluids (Ozturk et al., Proc. Natl. Acad. Sci. USA 86:419-423 (1989): de la Monte et. al., J. Clin. Invest. 86:1004-1013 (1990): de la Monte et. al., J. Neurol. Sci. 113:152-164 (1992): de la Monte et al., Ann. Neurol. 32:733-742 (1992); Gross et al., J. Clin. Invest. 76:2115-2126 (1985)). In addition, when combined with SDS-PAGE, M-IRMA can be used to determine molecular size of thread proteins and related species (Ozturk et al., Proc. Natl. Acad. Sci. USA 86:419-423 (1989); de la Monte et. al., J. Clin. Invest. 86:1004-1013 (1990); de la Monte et. al., J. Neurol. Sci. 113:152-164 (1992); de la Monte et al., Ann. Neurol. 32:733-742 (1992)). M-IRMA involves capturing the immunoreactive thread proteins present in biological samples using monoclonal antibodies Th7 and Th10 affixed to a solid-phase matrix, and then detecting the captured antigen with a third radiolabeled tracer monoclonal antibody (Th9) to the same protein. Briefly, 1/4" polystyrene beads (Precision Ball, Inc) were coated with one or two monoclonal antibodies to thread proteins (usually Th7 + TH10). Cell lysates or supernatant fractions of tissue homogenates (Ozturk et al., Proc. Natl. Acad. Sci. USA 86:419-423 (1989); de la Monte et. al., J. Clin. Invest. 86:1004-1013 (1990); de la Monte et. al., J. Neurol. Sci. 113:152-164 (1992); de la Monte et al., Ann. Neurol. 32:733-742 (1992)) were incubated over night with the coated beads to capture thread proteins present in the samples. The beads were washed 5x in PBS, and then incubated with ¹²⁵-I labeled Th9 as a tracer to detect the captured thread proteins. The concentration of thread protein in the lysate or tissue homogenate was determined from a standard curve generated with known quantities of purified thread protein. This highly sensitive assay can detect as little as 10 pmol of thread protein in solution. To assay for thread proteins fractionated by SDS-PAGE, the wet gels were sliced at 2 mm intervals, and the proteins were eluted from each fraction into 0.5 ml of PBS by shaking for 24 hours at room temperature. The eluates were assayed directly for thread proteins by M-1RMA.

Corresponding with the widespread immunocytochemical staining of PNET1 cells with Th7, Th10, Th34, and Th29, thread protein immunoreactivity was readily measured in these cells by M-IRMA. In other words, with Th7, Th10, Th34, and Th29 monoclonal antibodies (MoAb) used as capture antibodies, either singularly or with two of them together, and ¹²⁵-I labeled Th9 was used as the tracer, similarly high levels of thread protein were measured (Figure 2). In contrast, in PNET2, C6, and A172 cells, which exhibited intense immunoreactivity with Th9, but little or no immunocytochemical staining with the Th monoclonal antibodies that were used to capture antigen, the levels of thread protein detected by M-IRMA were much lower than those measured in the PNET1 cells (Figure 2). Similarly, Huh7 cells, which manifested no immunocytochemical staining with any of the thread protein monoclonal antibodies, had virtually nondetectable levels of thread proteins in the cellular lysates by M-IRMA. The concentrations of thread protein in the cell lysates were computed from a standard curve generated with purified PTP using Th7 and Th10 as capture antibodies. The results expressed as mean S.D. pg/mg of total protein were as follows: PNET1-13.1 ± 0.39; PNET2-2.06 ± 0.10; A172-3.38 ± 0.37; C6-2.52 ± 0.22; and Huh7-0.34 ± 0.05.

### Example 3

### Characterization of Neural Thread Proteins in Tumor Cell Lines

In Western Blot anaylsis, samples containing 100 µg of protein were fractionated by SDS-PAGE, along with pre-labeled molecular weight standards. The proteins were blotted onto nylon membranes (lmmobilon-P transfer membrane, Millipore) using a semi-dry transfer apparatus (Integrated Systems). The membranes were washed in Tris buffered saline (TBS; 10 mM Tris, 0.85% sodium chloride, pH 7.5), and then blocked with TBS containing 3% BSA. The blots were incubated overnight at 4°C with ¹²⁵-I labeled Th9 monoclonal antibody. Unspecifically bound probe was removed by washing the membranes at room temperature in TBS-BSA 3 x 15 min, and 1 x 30 min. The results were analyzed by autoradiography using Kodak XAR film.

To prepare samples for immunoprecipitation studies, one milliliter samples of cell lysate containing approximately 1 mg/ml of protein were used for immunoprecipitation studies. The lysates were initially pre-cleared with non-relevant antibody (5C3 or antidesmin), and then with Protein A sepharose. Thread proteins were immunoprecipitated using 5-10 µg of Th9 and Protein A sepharose (Sasaki et al., J. Biol. Chem. 268:1-4 (1993)). The immune complexes collected by centrifugation were resuspended in buffer containing 2 % SDS and 10 mM β-mercaptoethanol, and then subjected to SDS-PAGE under denaturing and reducing conditions (*Id*.). Crude cellular lysates (100 µg protein) were analyzed simultaneously. The proteins were blotted onto Immobilon-P membranes and probed with ¹²¹-I labeled (*Id*.) Th9 to detect thread proteins and related molecules. Negative control experiments were performed simultaneously using either monoclonal antibodies to Hepatitis B surface antigen (5C3) or to desmin.

Metabolic labeling experiments were performed using monolayers of cells cultured in 100 mm² petri dishes. Prior to labeling, the cells were exposed to methionine- and cysteine-free medium for 2 h. The medium was then replaced with 3 ml of DMEM containing 300 µCi each of [³⁵S] methionine or [³⁵S] cysteine. After labeling for 3 hours, the cells were incubated for various intervals with complete medium devoid radiolabeled amino acids and supplemented with 10 mM methionine. Cell lysates were prepared as described above. Thread proteins were immunoprecipitated using the Th9 monoclonal antibody and protein A sepharose, and the immunoprecipitation products were analyzed by SDS-PAGE and film autoradiography.

For the *in vivo* phosphorylation studies, cells cultured as described for metabolic labeling studies were washed twice with TBS and incubated for 2 h with phosphate-free Dulbecco's MEM containing 10% dialyzed fetal calf serum. Then the cells were washed with TBS and incubated for 3 h with the same medium containing 400 µCi/ml of [³²P] orthophosphoric acid. The cell lysates were analyzed by immunoprecipitation with thread protein, and both positive (p36) and negative (desmin) control monoclonal antibodies, followed by SDS-PAGE.

In order to study the glycosylation state of neural thread proteins, cell culture lysates containing approximately 100 *µg* or protein were subjected to SDS-PAGE, and the fractionated proteins were transferred to Immobilon-P membranes (Millipore). O- and N-glycans were detected by periodate oxidation followed by biotinylation, and then Western blot analysis with a Streptavidin-alkaline phosphatase probe and NBT/BCIP as the colorimetric substrate. The assays were performed using the GlycoTrack Kit (Oxford Glycosystems, Rosedale, NY) according to the protocol provided by the manufacturer.

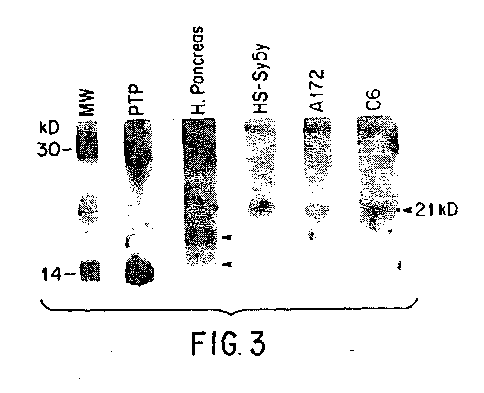

Th9-immunoreactive proteins were detected in lysates of PNET1, PNET2, SH-Sy5y, C6, and A 172 cells by four different methods: Western blot analysis, immunoprecipitation followed by Western blot analysis, metabolic labeling followed by immunoprecipitation, and SDS-PAGE combined with M-IRMA. Western blot analysis of crude cellular lysates using ¹²⁵I-labeled Th9 demonstrated ∼ 21 kDa bands in the above cell lines (as indicated by the arrow in Figure 3), but the signal intensity was low. In contrast, in lysates of human pancreatic tissue, the expected 17 kDa uncleaved and 14 kDa cleaved forms of pancreatic thread protein were readily detected by Western blot analysis (Figure 3). Thread proteins were not detected in lysates of human hepatocellular carcinoma cell lines. The strikingly greater abundance of thread proteins in pancreatic tissue compared with neuronal and glial cell lines is consistent with a previous finding of 10⁶-fold higher levels of thread proteins in pancreas and pancreatic juice compared with brain tissue and cerebrospinal fluid (Ozturk et al., Proc. Natl. Acad. Sci. USA 86:419-423 (1989); de la Monte et. al., J. Clin. Invest. 86:1004-1013 (1990); de la Monte et. al., J. Neurol. Sci. 113:152-164 (1992); de la Monte et al., Ann. Neurol. 32:733-742 (1992)). Although one would expect that thread proteins synthesized by PNET and glial cells are secreted as is the case for PTP and NTP, thread proteins were not detected in the tissue culture medium by Western blot analysis, even after concentrating the medium four- or five-fold by lyophilization.

Th9-immunoreactive thread proteins were more readily detected in PNET and glial cell lines by first immunoprecipitating from the lysates with either Th7 + Th10 or Th9, and then performing Western blot analysis using ¹²⁵I-labeled Th9 (direct) (Figure 3), or unlabeled Th9 with ¹²⁵I-labeled Protein A (indirect). Both methods demonstrated 21 kDa thread protein-related species, similar to those detected by Western blot analysis. In addition, ~17 kDa bands were also observed in both PNET and glial cells, but the signal was inconsistent and low-level, as determined by Western blot analysis. As negative controls, the Huh7, HepG2, and FOCUS (Lun et al., In Vitro (Rockville) 20:493-504 (1984)) human hepatocellular carcinoma cell lines were studied simultaneously under identical conditions, and Th9-immunoreactive proteins were not detected in the cellular lysates.

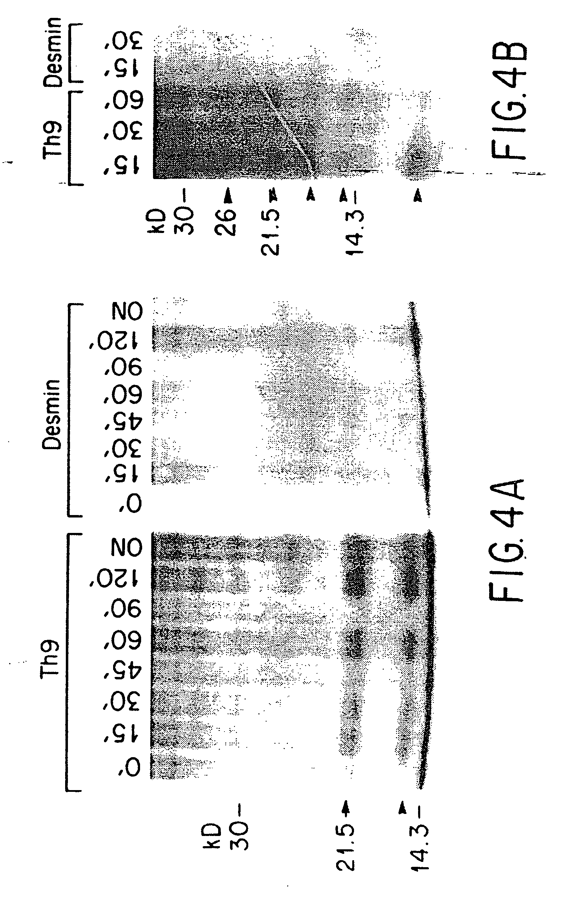

The molecular sizes of thread proteins present in PNET and glial cells were most prominently demonstrated by metabolical labeling with ³⁵S-methionine or ³⁵S-cysteine, followed by immunoprecipitation using Th9 monoclonal antibody. Monoclonal antibodies to desmin or to hepatitis B surface antigen (5C3) were used as negative controls for immunoprecipitation. In both PNET and glial cell lines, ~26 and ~21kDa Th9-immunoreactive proteins were detected by SDS-PAGE analysis of the immunoprecipitated products (Figure 4B). In PNET1 cells, the 21 kDa band appeared as a doublet (Figure 4A); the accompanying slightly higher molecular weight species appeared to be less abundant than the dominant band at ∼21 kDa. In addition, in both PNET and glial cell lines, there were also ∼17 kDa Th9-immunoreactive proteins associated with bands of nearly the same intensity as the ∼21 kDa bands. In C6 cells, there were also ∼26 kDa, ∼14-15 kDa and ∼8 kDa Th9-immunoreactive proteins which were not detected in PNET cells (Figures 4A and 4B, arrows).

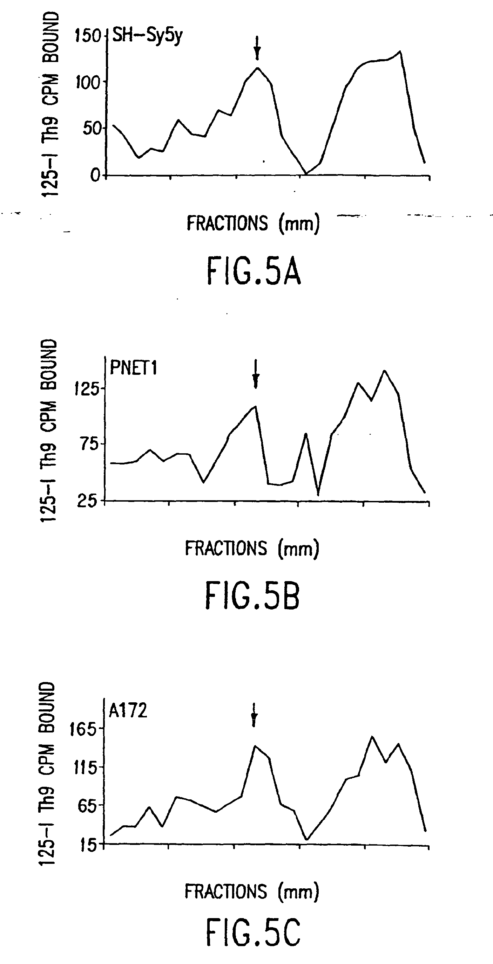

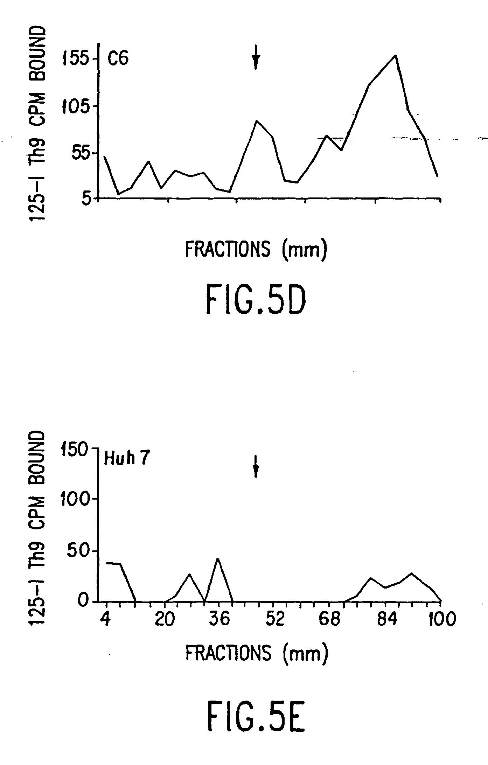

The 21 kDa and 17 kDa thread proteins in SH-Sy5y, PNET1, A172 and C6 cells, and their absence in hepatocellular carcinoma cells were also demonstrated by SDS-PAGE/M-IRMA (Figures 5A-5E). Cellular proteins fractionated by SDS-PAGE were eluted from the gels sliced at 2 mm intervals, and assayed directly for thread protein immunoreactivity by M-IRMA using Th7+Th10 as capture antibodies, and ¹²⁵I-labeled Th9 as the tracer. Despite low levels, two distinct peaks were evident in all neuroectodermal cell lines, but not in Huh7 hepatocellular carcinoma cells assayed simultaneously and in the same manner. The resolution of these gels did not permit distinction of ∼17 kDa from ∼14-15 kDa proteins which might have been preset.

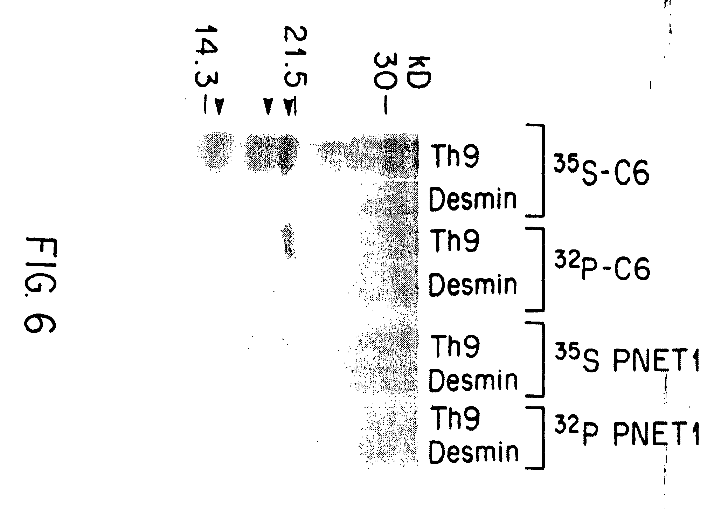

PNET1 and C6 cells were metabolically labeled with ³²P or ³⁵S-methionine, and thread proteins were immunoprecipitated from the lysates using Th9 monoclonal antibody (Figure 6). As a negative control, immunoprecipitation studies were conducted using an equal portion of the cellular lysate and monoclonal antibodies to desmin protein (Figure 6, right panel). In the cells labeled with ³⁵S methionine, Th9-immunoreactive bands were detected at ~26 kDa and ~21 kDa (upper arrows), ~17 kDa (lower arrows), and also at ∼ 14-15 kDa (Figure 6). After ³²P labeling, only the 21 kDa band was observed by immunoprecipitation with Th9 monoclonal antibody; the other molecular weight species did hot appear to be phosphorylated (Figure 6). Phosphorylated Th9-immunoreactive proteins were detected in C6 cells, but not in PNET1 cells, but this might be due to less efficient labeling since PNET1 cells grow slower than C6 cells. No bands in the 14 kDa to 26 kDa range were detected using monoclonal antibodies to desmin for immunoprecipitation (Figure 6). Carbohydrate moieties were not detected in Th9 immunoprecipitated proteins (data not shown).

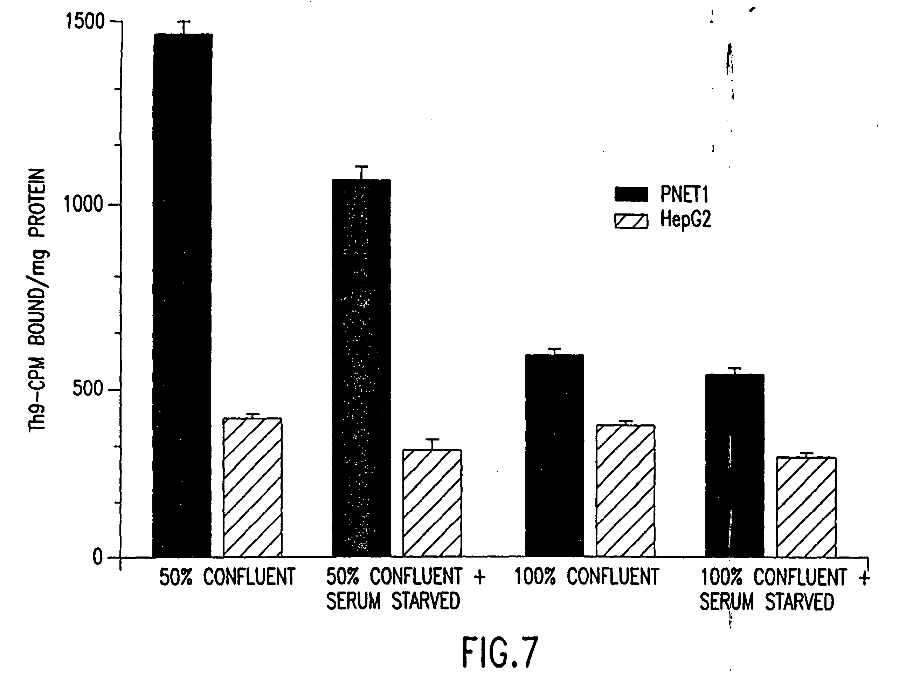

The highest concentrations of thread protein were measured in subconfluent cultures of PNET1 cells, i.e. during the log phase of growth, and the lowest concentrations in overnight serum-starved cultures (growth arrest) (Figure 7). Cultures that were 100% confluent also had lower levels of thread protein expression compared with proliferating cultures. Huh7 hepatocellular carcinoma cells (negative control were simultaneously studied using identical culture conditions, but the levels of thread protein remained low throughout.

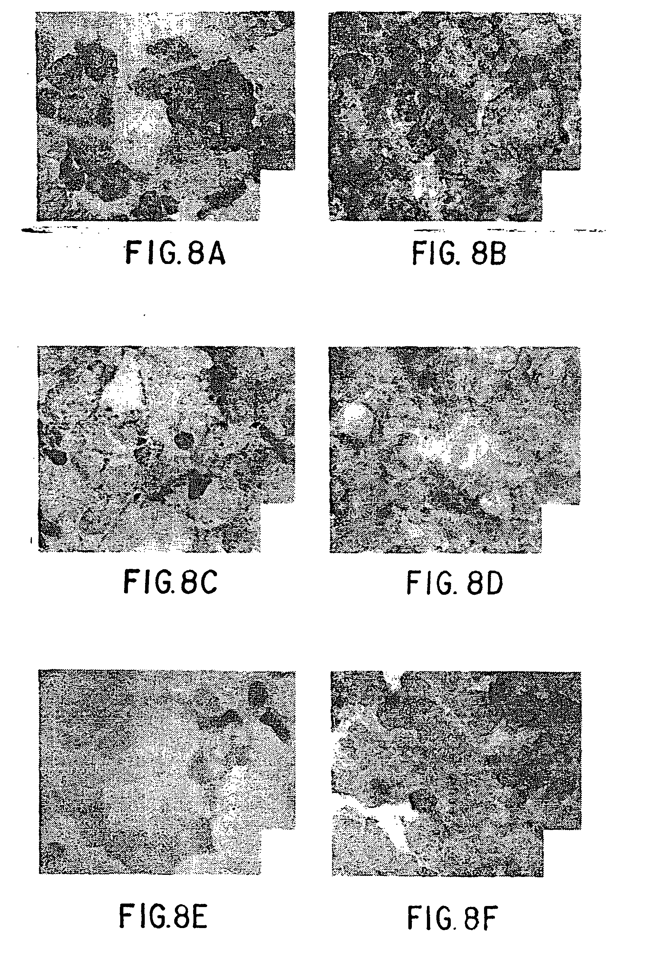

Surprisingly, there was no change in the degree of thread protein immunocytochemical staining of PNET cells cultured under these various conditions. However, the degree to which the levels of thread proteins changed by M-1RMA measurement may not have been detectable by immunocytochemistry. Nevertheless, the reduction in cellular thread protein content induced by serum starvation was associated with a change in the phenotype of the cells. When the cells achieved 100% confluence or after they had been subjected to overnight serum starvation, the cell bodies reduced in size, and they exhibited striking changes in the degree and distribution of immunoreactivity for neurofilament protein, GAP-43, and GFAP (Figure 8). In PNET culture that were 50% confluent, the cells exhibited punctate and often a polar distribution of neurofilament and GAP-43 immunoreactivity, whereas 100% confluent and serum-starved PNET cultures exhibited diffuse perikaryal immunoreactivity for both neurofilament and GAP-43. The punctate immunoreactivity may have corresponded with distribution of neurofilament and GAP-43 in neurites. In contrast, 50% confluent PNET cultures were devoid of GFAP immunoreactivity, while 100% confluent and serum-starved cultures contained conspicuous proportions of GFAP-positive cells. Moreover, the proportion of GFAP-immunoreactive cells was greatest in 100% confluent serum-starved cultures, followed by 50% confluent serum-starved cultures, and then 100% confluent cultures with medium containing 10% fetal calf serum. Therefore, the reduction in thread protein levels measured in PNET cells subjected to overnight serum starvation may have been due to differentiation of the cells toward an astrocytic phenotype. C6 cells and other glioblastoma cell lines exhibited intense immunoreactivity with the Th9 monoclonal antibody, but the levels of thread protein measured by M-IRMA were often low, possibly due to low-level immunoreactivity with others thread protein antibodies, including Th7 and TH10 (*see* Figures 1A-1J).

### Example 4

### Cloning of Thread Proteins from Human cDNA Libraries

Human brain DNA libraries made from 17-18 week old fetal brain (Stratagene, Inc., La Jolla, CA), 2 year-old temporal lobe neocortex (Stratagene), and end-stage Alzheimer's disease cerebral cortex (In Vitrogen, San Diego, CA) were screened using probes generated from a 4161 bp DNA fragment corresponding to nucleotides 235-650 of the rat PTP cDNA. The rat PTP cDNA, designated O18, was isolated from a rat pancreatic cDNA library using synthetic 60mer DNA probes corresponding to nucleotides 45-104 and 345-404 of the published sequence (Terazono et al., J. Biol. Chem. 263:2111-2114 (1988); Watanabe et al., J. Biol. Chem. 265:7432-7439 (1990)). Approximately 2 x 10⁶ plaques or colonies from each library were screened with low-stringency hybridization using standard techniques (*see* Sambrook *et al., supra*). Putative clones were plaque/colony purified, and the RNA inserts were sequenced by the dideoxynucleotide chain termination method using T7 polymerase (US Sequenase; United States Biochemical Corp., Cleveland, OH). The sequences were compared with the Genebank database, and aligned with the nucleic acid sequences of other thread protein cDNAs.

### 1. CNS Neural Thread Protein cDNA Isolated from Human Fetal Brain Library

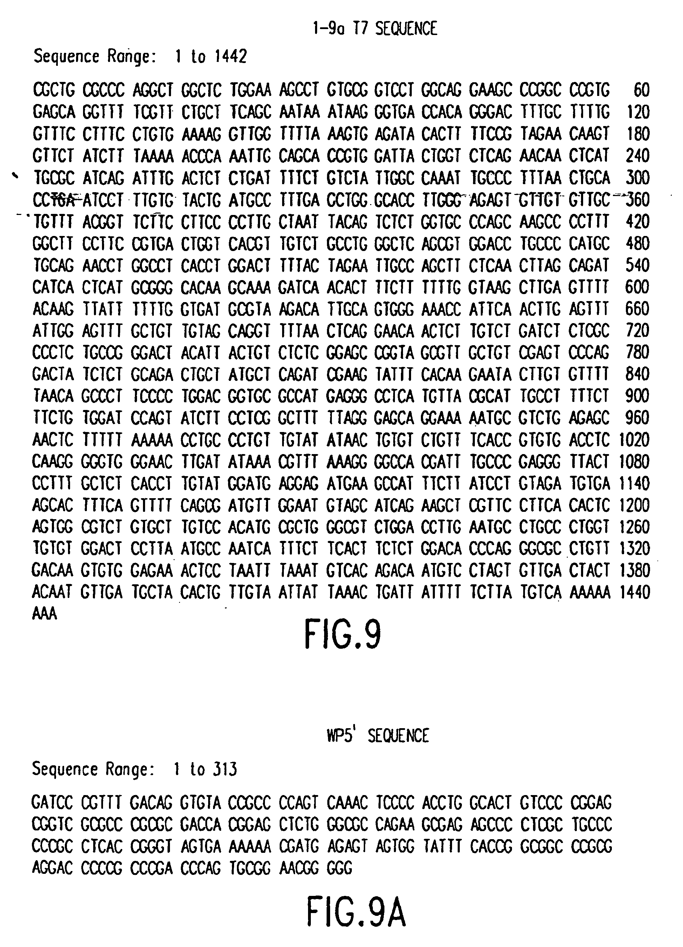

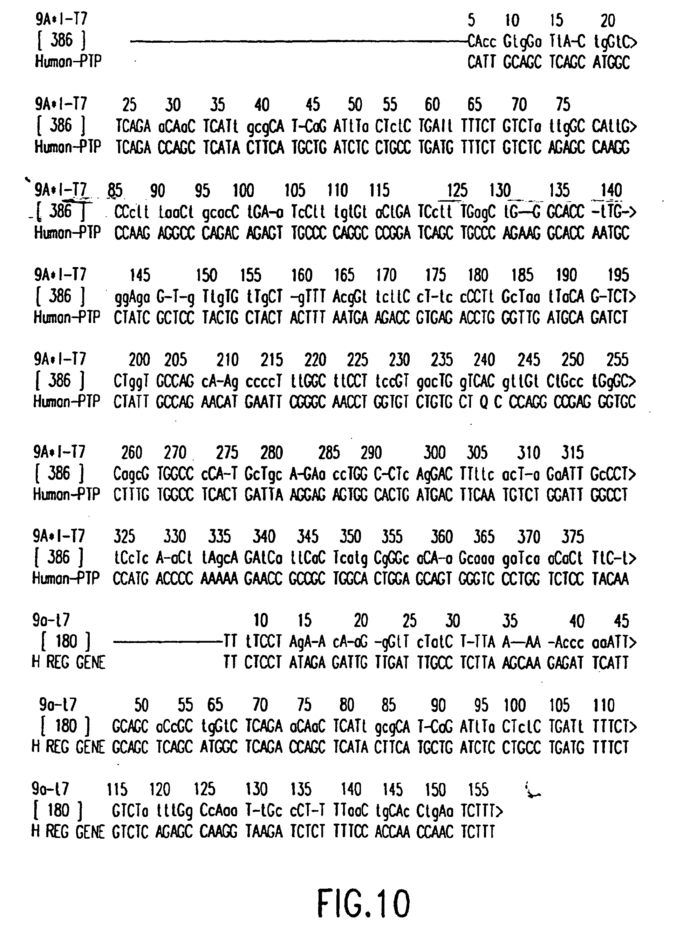

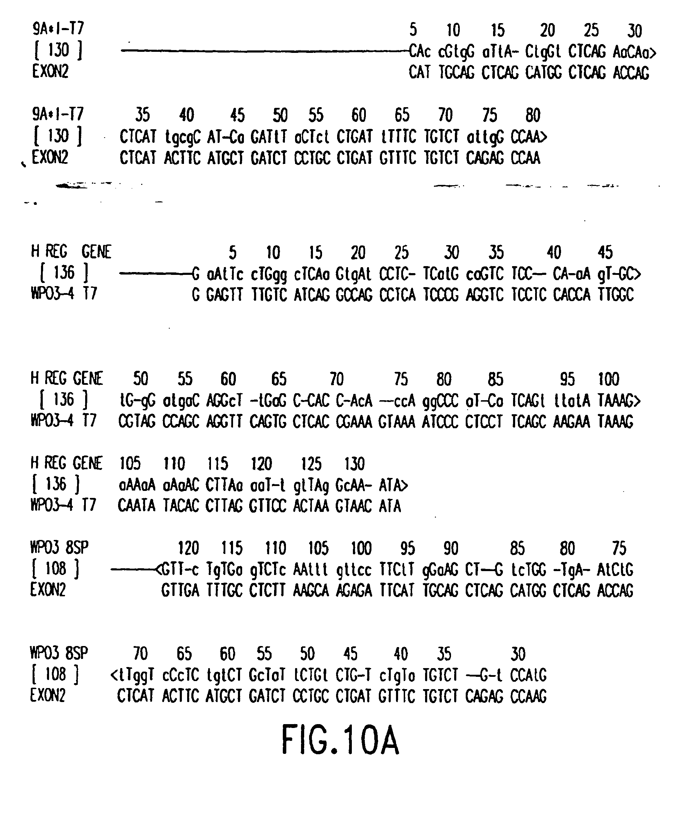

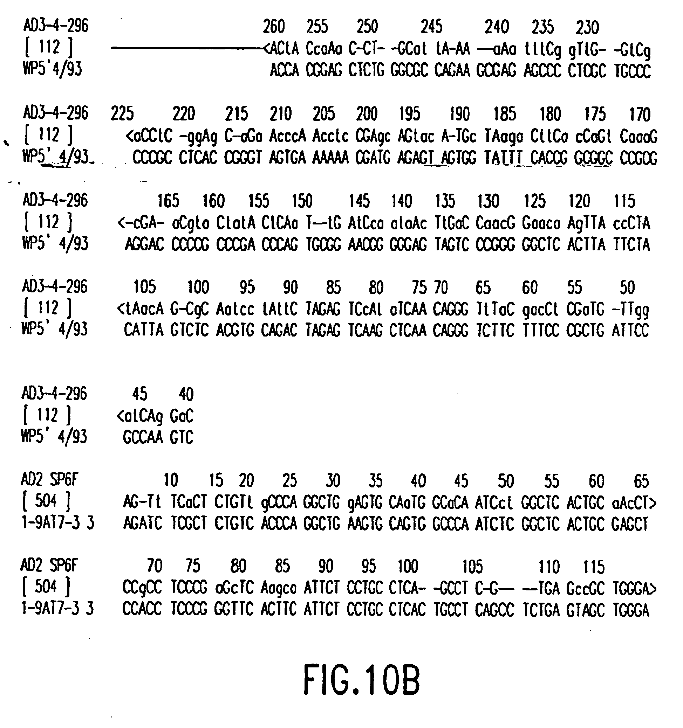

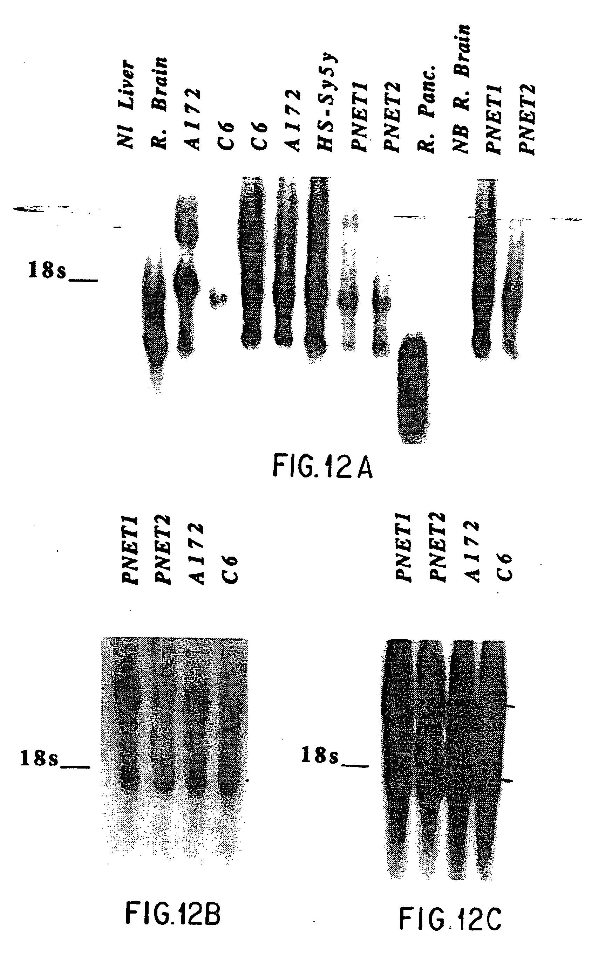

A 1.35 kilobase (kb) 1-9a CNS thread protein partial cDNA was isolated in which only a small segment corresponds to an open reading frame, and the remainder, to a 3' untranslated region (Figure 9A). The sequence of an additional 150 nucleotides was obtained from 5' anchor PCR amplification products. A second round of 5' anchor PCR amplification yielded a further upstream 600 bp product (Figure 9b). A portion of the 1-9a cDNA sequence shares significant homology with the 5' end of the human PTP cDNA and the Reg gene (Figure 10A). In addition, the initial 5' anchor PCR product has 60% homology with the 5' end of the Reg gene, and 63% homology with Exon 2 of the human Reg gene (Figure 10B). Moreover, probes generated from the 590 bp 5'-end fragment of 1-9a cDNA hybridized with human brain and pancreas mRNA (Figures 12A-12C). The 1-9a sequence is also homologous with the AD2-2 and AD3-4 cDNAs in that at one end of their completed sequences, the overlaps are substantial (Figure 10B).

### 2. CNS Neural Thread Protein cDNA Isolated from a Two-Year Old Temporal Cortex Library

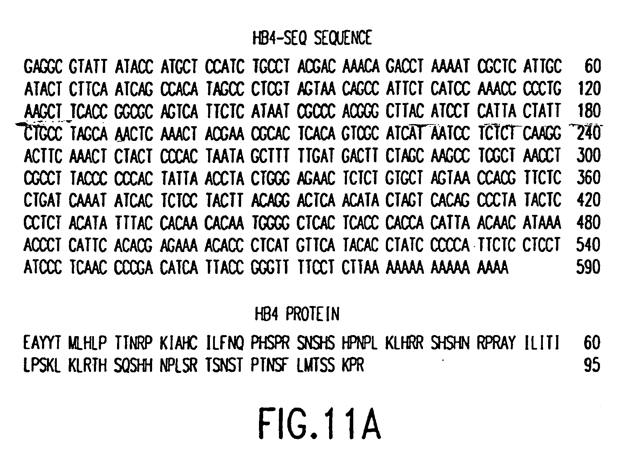

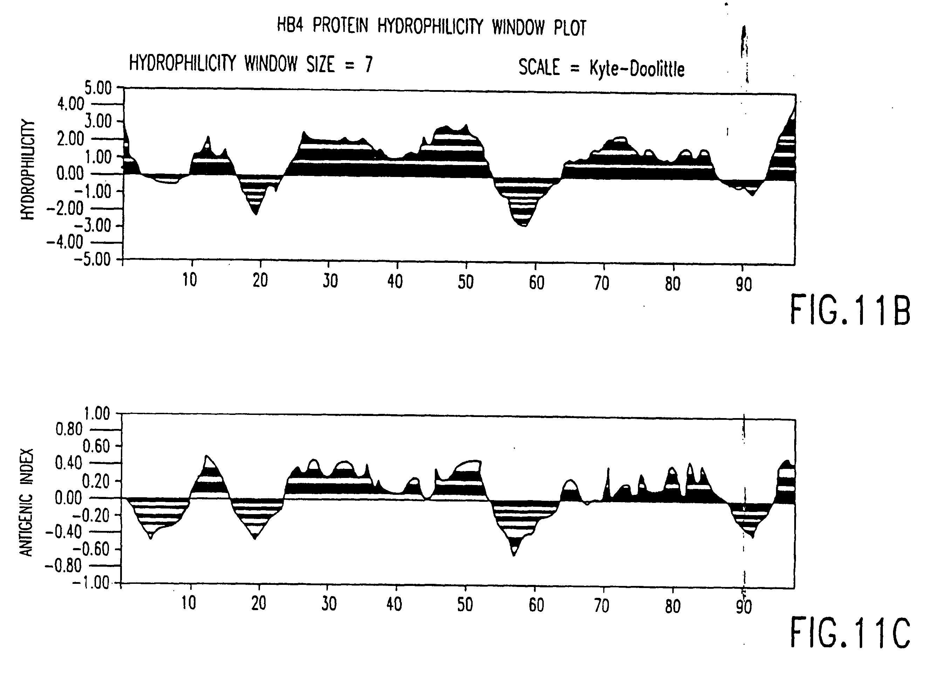

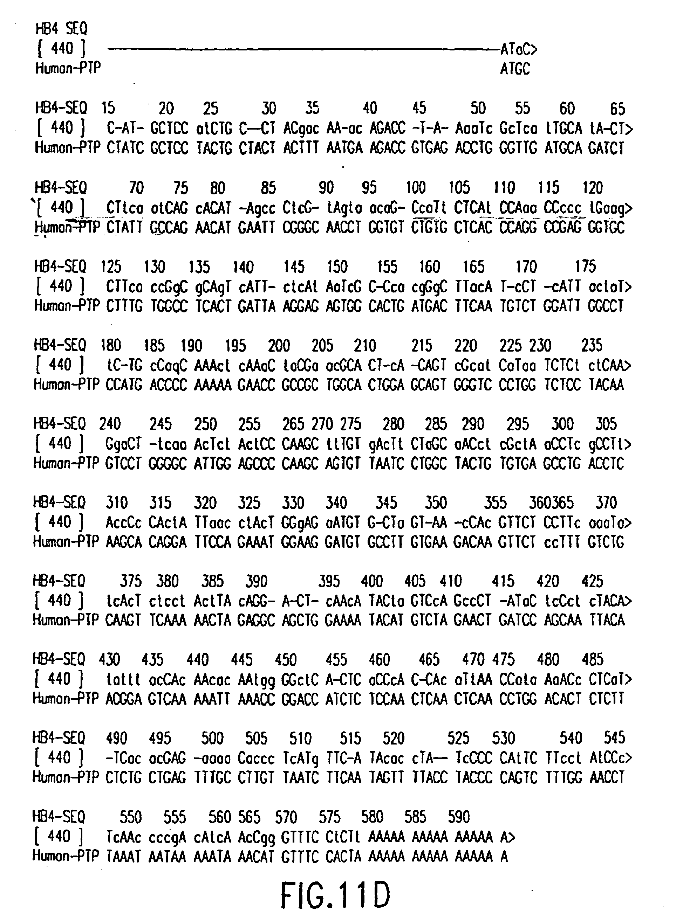

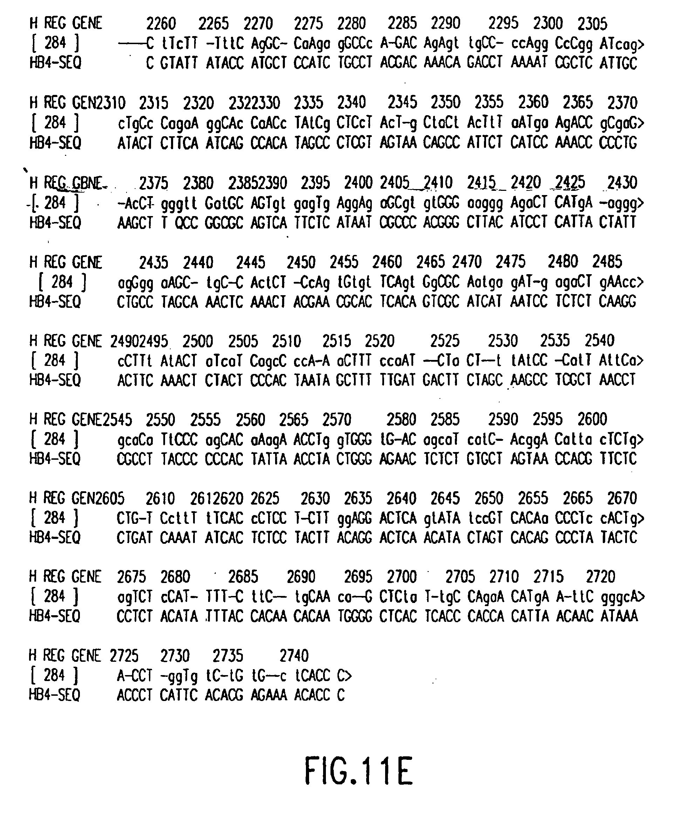

The HB4 clone is a 593 base pair partial cDNA that was isolated from a 2-year old temporal cortex library. This cDNA contains an open reading frame at its 5' end and terminates at nucleotide 275. There is a polyadenylation signal beginning at nucleotide 475, and the sequence ends with a poly-A tail (Figure 11D). The deduced amino acid sequence of the partial HB4 clone predicts a protein with a molecular weight of 10.4 kDa, and a pI of 12.1. The HB4 cDNA exhibits 50% overall nucleic acid homology with the human PTP cDNA (Figure 11E), a segment of the human Reg gene (Figure 11E).

### 3. Isolation of Neural Thread Protein cDNAs from an Alzheimer's Disease Library

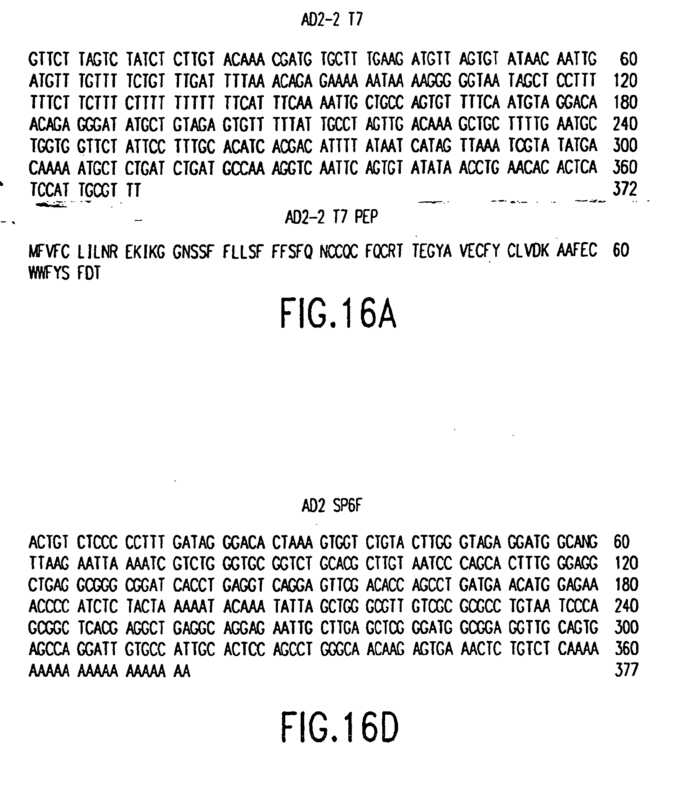

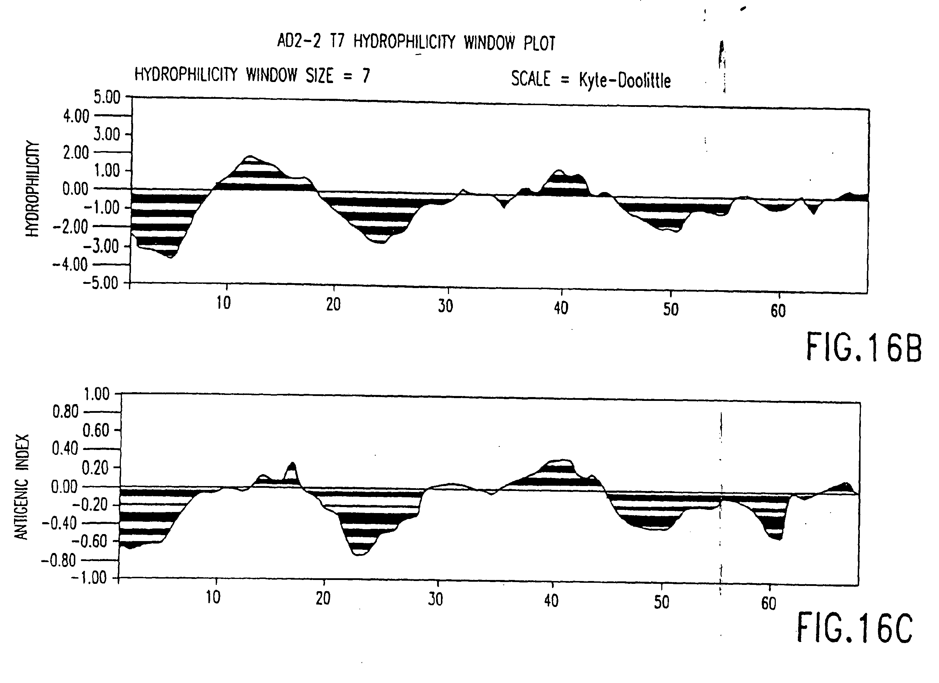

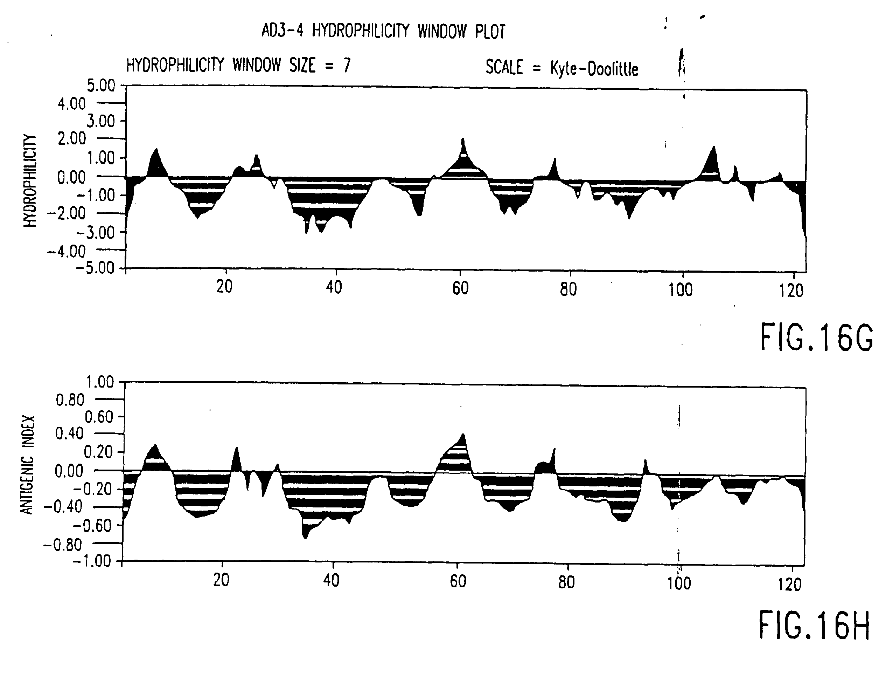

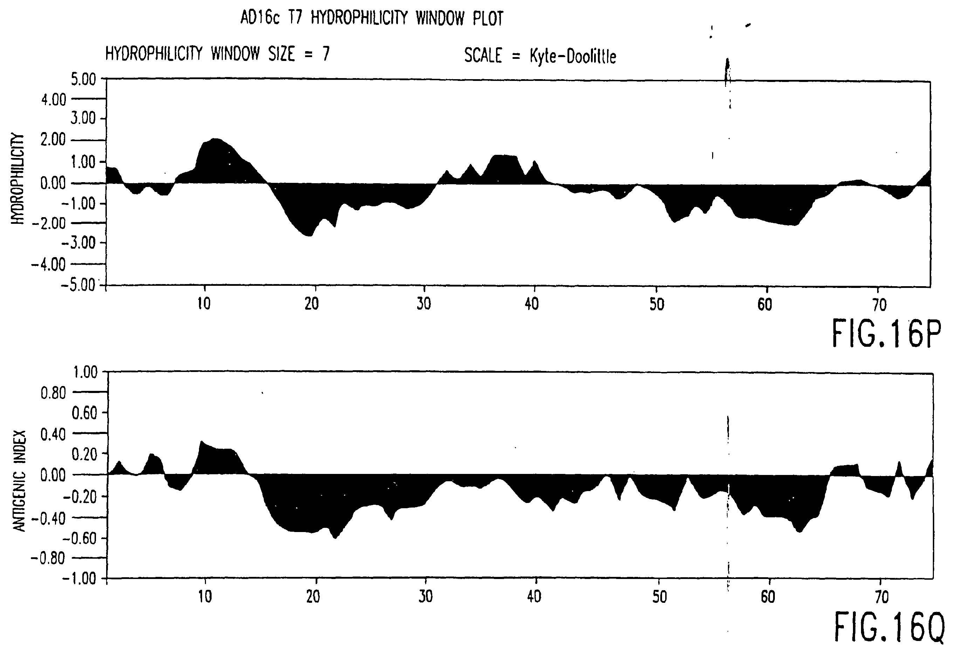

Using the 018 rat PTP cDNA probe, four related cDNAs were isolated from an AD brain library. These clones were designated: AD 2-2, AD 3-4, AD 4-4 and AD 16c (also called AD 10-7) (Figures 16D-16S).

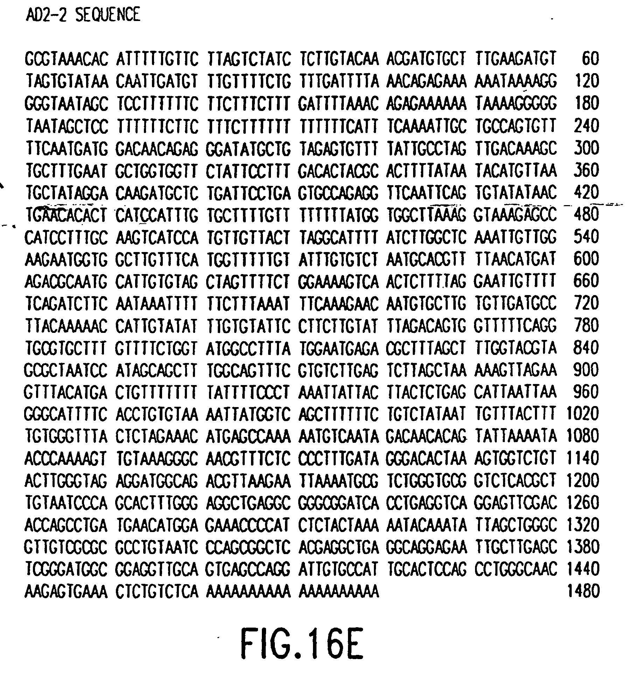

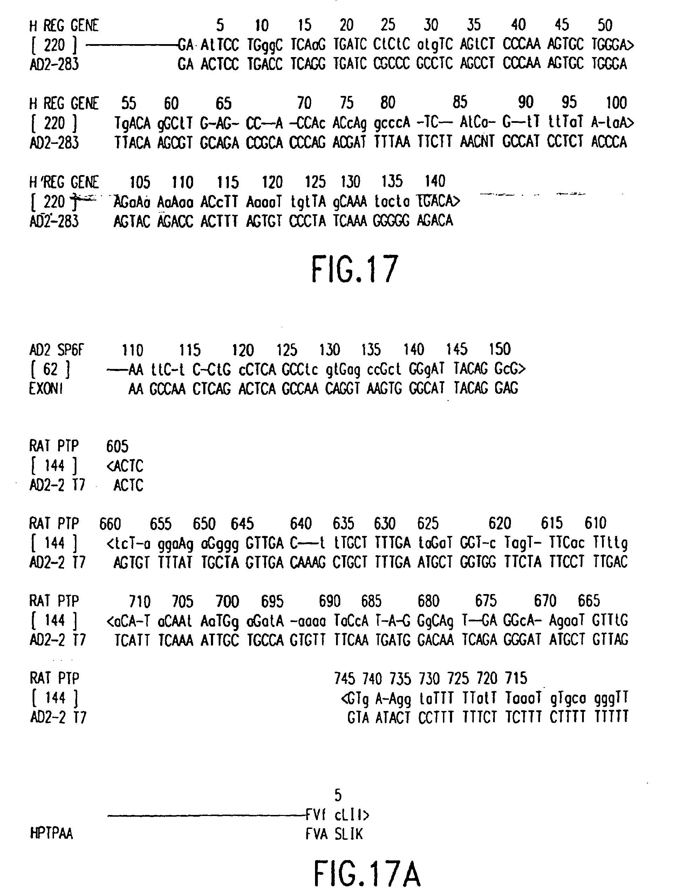

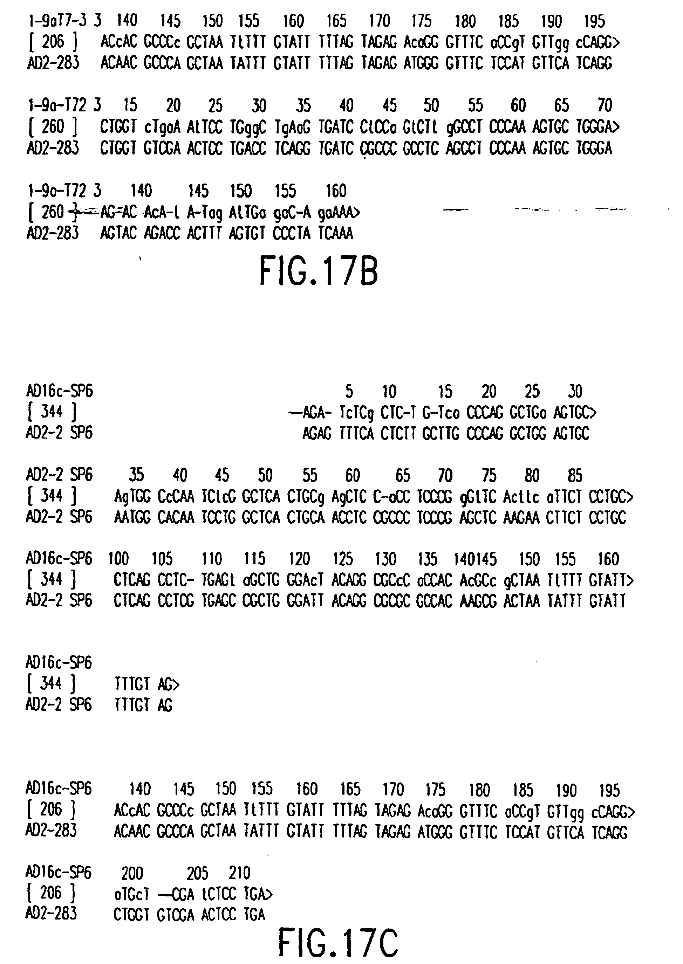

The AD 2-2 cDNA is approximately 1.2 kb and it shares significant homology with the 1-9a cDNA, AD 16c, rat PTP cDNA, and Exon 1 of the human Reg gene (Figure 17). The AD 2-2 probe generate a genomic Southern blot pattern similar to that obtained witch the AD 3-4 probe. Figure 16E depicts the complete nucleotide sequence of the AD2-2 DNA clone that was isolated from an AD brain library. Random primer generated probes based on this sequence hybridized with human brain and neuronal samples but not with glial cell lines of with pancreatic RNA.

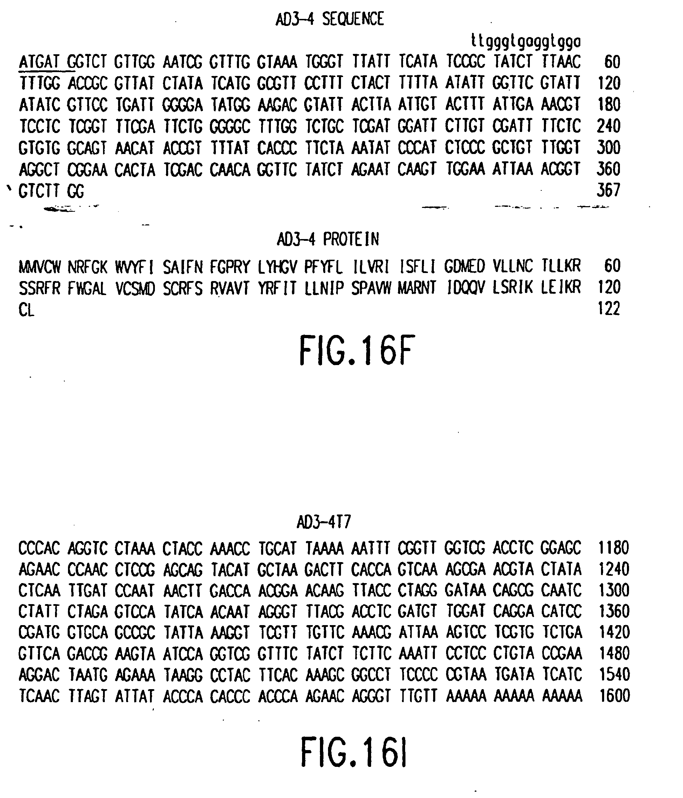

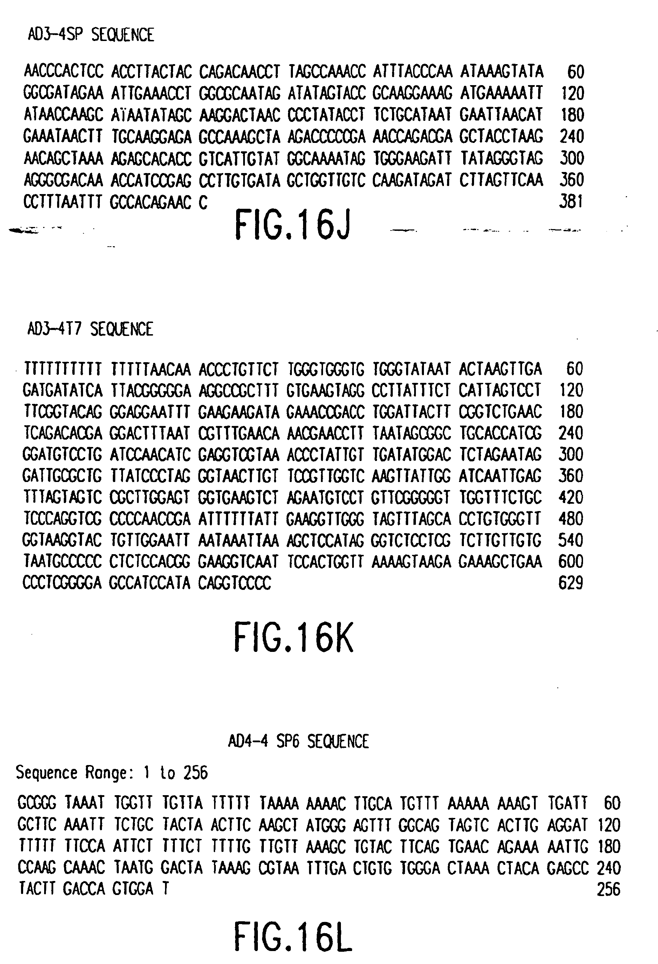

Figures 16F,16I, 16J and 16K depict partial nucleotide sequences of the AD3-4 cDNA clones that were isolated from an AD brain library. Rnadom primer generated AD3-4 probes yielded two mRNA transcripts, 1.6 kB and 3.4 kB. These mRNA species are over-expressed in AD brains, with an average of two-fold elevation compared with aged matched controls (N =8).

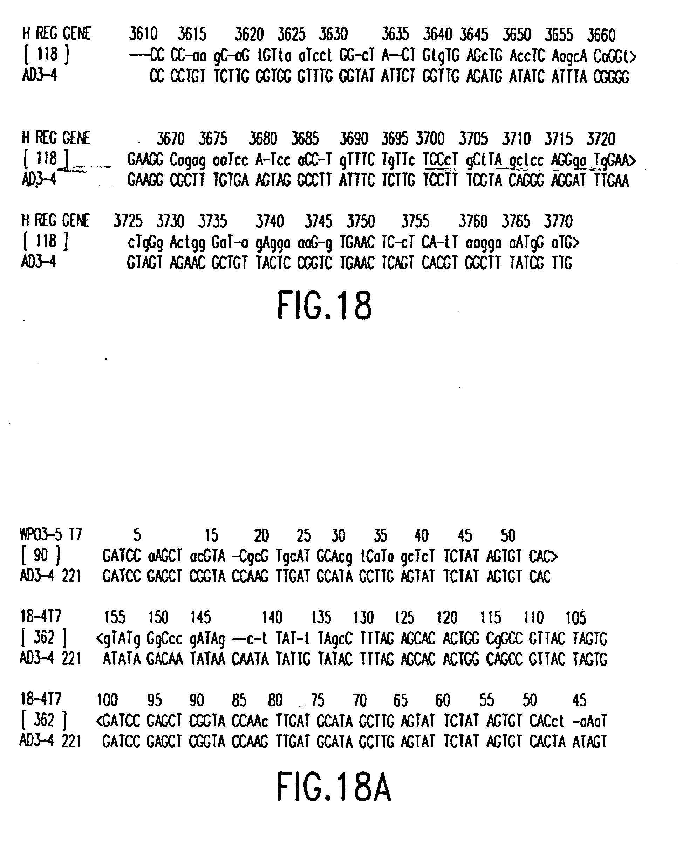

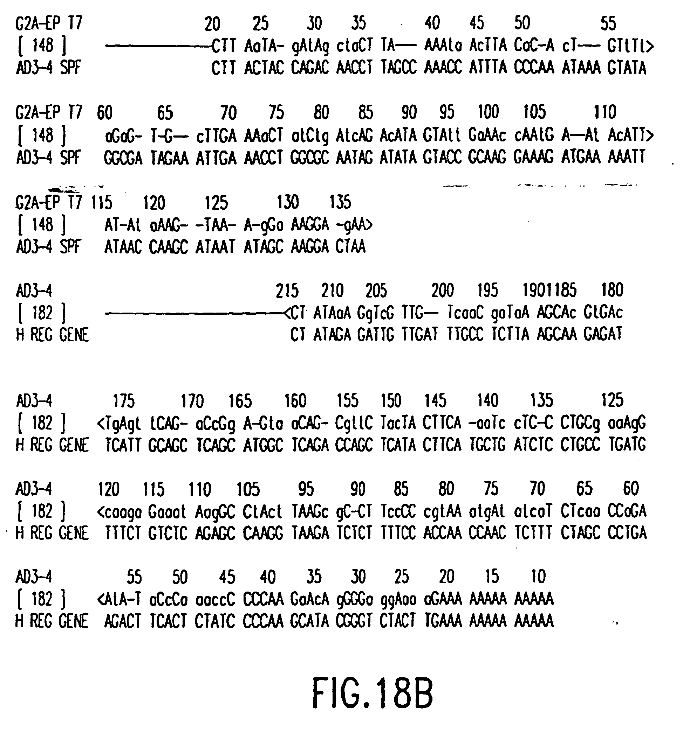

The AD 3-4 cDNA 1.6 kb clone is identical to another clone isolated at the same time (AD 5-3) (Figure 18A). The AD 3-4/AD 5-3 cDNA exhibits substantial homology with the 1-9a 5' anchor PCR products (Figure 18B), as well as with the human Reg gene and the Gen2a-EP genomic clone (Figure 18B). Southern blot analysis of human genomic DNA with the AD 3-4 probe revealed a pattern similar to that obtained with the AD 2-2 probe.

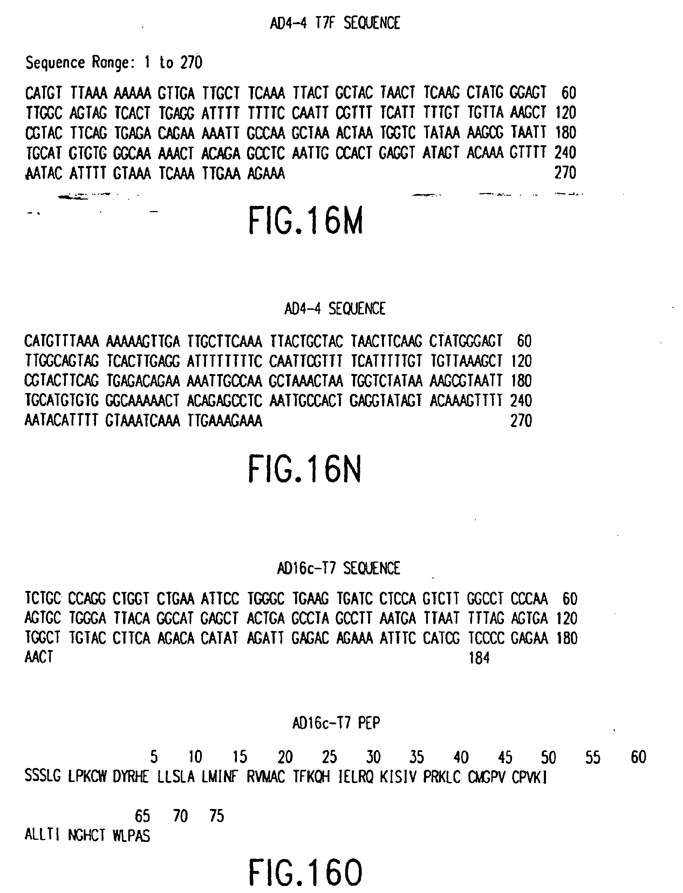

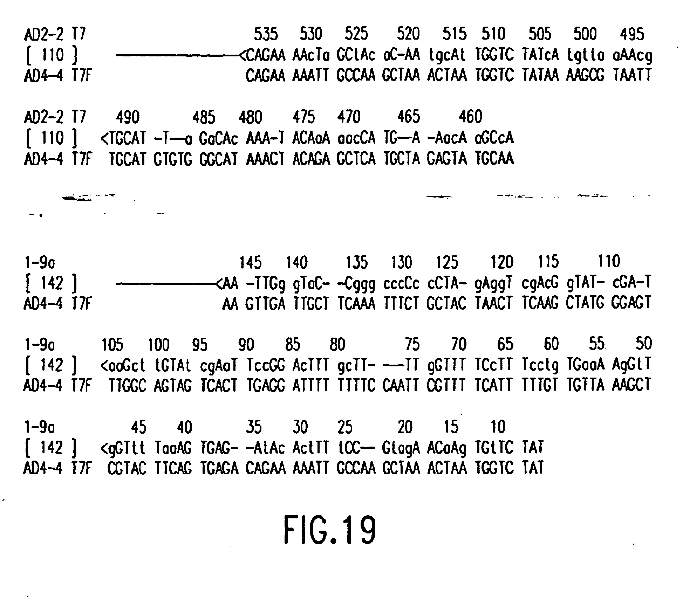

Figures 16L and 16M depict the partial nucleotide sequence of AD 4-4 which is a 0.8 kb partial cDNA clone which is identical to another cDNA isolated at the same time (AD 3-5). This AD 4-4 clone shares substantial sequence homology with AD 2-2 and 1-9a cDNAs (Figure 19). Figure 16N depicts the complete nucleotide sequence of a partial cDNA clone isolated from an AD brain library. This cDNA hybridized with brain and neuronal cell line mRNA, yielding a single 1.4 kB transcript.

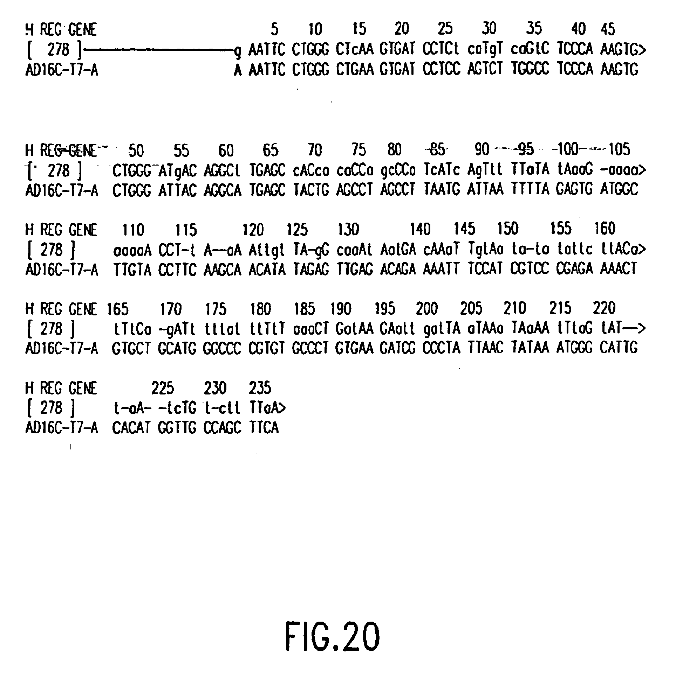

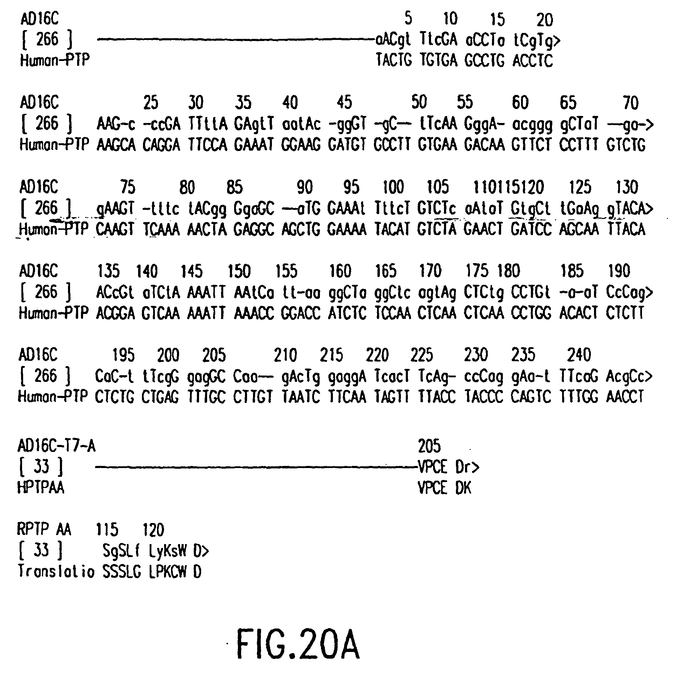

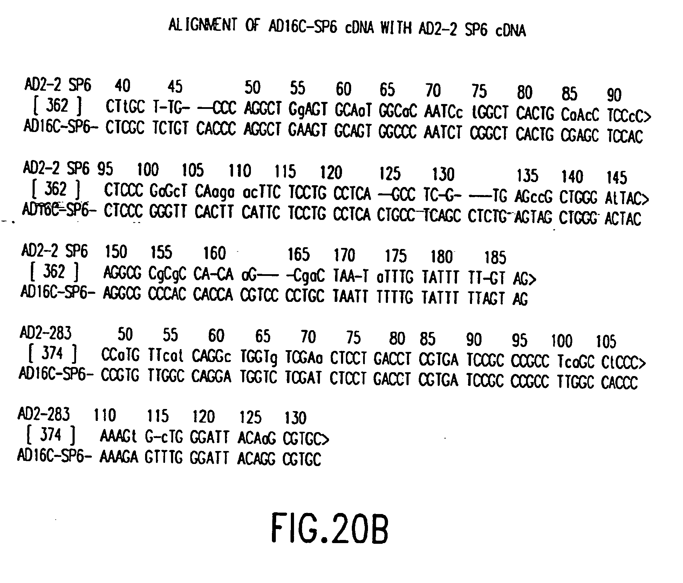

Figure 16O depicts the nucleotide sequence of the 0.5 kb partial cDNA clone AD 16c (also called AD 10-7) that is 72% homologous with AD 2-2, and also aligns with human PTP and the human Reg gene sequences (Figures 20A and 20B).

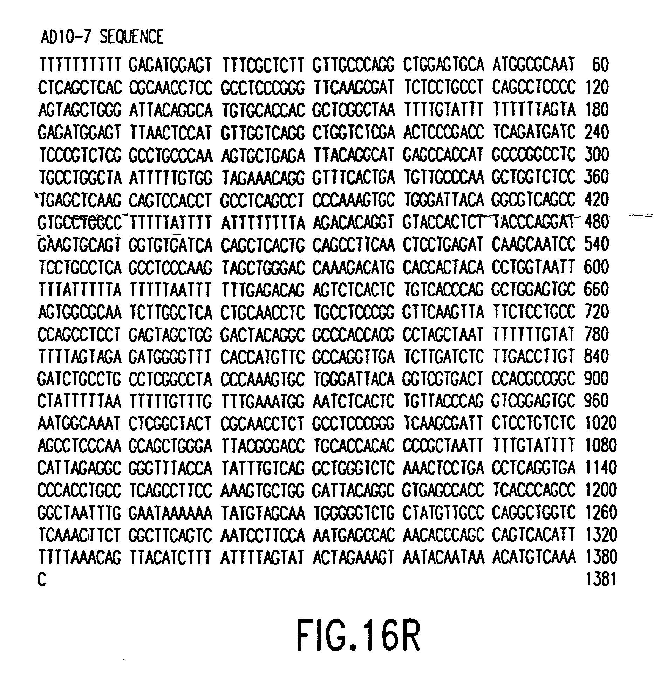

Figure 16R depicts the complete nucleotide sequence of the AD10-7 clone that was isolated from an AD brain library. Hybridization of Northern blots using either antisense cRNA probes or random primer generated DNA probes detected 2.6, 1.9, 1.4 and 0.9 kB mRNA transcripts in neuronal cells. Neuronal cell lines expressed only the two largest transcripts, while manure adult human brains expressed predominantly the two smallest transcripts, and either very low or nondetectable levels of the 2.6 kB and 1.9 kB transcripts. Using an AD10-7 probe, Northern blot analysis of RNA obtained from human liver, ovary, fallopian tube, colon, stomach, spleen, rectum, thyroid, 12 week placenta and kidney was negative.

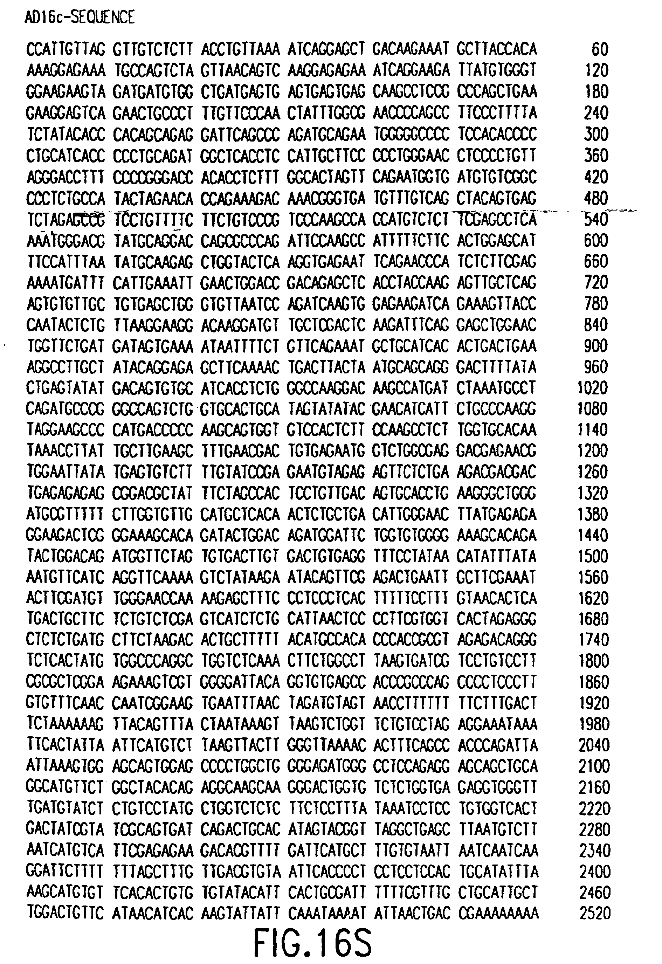

Figure 16S depicts the complete nucleotide sequence of the AD16c cDNA clone that was isolated from an AD brain library. Hybridization of Northern blots using random primer generated DNA probes yielded the same results as obtained with the AD10-7cDNA clone. The AD16c clone shares a 650 bp segment of linear identity with AD10-7. In addition, elevated levels of AD16c mRNA were detected in AD brains compared with aged control brains by Northern blot analysis.

### Example 5

### Analysis of Brain Thread Protein Gene Expression

Thread protein mRNA expression was examined in the following neuroectodermal tumor derived cell lines: central nervous system primitive neuroectodermal tumor cells designated PNET1 and-PNET2; HGL-16 and HGL-17 human glioblastoma cells; A172 human glioma cells; C6 rat glioma cells; and SH-Sy5y neuroblastoma cells. In addition, human brain tissue from patients with Alzheimer's disease or no neurological disease (aged controls), and embryonic and postnatally developing rat brain were assayed for thread protein mRNA expression. RNA extracted from human and rat pancreas served as positive controls.

RNA was extracted in 5 M guanidinium isothiocyanate, and then isolated by centrifugation through a cesium chloride step gradient (*see* Sambrook *et al., supra*). RNA was quantified by measuring the absorbance at 260 nm and 280 nm. The thread protein mRNA transcript sizes were assessed by northern blot analysis, and the levels of expression were evaluated by RNA dot blot hybridization. Northern blot analysis was performed by electrophoresing samples containing 15 µg of total cellular RNA through 1% agarose-formaldehyde gels. The RNA was transferred to nylon membrane, cross-linked with ultraviolet light, and hybridized with probes generated from a 600 bp fragment of the 1-9A cDNA clone. The fragment used for hybridization studies contained the regions most homologous with the human PTP cDNA. The probes were labeled with [³²P] α-dCTP by the random primer method (Amersham Corporation; Arlington Heights, IL). The blots were hybridized overnight at 42°C with 2 x 10⁶ dpm /ml of probe in buffer containing 50% formamide, 5x SSPE, 10x Denhardt's (100x Denhardt's is 2% Ficoll, 2 % bovine serum albumin, 2% polyvinylpyrollidine), 0.5% SDS (sodium dodecyl sulfate), and 100 µg/ml of sheared denatured salmon sperm DNA. The membranes were washed in SSPE containing 0.25% SDS using standard methods. Autoradiograms were generated by exposing the membranes to Kodak XAR film at -80°C. The membranes were subsequently stripped of probe and then rehybridized with a synthetic 30mer corresponding to 18s RNA to evaluate sample loading.

Northern analysis of total cellular RNA using probes made from the 1-9a cDNA disclosed two dominant transcripts in central nervous system (CNS) tumor cell lines: one transcript was 1.6 kb, and the other was 0.9 kb (Figure 12A). In addition, in the SH-Sy5y neuroblastoma and PNETI cell lines, a larger 4.2 kb mRNA transcript was also detected. The 4.2 kb transcript may represent preprocessed mRNA. The same size transcripts were detected in adult (R. Brain) and newborn (NB) rat, but the 0.9 kb transcript was more abundant in the adult brain whereas the 1.6 kb transcript was more abundant in the newborn rat brain. In rat pancreas (R. Panc.), only a 0.9 kB transcript was detected, corresponding to the size of rat PTP mRNA (Terazono et al., J. Biol. Chem. 263:2111-2114. (1988); Watanabe et al., J. Biol. Chem. 265:7432-7439 (1990)). mRNA transcripts were not detected in normal liver (NI Liver). Using a probe generated from the 3' region of the 1-9a cDNA, the 1.6 kb, but not the 0.9 kb transcript was revealed (Figure 12B). Using a 30-mer probe corresponding to the most 5.'-end of the 1-9a cDNA, the higher molecular weight mRNA transcripts were detected (Figure 12C). The 0.9 kb transcript was also evident with longer exposure of the blot.

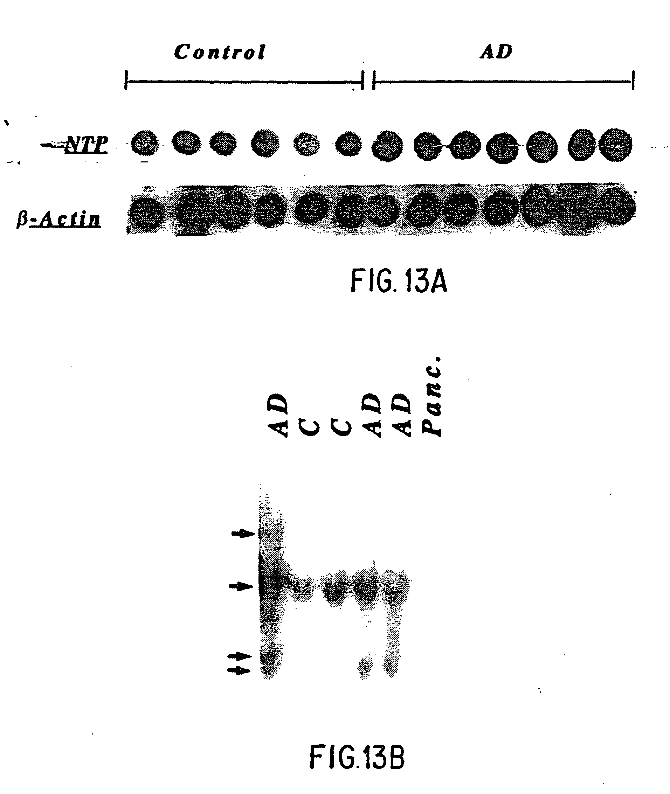

Northern analysis of human brain RNA disclosed a dominant 1.6 kb transcript, but also two and sometime three smaller transcripts of 1.2 kb, 0.9 kb, and 0.8 kb (Figure 13B). In contrast to the findings in cell lines, the 4.2 kb mRNA transcript was seldom observed in adult human brain. Hybridization with human pancreas disclosed a 0.8 kb transcript, corresponding with the size of PTP mRNA. The transcripts detected in human brain and pancreas using 1-9a probes were identical in size to the transcripts observed using PTP cDNA probes.

Dot blot DNA hybridization to 5 µg of total RNA using the 600 bp fragment of the 1-9a cDNA (NTP) demonstrated higher levels of expression in AD, compared with aged control brains (Figure 13A). Rehybridization of the same membrane with a DNA corresponding to β-action demonstrated similar loading of RNA in each dot. The observation of elevated levels of 1-9a-related mRNA in AD brain tissue is similar to that reported previously using 60mer probes corresponding to human PTP cDNA (de la Monte et. al., J. Clin. Invest. 86:1004-1013 (1990)). The differences between AD and control brains appeared to be due to differences in the levels of the 1.6 kb, 0.9 kb and 0.8 kb transcripts, as shown in Figures 13A and 13B.

The AD-NTP 3-4 cDNA, isolated from the AD library, hybridizes with RNA from neuronal-derived neuroectodermal tumor cell lines, and human brain tissue. In the cell lines, 1.6 kb and 0.9 kb transcripts as observed with then 1-9a probe were detected (Figure 21C). However, in human brain, ∼ 4 kb, 1.6 kb, and 0.9 kb transcripts were detected, and the levels of expression for all three transcripts were higher in AD compared with aged control brains (Figure 21D).

AD 4-4 cDNA probe hybridized only with a 0.9kb transcript, and only in neuronal cell lines.

### Example 6

### Direct Cloning and Sequencing of Thread Protein cDNAs from Neuroectodermal Tumor Cell Lines and Alzheimer's Disease Brain

Thread protein cDNAs were cloned directly from PNET1, PNET2, SH-Sy5y, and A172 cells, and from Alzheimer's disease and aged control brain RNA using the 3'- and 5'-RACE methods (Frohman et al:, Proc. Natl. Acad. Sci, USA 85:8998 (1988); Ohara et al., Proc. Natl. Acad. Sci. USA 86:5673 (1989); Loh et al., Science 243:217 (1989)). Briefly, RNA was reverse transcribed using oligo-dT primers. For the 5'-RACE reaction, the cDNAs were amplified by polymerase chain reaction (PCR) using a specific 17-mer corresponding to a 5'-region of the 1-9a sequence, and a 17 dT primer. The resulting PCR products were subjected to another round of amplification using another internal but overlapping 5'-end primer, and a specific 3'-17-mer corresponding to a 3' region of the 1-9a sequence. For the 3'-RACE reactions, the cDNAs were first tailed with dCTP using terminal deoxynucleotide transferase, and then they were amplified using a specific 17-mer corresponding to nucleotides 781-797 of the 1-9a clone and dG (17mer). A second nested PCR amplifcation was performed using a specific 17mer corresponding to nucleotides 766-792 at the 3'end, and dGTP (17mer) for the 5' end. The PCR product were subjected to Southern blot analysis using probes generated from an internal DNA fragments of the 1-9a cDNA clone, and from the O18 rat PTP cDNA clone. The PCR products were gel purified and ligated into pAmpl vectors using uracil deoxytransferase. The subcloned DNA inserts were sequenced by the dideoxynucleotide chain termination method using T7 DNA polymerase.

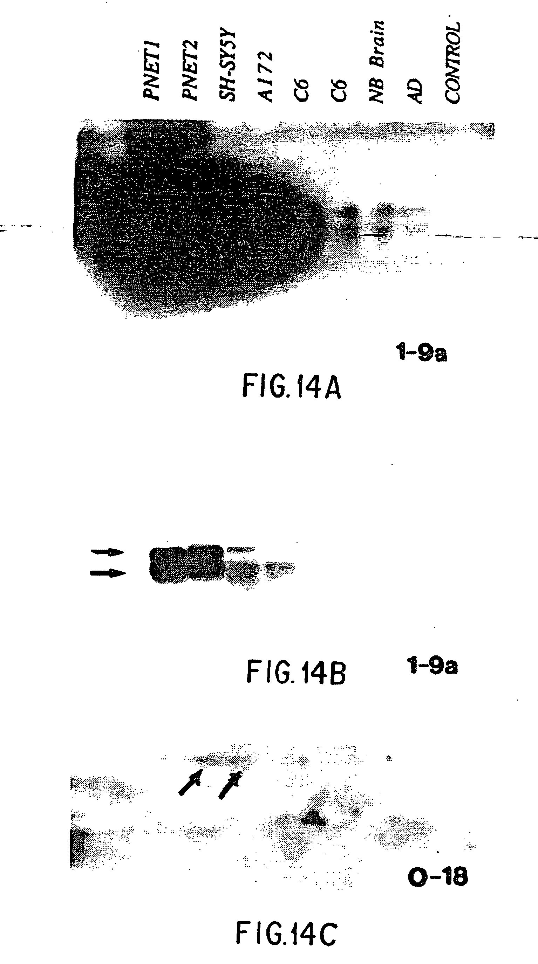

CNS thread protein transcripts were detected in neuroectodermal tumor cell lines and in AD human brain tissue by reverse transcription followed by PCR using specific primers corresponding to the-5' and 3' regions of the 1-9a cDNA sequences. Southern blot analysis of the PCR products demonstrated two dominant cross-hybridizing species, 0.8kb and 1.0 kb (Figures 14A and 14B). In addition, in the SH-Sy5y cells, a larger 1.8 kb PCR product was also detected. In the PNET1; PNET2; SH-Sy5y, and A172 cells, a 0.4 kb PCR product that hybridized with the 1-9a probe was observed. Corresponding with the higher levels of thread protein mRNAs in Alzheimer's disease brains, the hybridization signal was more intense in AD samples compared with aged control samples.

The PCR products generated from the SH-Sy5y cells were subcloned and sequenced. Southern analysis of the cloned fragments exhibited intense hybridization with the 1-9a cDNA, and less intense but definite hybridization with the O18. cDNA (rat PTP) (Figure 14C). The nucleic acid sequence of the SH-Sy5y PCR clone (Sy-NTP) was identical to the 1-9a cDNA sequence.

### Example 7

### Isolation of Genomic Clones Coding for Human Brain Thread Proteins

A human genomic DNA library was screened using probes made with a 600 bp fragment of the 1-9a human brain thread protein DNA that was isolated from the two year-old temporal cortex library. The 1-9a cDNA fragment contained a region with 60% nucleic acid sequence homology with human PTP. After colony purification, the putative genomic clones were checked for cross-hybridization with the 018 rat PTP cDNA fragment. *Eco*RI*, Pst*I*,* and *Eco*RI/*Pst*I restriction fragments that hybridized with both the 1-9a and O18 probes were subcloned into pBluescript II vectors (Promega, Inc., Madison, WI) and then sequenced by the dideoxynucleotide chain termination method using either T7 polymerase (USE Sequenase) or Polymerase chain reaction amplification and Vent polymerase.

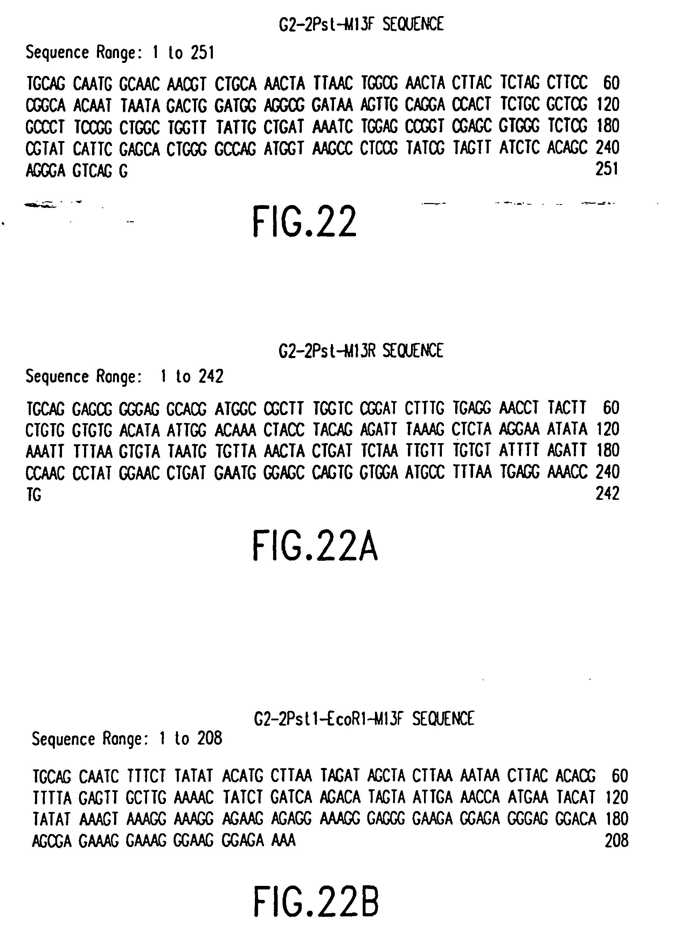

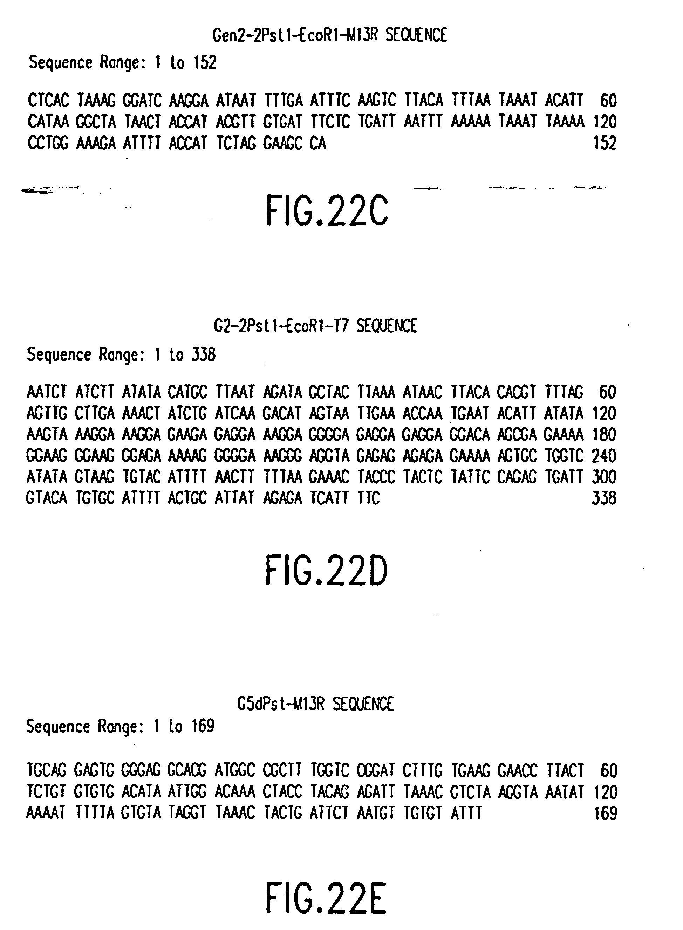

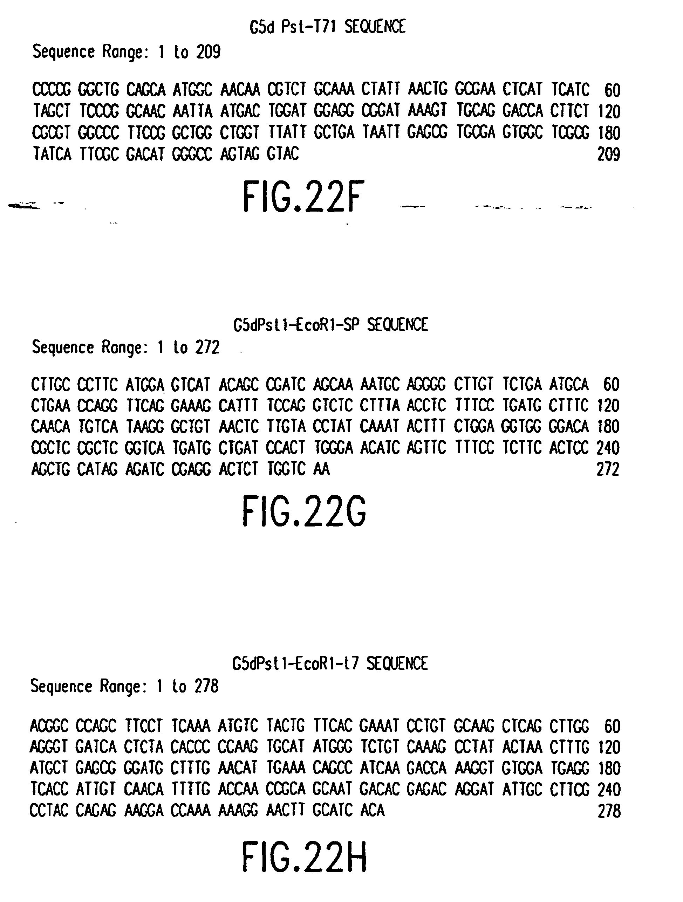

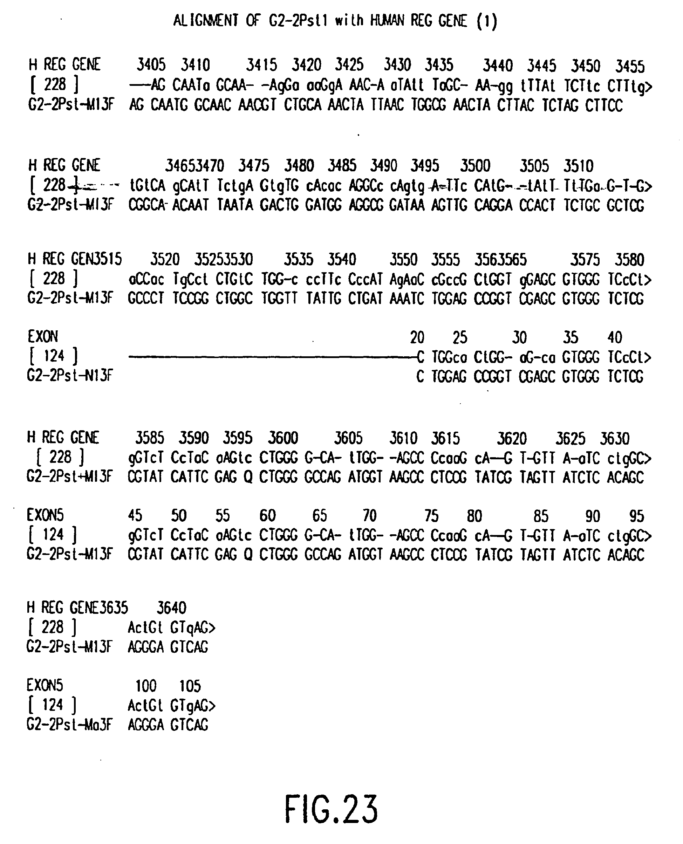

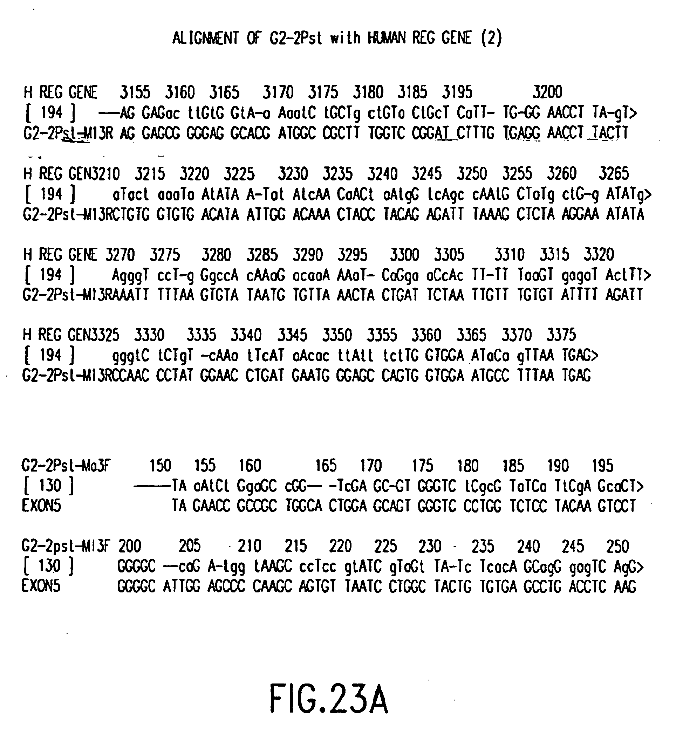

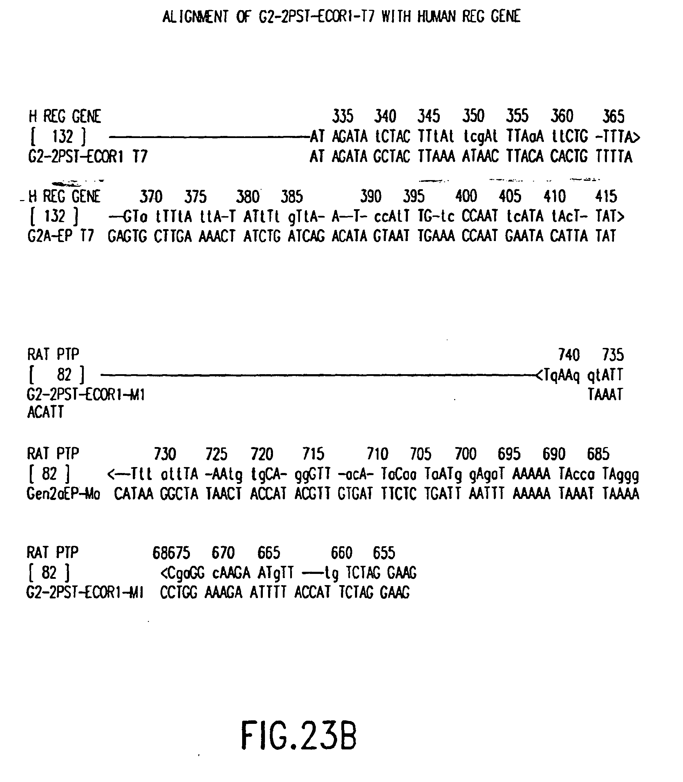

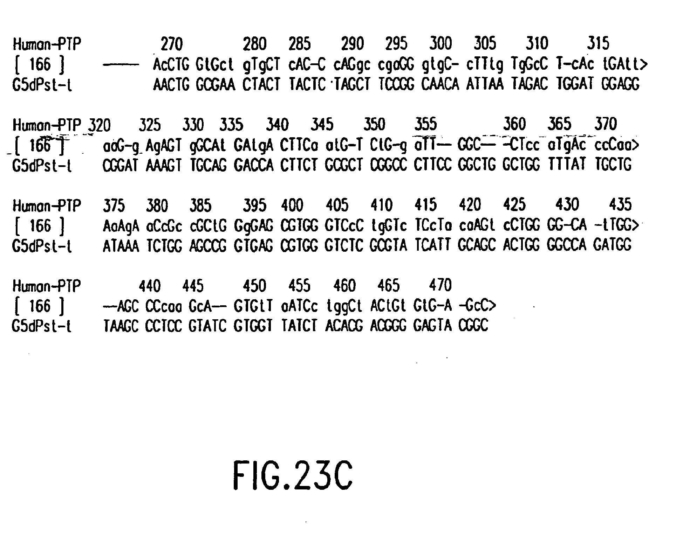

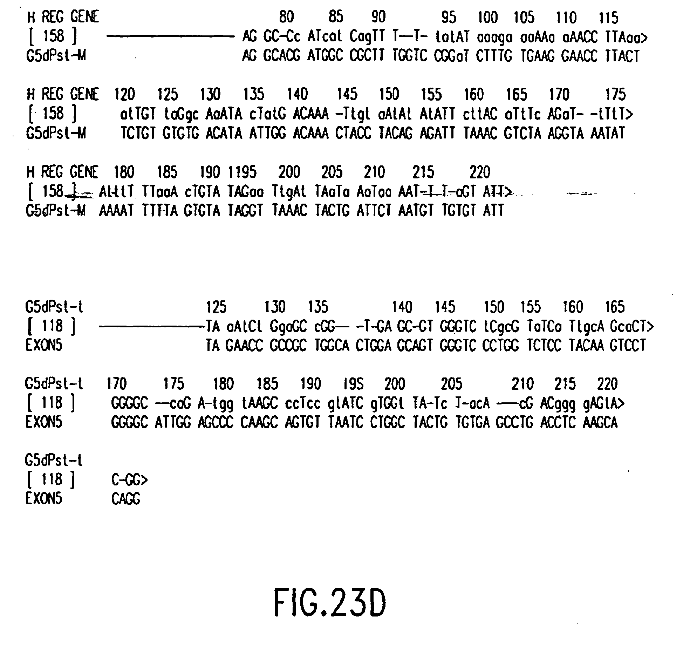

Four genomic fragments designated G2-2 *Pst*I, G2-2 *Pst*I*-Eco*RI*,* G5d-1 *Pst*I*,* and G5d-1 *Pst*I*-Eco*RI were isolated from a human genomic DNA library (Figures 22A-22D). These genomic fragments all hybridized with both the 1-9a and 018 cDNA probes, and they ranged in size between 1.5 kb and 3 kb. Partial nucleic acid sequence information demonstrated homology between G2-2*Pst*I and the human Reg gene and human and rat PTP cDNAs (Figure 23A); between G2-2 *Pst*I*-Eco*RI and both the Reg gene and rat PTP cDNA -(Figure 23B), and also with AD 2-2, AD 3-4, and the 1-9a cDNAs (data not shown); between G5d-1 *Pst*I and the Reg gene and human PTP (Figure 23C); and between G5d-1 *Pst*I*-Eco*RI and Reg gene, human PTP, 1-9a, and AD 4-4.

### Example 8

### In vitro Expression of the LacZ Fusion Protein and Demonstration of its Relatedness to Thread Proteins

Fusion protein expression in bacteria containing the 1-9a cDNA clone, or one of the four genomic clones was induced with isopropylthio-β-D-galactoside (IPTG) using standard techniques (Sambrook *et al*., *supra*). Crude bacterial lysates from induced and uninduced cultures were subjected to SDS-PAGE and Western blot analysis using the Th9 monoclonal antibody to thread proteins (Sasaki et al., J. Biol. Chem. 268:1-4 (1993)), and ¹²⁵-I labeled protein A to detect the bound antibody. In addition, bacterial lawns containing cloned DNA were induced to express the fusion protein with IPTG, and replica filters were probed directly with Th9 monoclonal antibody followed by ¹²⁵-I labeled protein A.

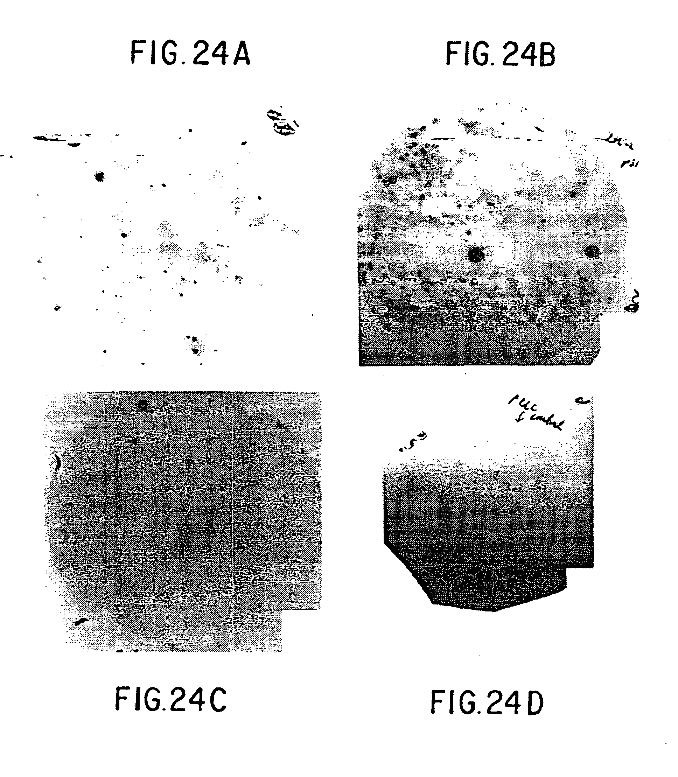

Thread protein immunoreactivity was demonstrated in the bacterial fusion proteins by direct antibody binding to the IPTG-induced colonies (Figures 24A-24D). Thread protein immunoreactivity was detected using a cocktail of Th9, Th7, and Th10 monoclonal antibodies to PTP (Sasaki et al., J. Biol. Chem. 268:1-4 (1993); and ¹²⁵-I labeled Protein A.

### Example 9

### Relative Levels of AD16c mRNA in AD and Aged Control Brains

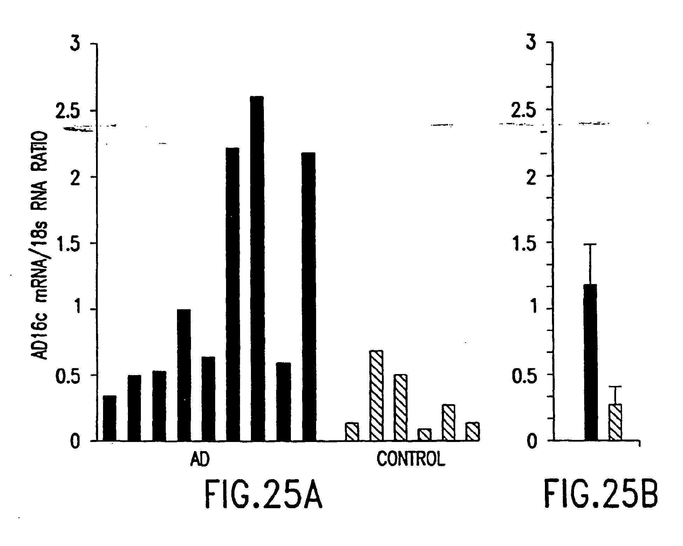

Northern blot analysis was performed using an AD16 cDNA probe. The blots were re-probed to detect 18s ribosomal RNA to evaluate loading of RNA in each lane. The unsaturated autoradiograms were subjected to densitometric analysis using a Molecular Dynamics Image-Analyzer. The ratios of the AD16c and 18s RNA hybridization signals were plotted for each case, and the results are depicted graphically in Figures 25A and 25B. The mean rations (relative levels of AD16c) with standard errors are depicted in the smaller right hand graph. The findings confirm that there are elevated levels of AD16c mRNA expression in 6 of 9 AD brains compared to 1 of 6 age-matched controls. The difference between the mean levels is highly statistically significant (P<0.005). Similar results were obtained using AD10-7 probes. Theses results demonstrate that there is a statistically significant increase in levels of expression in AD brains compared to control brains.

### Example 10

### Preparation of Recombinant AD10-7 Fusion Protein and Detection Thereof With Monoclonal Antibodies

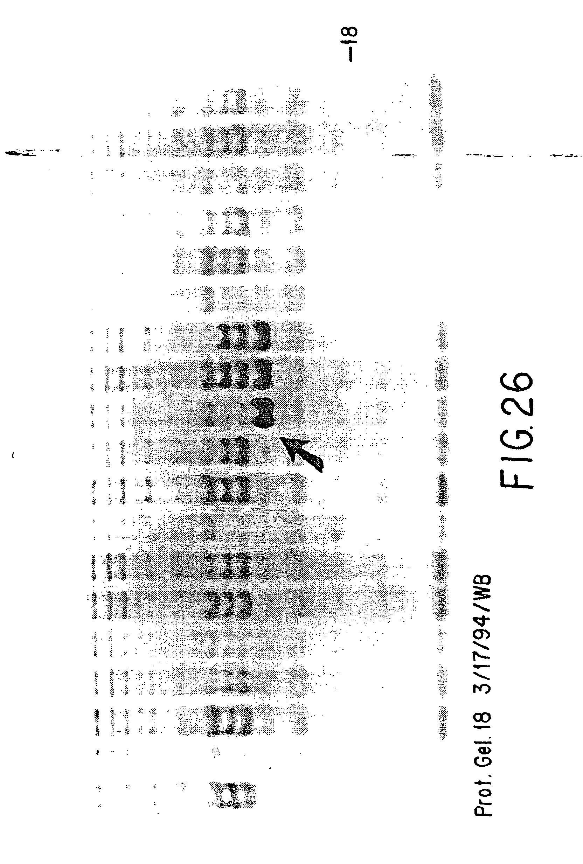

AD10-7 cDNA was ligated into pTrcHIS vectors (In Vitrogen, San Diego) in three different reading frames (two incorrect-A and B, and one correct-C). Bacteria transformed with one of the three plasmids were induced with IPTG and bacterial lysates were examined for protein expression 0, 1 and 5 hours later. The proteins were fractionated by SDS-PAGE, and Western blot analysis was performed using monoclonal antibodies against the expressed tag protein (T7-tag mouse monoclonal antibodies; Novogen). The blots were developed using the avidin-biotin, horseradish peroxidase method, with diaminobenzidine as the chromogen (Figure 26). A band corresponding to ∼45 kDA was detected in bacteria that had been transformed with plasmid DNA which contained AD10-7 ligated only in the correct reading frame (C) (arrow). The same size protein was observed by *in vitro* translation of the AD10-7 cDNA in a rabbit reticulocyte lysate assay system. In both systems, the fusion partner peptide was ∼ 3 kDA, indicating that the cDNA encodes a protein of about ∼ 42 kDA. A ∼42 kDA NPT species is routinely detected by Western Blot analysis of neuronal cell lines and of human brain tissue.

### Example 11

### Demonstration of Neuronal Localization of AD10-7 mRNA Expression by In Situ Hybridization

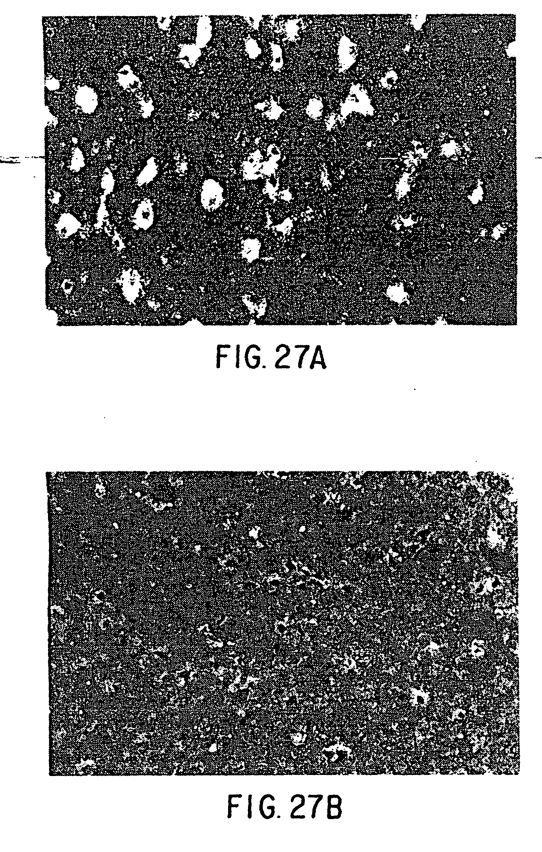

Sense and antisense cRNA probes were generated from linearized AD10-7 plasmid DNA using SP6 or T7 DNA-dependent RNA polymerase, respectively. The antisense probes hybridized with neuronal cell line mRNA as described above for this clone. The cRNA sense probes, on the other hand, failed to hybridize with RNA by Northern blot analysis. cRNA probes labeled with digoxigenin-UTP were hybridized with human brain tissue sections from early AD. After washing the sections extensively (de la Monte et al., J. Clin. Invest. 86:1004-1013 (1990)), the hybridized probes were detected using peroxidase or alkaline phosphatase conjugated monoclonal antibodies to digoxigenin, and the colorimetric reactions were revealed using standard methods. Examination of the sections by brightfield and darkfield microcopy demonstrated hybridization of AD10-7 only in neurons (Fig. 27; dense aggregates of white grains over cell bodies in (Fig. 27A)). In contrast, and similar to the findings by Northern blot analysis, the sense AD10-7 cRNA probes failed to hybridize with brain tissue (Fig. 27B).

Although the foregoing refers to particular preferred embodiments, it Will be understood that the present invention is not so limited. It will occur to those of ordinary skill in the art that various modifications may be made to the disclosed embodiments and that such modifications are intended to be within the scope of the present invention, which is defined by the following Claims.

## Claims

1. A Neural Thread Protein (NTP) substantially free of any natural impurities and coded for by the AD3-4DH1 DNA molecule present in the E. *coli* cells that are on deposit at the American Type Culture Collection, Manassass, Va., under accession no. 69260.

2. An isolated nucleic acid molecule encoding a Neural Thread Protein (NTP) comprising the AD3-4DH1 DNA molecule present in the E. *coli* cells that are on deposit at the American Type Culture Collection, Manassass, Va., under accession no. 69260.

3. The nucleic acid molecule of claim 2 comprising a genetic sequence coding for an NTP wherein said NTP is human NTP.

4. A plasmid comprising the nucleic acid molecule of claim 2 or claim 3.

5. A non-human host transformed with the plasmid of claim 4.

6. An *in vitro* method of using the plasmid of claim 4 to prepare an NTP, said method comprising:
(a) introducing said plasmid comprising said genetic sequence coding for said NTP into a host cell to produce a recombinant host cell;
(b) culturing said recombinant host cell; and
(c) isolating said NTP.

7. The method of claim 6, wherein said genetic sequence coding for said NTP is obtained from brain tissue of a patient with Alzheimer's Disease or from a human neural tumor cell line.

8. The method of claim 6 or claim 7, wherein said host is *E*. *coli.*

9. The method as claimed in any one of claims 6 to 8, wherein said gene is contained by a vector.

10. The method as claimed in any one of claims 6 to 9, wherein said gene is under control of an inducible promoter.

11. The method of claim 10, wherein said promoter is a lambda P₁ promoter, or a *tac* promoter.

12. An expression vector comprising the nucleic acid molecule of claim 2.

13. A virion comprising the expression vector of claim 12.

14. A ribozyme comprising a target sequence which is complementary to a nucleic acid sequence coding for the NTP of claim 1, and non-homologous to the PTP nucleic acid sequence.

15. A DNA molecule which codes for the ribozyme of claim 14.

16. A ribonucleotide NTP External Guide Sequence comprising:
(a) a 10-15 nucleotide sequence which is complementary to a nucleic acid sequence which codes for the NTP of claim 1, and which is non-homologous to the PTP nucleic acid sequence; and
(b) a 3'-NCCA nucleotide sequence wherein N is purine.

17. The NTP External Guide Sequence of claim 16 which is DNA.

18. An *in vitro* method for detecting the presence of Neural Thread Protein (NTP) encoded by the nucleic acid of claim 2, said method comprising:
(a) contacting a biological sample from a human subject that is suspected of containing an NTP encoded by the nucleic acid of claim 2 with at least one molecule capable of binding to said NTP;
(b) detecting any of molecule bound to said NTP.

19. A method as claimed in claim 18, wherein said molecule is:
(a) an antibody substantially free of natural impurities;
(b) a monoclonal antibody; or
(c) a fragment of (a) or (b).

20. A method as claimed in claim 18 or claim 19, wherein the detecting of any of said molecule bound to said NTP is performed by *in situ* imaging.

21. A method as claimed in any one of claims 18 to 20, wherein the detecting of any of said molecule bound to said NTP is performed by *in vitro* imaging.

22. A *in vitro* method of diagnosing the presence of a Neural Thread Protein (NTP) - related condition in a human subject suspected of having such a condition, which comprises:
(a) incubating a biological sample, which is suspected of containing an NTP encoded for by said nucleic acid molecule of claim 2, in the presence of at least one binding molecule capable of identifying said NTP; and
(b) detecting said binding molecule which is bound in said sample, wherein said detection indicates that said subject has an NTP-related condition;
wherein said NTP-related condition is Alzheimer's Disease, the presence of neuroectodermal tumors, the presence of a malignant astrocytoma or the presence of glioblastomas.

23. A diagnostic method as claimed in claim 22, wherein said detection is by immunometric assay.

24. A diagnostic method as claimed in claim 23, wherein said immunometric assay is a monoclonal antibody-based immunometric assay.

25. A diagnostic method as claimed in claim 24, wherein said method comprises:
(a) incubating said biological sample with two different NTP monoclonal antibodies bound to a solid phase support; and
(b) detecting NTP as defined in claim 1 bound to said monoclonal antibodies with a third different detectably labelled NTP monoclonal antibody solution, wherein said NTP monoclonal antibodies are specific for the NTP molecule of claim 1.

26. A diagnostic method as claimed in any one of claims 22 to 25, wherein said incubating step further includes adding a known quantity of labelled NTP as defined in claim 1 whereby a competitive immunoassay is established.

27. A diagnostic method as claimed in claim 26, wherein said label is capable of emitting radiation.

28. A diagnostic method as claimed in claim 27, wherein said label is ¹²⁵1.

29. A diagnostic method as claimed in any one of claims 22 to 28, wherein said detection is by immuno-polymerase chain reaction.

## Patentansprüche

1. Neurofilamentprotein (NTP; "Neural Thread Protein"), das im Wesentlichen frei von jeglichen natürlichen Verunreinigungen und durch das AD3-4DH1-DNA-Molekül kodiert ist, welches in den E.coli-Zellen, die in der "American Type Culture Collection", Manassass, Va., unter der Hinterlegungsnummer 69260 hinterlegt sind, vorhanden ist.

2. Isoliertes Nukleinsäuremolekül, kodierend ein Neurofilamentprotein (NTP) umfassend das AD3-4DH1-DNA-Molekül, welches in den E.coli-Zellen, die in der "American Type Culture Collection", Manassass, Va., unter der Hinterlegungsnummer 69260 hinterlegt sind, vorhanden ist.

3. Nukleinsäuremolekül nach Anspruch 2, umfassend eine genetische Sequenz, die für ein NTP kodiert, wobei das NTP ein menschliches NTP ist.

4. Plasmid, umfassend das Nukleinsäuremolekül nach Anspruch 2 oder 3.

5. Nicht-menschlicher Wirt, der mit dem Plasmid nach Anspruch 4 transformiert ist.

6. In-vitro-Verfahren zur Verwendung des Plasmids nach Anspruch 4 zur Herstellung eines NTP, wobei das Verfahren umfasst:
(a) Einbringen des Plasmids, umfassend die genetische Sequenz, die für das NTP kodiert, in eine Wirtszelle, um eine rekombinante Wirtszelle zu produzieren;
(b) Züchten der rekombinanten Wirtszelle; und
(c) Isolieren des NTP.

7. Verfahren nach Anspruch 6, wobei die genetische Sequenz, die für das NTP kodiert, aus Gehirngewebe eines Patienten, der an der Alzheimerschen Krankheit leidet, oder aus einer menschlichen neuralen Tumorzelllinie erhalten wurde.

8. Verfahren nach Anspruch 6 oder 7, wobei der Wirt *E*. *coli* ist.

9. Verfahren nach einem der Ansprüche 6 bis 8, wobei das Gen in einem Vektor enthalten ist.

10. Verfahren nach einem der Ansprüche 6 bis 9, wobei das Gen unter Kontrolle eines induzierbaren Promotors steht.

11. Verfahren nach Anspruch 10, wobei der Promotor ein Lambda-P₁-Promotor oder ein tac-Promotor ist.

12. Expressionsvektor, umfassend das Nukleinsäuremolekül nach Anspruch 2.

13. Virion, umfassend den Expressionsvektor nach Anspruch 12.

14. Ribozym, umfassend eine Targetsequenz, die zu einer Nukleinsäuresequenz, die für das NTP nach Anspruch 1 kodiert, komplementär und zur PTP-Nukleinsäuresequenz nicht homolog ist.

15. DNA-Molekül, das für das Ribozym nach Anspruch 14 kodiert.

16. Externe Ribonukleotid-NTP-Führungssequenz, umfassend:
(a) eine 10-15 Nukleotidsequenz, die zu einer Nukleotidsequenz, die für das NTP nach Anspruch 1 kodiert, komplementär und zur PTP-Nukleinsäuresequenz nicht homolog ist; und
(b) eine 3'-NCCA-Nukleotidsequenz, wobei N ein Purin ist.

17. Externe NTP-Führungssequenz nach Anspruch 16, die DNA ist.

18. In-vitro-Verfahren zum Detektieren der Anwesenheit von Neurofilamentprotein (NTP), das von der Nukleinsäure nach Anspruch 2 kodiert ist, wobei das Verfahren umfasst:
(a) Kontaktieren einer biologischen Probe aus einem menschlichen Subjekt, bei der vermutet wird, dass sie ein NTP enthält, das von der Nukleinsäure nach Anspruch 2 kodiert wird, mit zumindest einem Molekül, das an dieses NTP binden kann;
(b) Detektieren jedes der an das NTP gebundenen Moleküle.

19. Verfahren nach Anspruch 18, wobei das Molekül:
(a) ein Antikörper, der im Wesentlichen frei von natürlichen Verunreinigungen;
(b) ein monoklonaler Antikörper; oder
(c) ein Fragment von (a) oder (b); ist.

20. Verfahren nach Anspruch 18 oder 19, wobei das Detektieren jedes der an das NTP gebundenen Moleküle durch *In*-*situ-*Bildgebung erfolgt.

21. Verfahren nach einem der Ansprüche 18 bis 20, wobei das Detektieren jedes der an das NTP gebundenen Moleküle durch In-vitro-Bildgebung erfolgt.

22. In-vitro-Verfahren zum Diagnostizieren der Anwesenheit eines mit Neurofilamentprotein (NTP) verbundenen Zustands bei einem menschlichen Subjekt, bei dem ein solcher Zustand vermutet wird, das umfasst:
(a) Inkubieren einer biologischen Probe, bei der vermutet wird, dass sie ein NTP enthält, das durch das Nukleinsäuremolekül nach Anspruch 2 kodiert ist, in Anwesenheit von zumindest einem Bindungsmolekül, das das NTP identifizieren kann; und
(b) Detektieren des Bindungsmoleküls, das in dieser Probe gebunden ist, wobei das Detektieren anzeigt, dass das Subjekt einen mit NTP verbundenen Zustand aufweist;
wobei der mit NTP verbundene Zustand die Alzheimersche Krankheit, die Anwesenheit von neuroektodermalen Tumoren, die Anwesenheit von bösartigem Astrozytom oder die Anwesenheit von Glioblastomen ist.

23. Diagnostisches Verfahren nach Anspruch 22, wobei die Detektion durch einen immunometrischen Assay erfolgt.

24. Diagnostisches Verfahren nach Anspruch 23, wobei der immunometrische Assay ein monoklonaler, auf Antikörpern basierender immunometrischer Assay ist.

25. Diagnostisches Verfahren nach Anspruch 24, wobei das Verfahren umfasst:
(a) Inkubieren der biologischen Probe mit zwei unterschiedlichen monoklonalen NTP-Antikörpern, die an einen Festphasenträger gebunden sind; und
(b) Detektieren von NTP nach Anspruch 1, das an die monoklonalen Antikörper gebunden ist, mit einer dritten unterschiedlichen, detektierbar markierten monoklonalen Antikörperlösung, wobei die monoklonalen NTP-Antikörper für das NTP-Molekül nach Anspruch 1 spezifisch sind.

26. Diagnostisches Verfahren nach einem der Ansprüche 22 bis 25, wobei der Inkubationsschritt weiters das Zugeben einer bekannten Menge an markierten NTP nach Anspruch 1 umfasst, wodurch ein kompetitiver Immunoassay erstellt wird.

27. Diagnostisches Verfahren nach Anspruch 26, wobei die Markierung Strahlung abgeben kann.

28. Diagnostisches Verfahren nach Anspruch 27, wobei die Markierung ¹²⁵I ist.

29. Diagnostisches Verfahren nach einem der Ansprüche 22 bis 28, wobei die Detektion durch eine Immuno-Polymerase-Kettenreaktion erfolgt.

## Revendications

1. Protéine des neurofilaments (NTP) pratiquement dépourvue de toute impureté naturelle et codée par la molécule d'ADN de AD3-4DH1 présente dans les cellules d'E. coli qui sont en dépôt à l'American Type Culture Collection, Manassas, VA, sous le no. d'accession 69 260.

2. Molécule d'acide nucléique isolé codant pour une protéine des neurofilaments (NTP) comprenant la molécule d'ADN de AD3-4DH1 présente dans les cellules d'E. coli qui sont en dépôt à l'American Type Culture Collection, Manassas, VA, sous le no. d'accession 69 260.

3. Molécule d'acide nucléique selon la revendication 2 comprenant une séquence génétique codant pour une NTP dans laquelle ladite NTP est une NTP humaine.

4. Plasmide comprenant la molécule d'acide nucléique selon la revendication 2 ou la revendication 3.

5. Hôte non humain transformé par le plasmide selon la revendication 4.

6. Procédé in vitro d'utilisation du plasmide selon la revendication 4 pour préparer une NTP, ledit procédé comprenant :
(a) l'introduction dudit plasmide comprenant ladite séquence génétique codant pour ladite NTP dans une cellule hôte pour produire une cellule hôte recombinante ;
(b) la culture de ladite cellule hôte recombinante ; et
(c) l'isolement de ladite NTP.

7. Procédé selon la revendication 6, dans lequel ladite séquence génétique codant pour ladite NTP est obtenue à partir de tissu cérébral d'un patient souffrant de la maladie d'Alzheimer ou à partir d'une lignée cellulaire de tumeur neurale humaine.

8. Procédé selon la revendication 6 ou la revendication 7, dans lequel ledit hôte est E. coli.

9. Procédé selon l'une quelconque des revendications 6 à 8, dans lequel ledit gène est contenu par un vecteur.

10. Procédé selon l'une quelconque des revendications 6 à 9, dans lequel ledit gène est sous le contrôle d'un promoteur inductible.

11. Procédé selon la revendication 10, dans lequel ledit promoteur est un promoteur P₁ de lambda ou un promoteur tac.

12. Vecteur d'expression comprenant la molécule d'acide nucléique selon la revendication 2.

13. Virion comprenant le vecteur d'expression selon la revendication 12.

14. Ribozyme comprenant une séquence cible qui est complémentaire d'une séquence d'acide nucléique codant pour la NTP selon la revendication 1, et non homologue de la séquence d'acide nucléique de la PTP.

15. Molécule d'ADN qui code pour le ribozyme selon la revendication 14.

16. Séquence guide externe ribonucléotidique de NTP comprenant :
(a) une séquence de 10 à 15 nucléotides qui est complémentaire d'une séquence d'acide nucléique qui code pour la NTP selon la revendication 1 et qui est non homologue de la séquence d'acide nucléique de la PTP ; et
(b) une séquence nucléotidique 3'-NCCA dans laquelle N représente une purine.

17. Séquence guide externe de NTP selon la revendication 16 qui est de l'ADN.

18. Procédé in vitro de détection de la présence de protéine des neurofilaments (NTP) codée par l'acide nucléique selon la revendication 2, ledit procédé comprenant :
(a) la mise en contact d'un échantillon biologique provenant d'un sujet humain qui est suspecté de contenir une NTP codée par l'acide nucléique selon la revendication 2 avec au moins une molécule capable de se lier à ladite NTP ;
(b) la détection d'une molécule quelconque liée à ladite NTP.

19. Procédé selon la revendication 18, dans lequel ladite molécule est :
(a) un anticorps pratiquement dépourvu d'impuretés naturelles ;
(b) un anticorps monoclonal : ou
(c) un fragment de (a) ou (b).

20. Procédé selon la revendication 18 ou la revendication 19, dans lequel la détection de l'une quelconque desdites molécules liées à ladite NTP est réalisée par une imagerie in situ.

21. Procédé selon l'une quelconque des revendications 18 à 20, dans lequel la détection de l'une quelconque desdites molécules liées à ladite NTP est réalisée par une imagerie in vitro.

22. Procédé in vitro de diagnostic de la présence d'une affection associée à la protéine des neurofilaments (NTP) chez un sujet humain suspecté de souffrir d'une telle affection, qui comprend :
(a) l'incubation d'un échantillon biologique, qui est suspecté de contenir une NTP codée par ladite molécule d'acide nucléique selon la revendication 2, en présence d'au moins une molécule de liaison capable d'identifier ladite NTP ; et
(b) la détection de ladite molécule de liaison qui est liée dans ledit échantillon, où ladite détection indique que ledit sujet souffre d'une affection associée à la NTP ;
dans lequel ladite affection associée à la NTP est la maladie d'Alzheimer, la présence de tumeurs neuroectodermiques, la présence d'un astrocytome malin ou la présence de glioblastomes.

23. Procédé de diagnostic selon la revendication 22, dans lequel ladite détection est par un test immunométrique.

24. Procédé de diagnostic selon la revendication 23, dans lequel ledit test immunométrique est un test immunométrique à base d'anticorps monoclonal.

25. Procédé de diagnostic selon la revendication 24, où ledit procédé comprend :
(a) l'incubation dudit échantillon biologique avec deux anticorps monoclonaux anti-NTP différents liés à un support de phase solide ; et
(b) la détection de la NTP telle que définie dans la revendication 1 liée aux dits anticorps monoclonaux avec une solution d'un troisième anticorps monoclonal anti-NTP différent marqué de manière détectable, où lesdits anticorps monoclonaux anti-NTP sont spécifiques de la molécule de NTP selon la revendication 1.

26. Procédé de diagnostic selon l'une quelconque des revendications 22 à 25, dans lequel ladite étape d'incubation comprend en outre l'addition d'une quantité connue de NTP marquée telle que définie dans la revendication 1, moyennant quoi un test immunologique compétitif est établi.

27. Procédé de diagnostic selon la revendication 26, dans lequel le marqueur est capable d'émettre des radiations.

28. Procédé de diagnostic selon la revendication 27, dans lequel ledit marqueur est ¹²⁵I.

29. Procédé de diagnostic selon l'une quelconque des revendications 22 à 28, dans lequel ladite détection est par une immuno-réaction en chaîne de la polymérase.
